# EUROPEAN PATENT APPLICATION

(11) **EP 4 610 267 A1**
(43) Date of publication of application: **03.09.2025**
(21) Application number: 24160538.5
(22) Date of filing: 29.02.2024
(51) Int. Cl.: C07H 21/02, A61K 31/7084, A61P 35/00

(54) **CYCLIC DINUCLEOTIDES AND CONJUGATES THEREOF WITH ANTIBODIES**

(71) Applicant: Ustav Organicke Chemie a Biochemie AV CR, v.v.i., 16000 Praha 6 (CZ)
(72) Inventor: Pav, Ondrej, Praha (CZ); Birkus, Gabriel, Praha (CZ)
(74) Representative: Harber IP s.r.o.

(57) **Abstract**

The present invention provides a compound of general formula I: 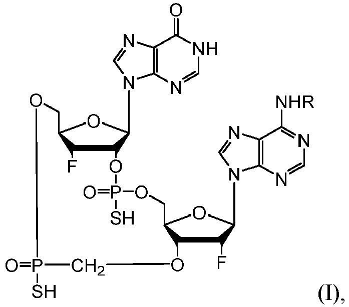 wherein R represents hydrogen, a linker structure or anl antibody attached by the linker structure. Compounds of general formula I are useful in treatment or prevention of cancer.

## Description

### Field of Art

The present disclosure relates to cyclic dinucleotides and their conjugates with antibodies that may be useful in the treatment of diseases in which modulation of STING adaptor protein (STING = Stimulator of Interferon Genes) is beneficial.

### Background Art

The innate immune system recognizes the presence of pathogens or disruptions in host homeostasis through a variety of Pattern Recognition Receptors (PRRs). These receptors detect a limited set of ligands associated with pathogens or damage, commonly referred to as Pathogen or Damage-Associated Molecular Patterns (PAMPs and DAMPs) (Takeuchi O et al., Cell, 2010:140, 805-820). Over the past three decades, several PRRs have been identified, including Toll-like receptors, retinoic acid-inducible gene (RIG-I)-like receptors, nucleotide-binding oligomerization domain-like (NOD) receptors, C-type lectin receptors, and cytosolic DNA sensors (Brubaker SW et al., Annu Rev Immunol, 2015:33, 257-290). Recognition of PAMPs and DAMPs by PRRs leads to the upregulation of cytokines and chemokines, including interferons, and recruits immune cells to sites of infection or damage. These processes contribute to the development of antitumoral adaptive immunity.

In healthy cells, cellular DNA is typically confined to the nucleus and mitochondria. The presence of DNA in the cytosol is therefore an indicator of either pathogenic activity or disruption in host homeostasis. Cytosolic DNA sensing is initiated by various DNA sensors, such as DNA-dependent activator of IRFs (DAI), cyclic guanosine-adenosine synthase (cGAS), DEAD-box polypeptide 41 (DDX41), and absent in melanoma 2 (AIM2) (Unterholzner L, Immunology, 2013: 218, 1312-1321). cGAS serves as the primary sensor of cytosolic DNA; upon DNA binding, it synthesizes a second messenger, cyclic dinucleotide (CDN), called 2'3'-cGAMP (Zhang et al., Molecular Cell, 2013:51, 226-235). This CDN activates STING (Stimulator Of Interferon Genes, also known as TMEM173, MITA, ERIS), which in turn triggers the expression of type I and III interferons and proinflammatory cytokines via TBK1/IKK kinases and transcription factors IRF3, STAT6, and NF-kB (Burdette et al., Nature, 2011: 478, 515-518; Motwani et al., Nat Rev Genet, 2019: 20, 657-674).

CDNs that have an affinity for STING contain two purine nucleotide monophosphates linked with either two 3'-5' (3'3'-CDNs), two 2'-5' (2'2'-CDNs), or a combination of 2'-5' and 3'-5' phosphodiester bonds (2'3'-CDNs). The prototype 2'3' cGAMP (c[G(2',5')pA(3',5')p]) is synthesized by the cGAS enzyme, while 3'3' cGAMP, 3'3' c-diAMP, and 3'3' c-diGMP are produced by bacterial dinucleotide cyclases (Zhang et al., Molecular Cell, 2013:51, 226-235; Novotna et al., Biochemistry, 2021:60, 3714-3727). Cyclic dinucleotides (CDNs) are believed to foster the priming of both cellular and humoral immunity. For instance, type I IFNs, whose expression is triggered by STING activation, have shown promise in the treatment of various human cancers (Cohen et al., N Engl J Med, 2005:353, 2477-2490; Tsao et al., N Engl J Med, 2004:351, 998-1012). They can directly inhibit tumor cell proliferation and demonstrate synergy with many approved anticancer agents. Furthermore, type I IFNs can act on immune cells to trigger antitumor responses (Musella et al., Oncoimmunology, 2017:6, e1314424). In mouse models, type I IFN signaling was found to be essential for tumor-initiated T-cell priming. Animals lacking the IFN-α/β receptor in dendritic cells failed to reject immunogenic tumors and showed defects in antigen cross-presentation to CD8+ T cells (Fuertes et al., J Exp Med, 2011:208, 2005-2016; Diamond et al., J Exp Med, 2011:208, 1989-2003). Consistent with these findings, intratumoral injections of STING agonists have been shown to induce regression of established tumors in mice, generating substantial systemic immune responses capable of rejecting distant metastases and providing long-lasting immunological memory (Corrales et al., Cell Rep, 2015:11, 1018-1030).

### Disclosure of the Invention

In one aspect, the invention relates to compounds of formula I: 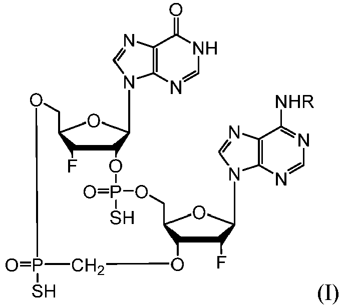 wherein
R is selected from H, 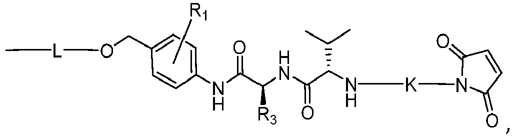 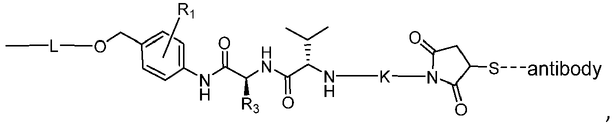 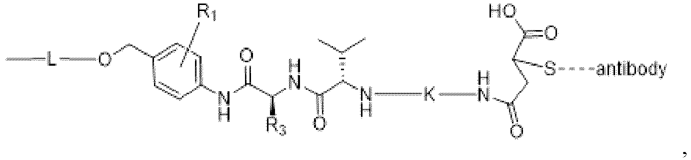 and 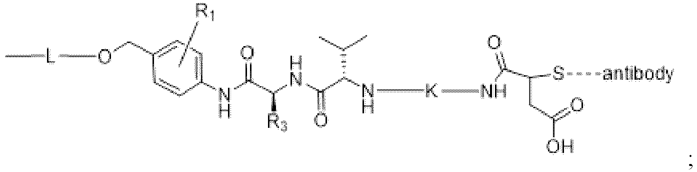 wherein
R1 is selected from H, F, and Cl;
R3 is selected from C₁-C₆ alkyl, and amino acid side chains;
-L- is selected from -C(O)- and -C(O)-O-CH₂-CH₂-N(CH₃)-C(O)-;
-K- is selected from -C(O)-(CH₂)ₘ-, -C(O)-(CH₂CH₂O)ₙ-CH₂CH₂-, -C(O)-(CH₂CH₂O)ₙ-CH₂CH₂NH-C(O)-(CH₂)ₚ-, -C(O)-(CH₂)ₘ-NH-C(O)-(CH₂)ₚ-, -C(O)-(CH₂)ₘ-N(CH₃)-C(O)-(CH₂)ₚ-;
wherein m=1-8, n=1-8, p=1-5;
or a pharmaceutically acceptable salt or solvate or formulation thereof.

In some embodiments, R1 is preferably H.

In some embodiments, R1 is preferably F or Cl. In these embodiments, R1 increases the plasma stability of the compounds.

Preferably, R3 is selected from Ci-Ce alkyl (preferably methyl), -(CH₂)₃-NH-C(O)-NH₂.

In some preferred embodiments, -K- is -C(O)-(CH₂)ₘ-.

In some embodiments, m is 1 to 5.

In some embodiments, p is 1.

In some embodiments, C₁-C₆ alkyl refers to linear or branched saturated alkyl chain. Examples include methyl, ethyl, propyl, isopropyl.

In some embodiments, "amino acid sidechain" refers to a side chain of an amino acid, said side chain being preferably selected from CH₃-, HN=C(NH₂)-NH-(CH₂)₃-, H₂N-CO-CH₂-, HOOC-CH₂-, HS-CH₂-, H₂N-CO-(CH₂)₂-, HOOC-(CH₂)₂-, H-, CH₃-CH₂-CH(CH₃)-, (CH₃)₂-CH-CH₂-, H₂N-(CH₂)₄-, CH₃-S-(CH₂)₂-, Ph-CH₂-, HO-CH₂-, CH₃-CH(OH)-, HO-Ph-CH₂-, (CH₃)₂-CH-, -(CH₂)₃-NH-C(O)-NH₂,

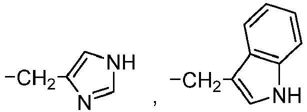

In a second aspect, the present invention relates to a compound of formula II, and its pharmaceutically acceptable salts or solvates:

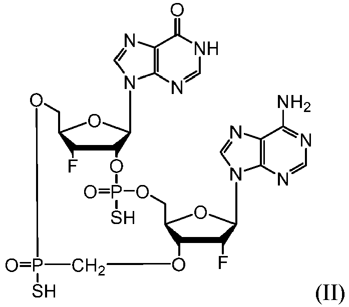

The compound of formula II is a cyclic dinucleotide bearing one phosphorothioate group and one phosphonothioate group. Within the scope of the invention, cyclic dinucleotides were found to exhibit varying activation abilities for different STING haplotypes. Only a limited number of cyclic dinucleotides exhibited activity against all STING haplotypes identified in humans. Compound of formula II demonstrated a potent activatory activity against all STING haplotypes. Furthermore, it has been found that the activity of antibody-CDN conjugates containing phosphorothio/phosphonothio linkages was significantly improved compared to analogs containing phosphodiester/phosphonoester linkages *in vivo.* Moreover, it was found that it is advantageous to limit the number of free hydroxy and amino groups on the cyclic dinucleotide to a minimum in order to facilitate the synthesis of CDN conjugates. The compound of formula II complies with all these requirements.

In a third aspect, the invention relates to compounds of general formula III 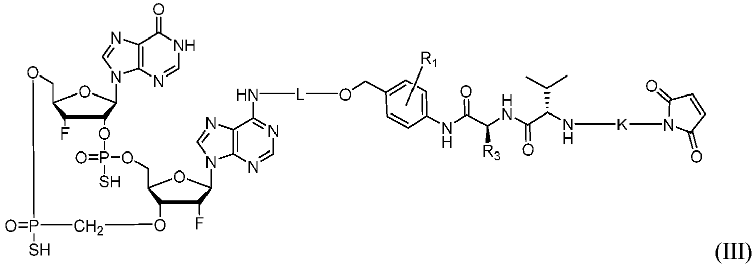 and their pharmaceutically acceptable salts and solvates,
wherein R1, R3, L and K are as defined herein above.

The conjugates of formula III contain an active moiety corresponding to the compound of formula II and a linker moiety allowing to bind the active moiety to a targeting carrier such as an antibody. Within the framework of the invention, it was found that binding the linker moiety via the thiol group on phosphorus in the parent compound II leads to a significant decrease in plasma stability of antibody CDN cojugates, and presents challenges in synthesis such as difficult resolution of regiomers and low yields. It is therefore necessary to bind the linker moiety via the adenine base.

The linker moieties are designed to allow for the targeting of the active moiety, to provide a sufficient stability of the conjugate and/or to enable a controlled release of the active moiety at the site of action. The linker connects the CDN and the antibody through a covalent linkage. Within the framework of the invention, the linker part is a cleavable linker comprising one or more cleavable elements. Each cleavable element is independently selected from spacer -L- containing a self-immolative linker or carbonyl group; an enzymatically-cleavable peptide linker containing preferably PAB-Cit-Val or PAB-Ala-Val spacer; and linker part -K- containing alkyl and/or PEG spacer which is connected to maleimide group. Maleimide group is typically suitable for a coupling with reactive thiol moieties of antibodies in antibody-drug conjugates.

In some preferred embodiments, the invention relates to compounds of formula 3.3-3.10 and 3.13-3.18, and their pharmaceutically acceptable salts and solvates.

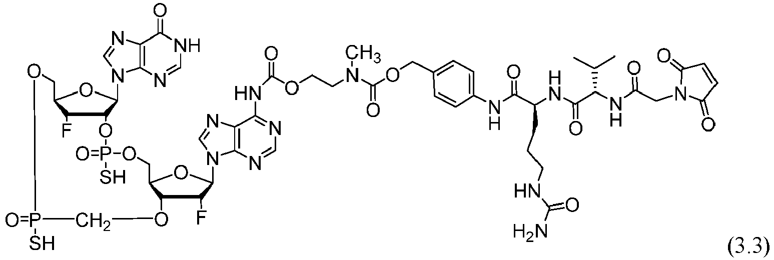

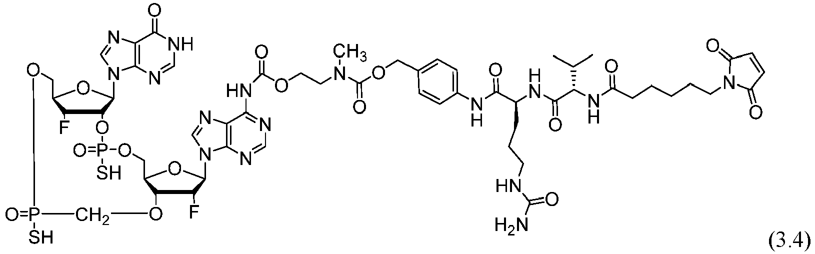

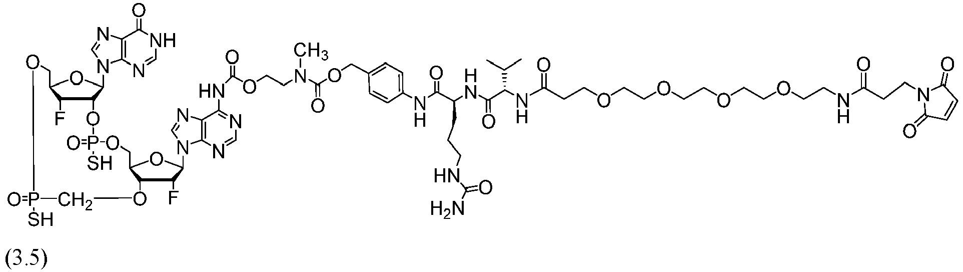

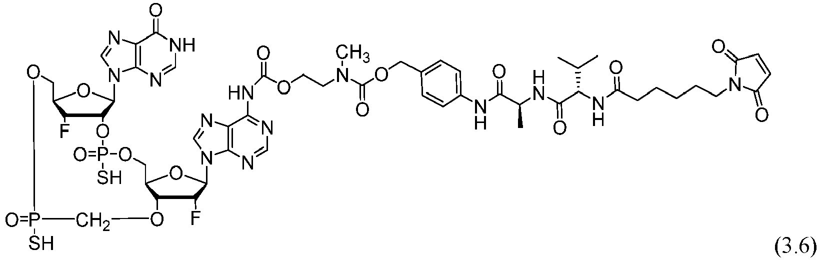

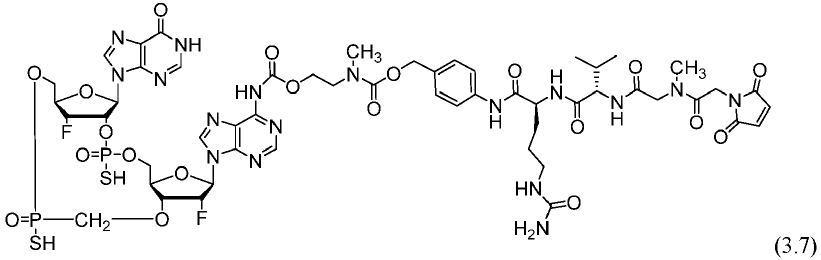

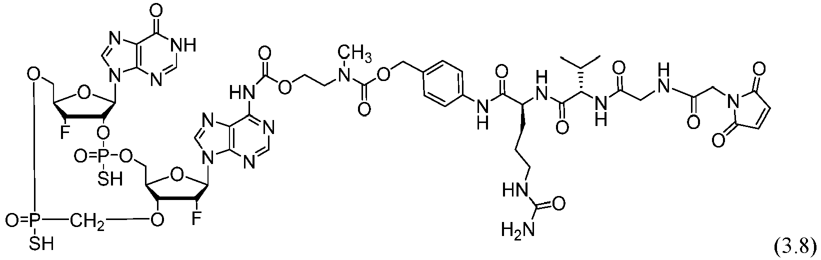

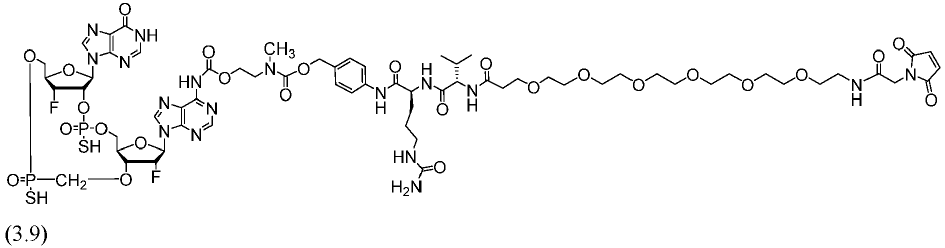

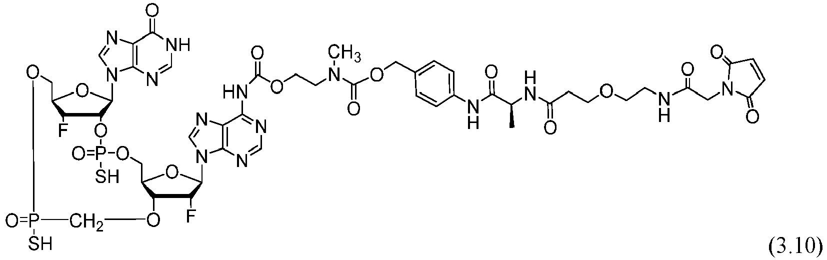

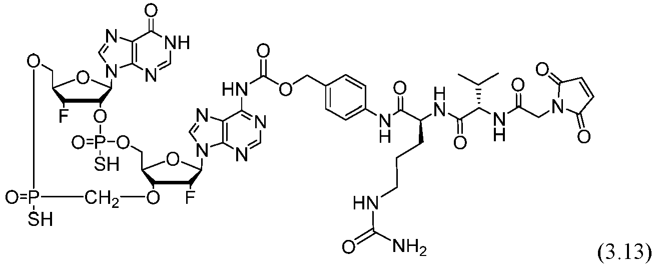

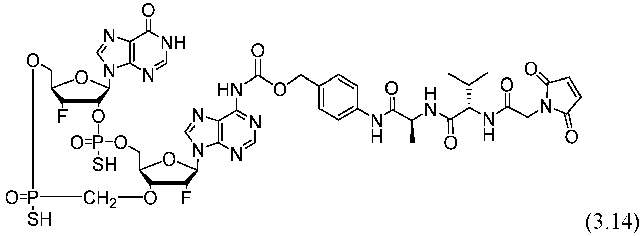

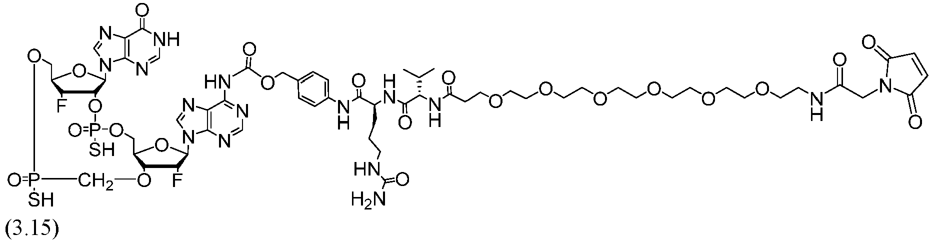

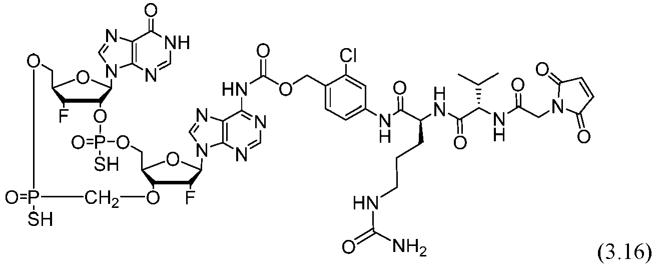

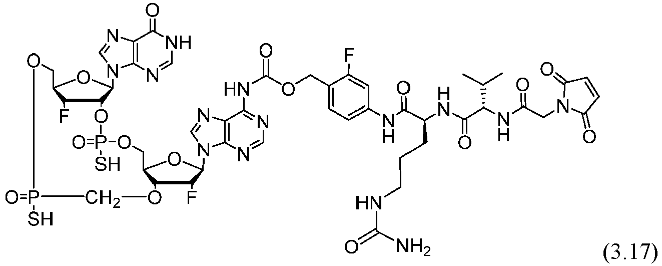

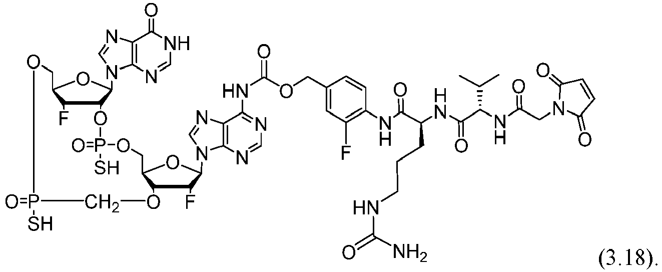

In a fourth aspect, the invention relates to compounds of formula IV, V or VI, and their pharmaceutically acceptable salts and solvates,
wherein R1, R3, L and K are as defined herein above:

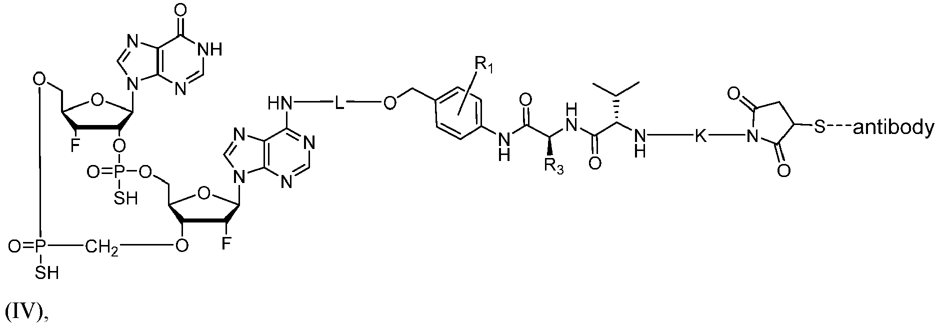

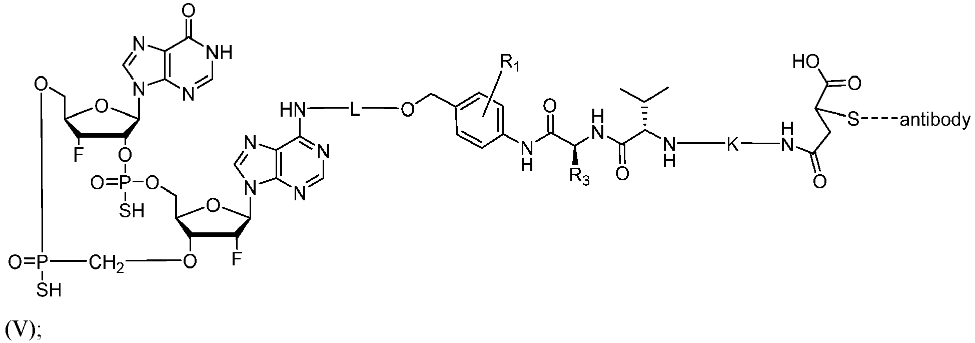

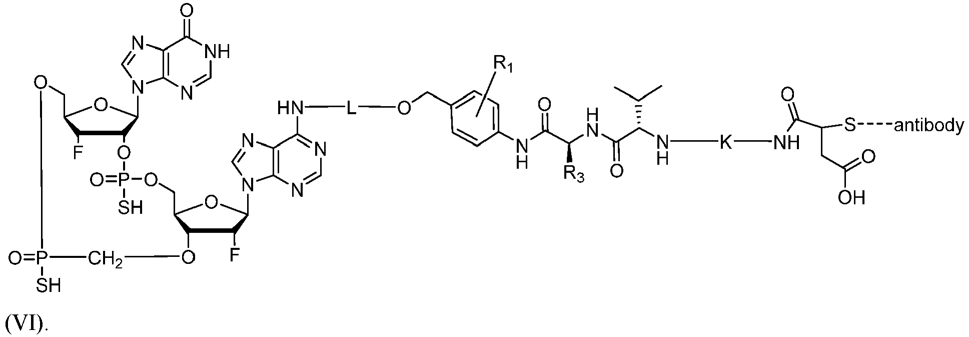

In the compounds of formulas IV, V, VI the active moiety is bound via the linker to an antibody to form an antibody-CDN conjugate. The antibody is bound via its cysteine residue. "-S-antibody" thus refers to an antibody bound via a cysteine residue.

The antibody serves to target the active moiety as desired and/or to increase the therapeutic effect by increasing the amount of the active moiety at the site of the action. It is thus preferred if the target antigen of the antibody is a tumor antigen.

It was found that the antibodies significantly improve pharmacokinetic properties of compounds of general formula (II), such as half-life in the organism (half-life in mice is <10 minutes for compounds of general formula (II), and more than 9 days when the compound of general formula (II) is conjugated to antibody via the linker as shown in formulas (IV), (V), (VI)).

As used herein, the term "antibody" refers to an immunoglobulin molecule that specifically binds to a particular target antigen. Antibodies may be of human or non-human origin. In some embodiments, the antibodies may be humanized. The antibodies may be polyclonal, monoclonal, genetically engineered, and/or otherwise modified in nature. The antibodies composing the antibody CDN conjugates of the disclosure are suitable for administration to humans, for example, as humanized antibodies or fully human antibodies.

Antibodies composing antibody CDN conjugates of the disclosure may be in the form of full-length antibodies that may be of any antibody isotype, or derived from any antibody isotype, including for example, IgA, IgD, IgE, IgG, IgM, or IgY. In some embodiments, the antibody present in the antibody-CDN conjugates is an IgG (e.g., IgG1, IgG2, IgG3, or IgG4). In some embodiments, the antibodies comprise all or a portion of a constant region of an antibody.

In some embodiments, the antibody comprises a modified Fc region.

The antibody is preferably a monoclonal antibody.

The antibody is typically bound to the linker via a cysteine residue.

The antibody is preferably engineered to contain at least one cysteine in the heavy or light chain portion, preferably at positions 110 (light chain) and 152, 375 (heavy chain, EU numbering). Such modifications are known in the art as Thiomab technology and are described, for example, in WO 2006/034488 A2.

Antibodies may preferably be conjugated to the compounds of formulas 3.3-3.10 and 3.13-3.18 to form the antibody CDN conjugates of formulas (IV), (V), (VI), respectively.

The antibodies present in the antibody-CDN conjugates of formulas (IV), (V) or (VI) as contemplated in the present disclosure specifically bind to one or more cancer related tumor or immune cell associated antigens. Such antibodies or manners of obtaining them are known to a person skilled in the art.

The antigens are preferably Her2 (aliases ERBB2, CD340, HER-2, HER-2/neu), TROP2 (aliases TACSTD2, EGP-1, EGP1, GA733-1, GA7331, GP50, M1S) and FOLR1 (aliases FBP, FOLR, Folate receptor 1, folate receptor alpha).

Solvates of compounds of the invention are entities containing the compound and a molecule of solvent, preferably of a pharmaceutically acceptable solvent. Solvates include hydrates, i.e., solvates with water. Further solvents forming the solvates may include water, alcohols and polyalkylene glycols, in particular water, methanol, ethanol, n-propanol, isopropanol, n-butanol, isobutanol, t-butanol, polyethylene glycol, polypropylene glycol. Solvates formed with water may also be called hydrates.

Pharmaceutically acceptable salts are formed by a compound of the invention and an acid and/or a base. Pharmaceutically acceptable bases include cations of ammonia, aluminum, arginine, benzathine, calcium, chloroprocaine, choline, diethanolamine, ethanolamine, ethylenediamine, lysine, magnesium, histidine, lithium, meglumine, potassium, procaine, sodium, triethylamine, zinc.

Pharmaceutically acceptable acid salts include acetate, aspartate, benzenesulfonate, benzoate, besylate, bicarbonate, bitartrate, bromide, camsylate, carbonate, chloride, citrate, decanoate, edetate, esylate, fumarate, gluceptate, gluconate, glutamate, glycolate, hexanoate, hydroxynaphthoate, iodide, isethionate, lactate, lactobionate, malate, maleate, mandelate, mesylate, methylsulfate, mucate, napsylate, nitrate, octanoate, oleate, pamoate, pantothenate, phosphate, polygalacturonate, propionate, salicylate, stearate, succinate, sulfate, tartrate, teoclate, tosylate, trifluoroacetate.

Compounds of the invention may be in the form of enantiomers or mixtures thereof, such as racemate mixtures.

In a fifth aspect, the invention relates to the compounds of formula (I), (II), (IV), (V) and/or (VI) in the treatment or prevention cancers.

Activation of the cGAS-STING pathway induces the expression of type I and III interferons as well as cytokines. This is achieved through the activation of downstream transcription factors, specifically STAT6 and IRF3 via TBK1 kinase, and NF-κB through IKKβ kinase. It is generally believed that this cascade ultimately results in the release of a variety of signaling molecules, including type I and III interferons, as well as cytokines like IL-6, TNF-α. Chemokines such as IP10 and RANTES are also released.

The present invention also provides a method to induce a STING adaptor protein-dependent response in humans or animals. This involves administering a therapeutically effective amount of the compound of formula (I), (II), (IV), (V) and/or (VI), or its pharmaceutically acceptable salt, solvate or formulation.

Consequently, the compound formula (I), (II) and/or the immunoconjugate of formula (IV), (V) and/or (VI), or the cleavage product thereof, has STING agonist activity if it binds to STING and is able to stimulate production of one or more STING-dependent cytokines selected from interferon, type 1 interferon, IFN-α, IFN-β, type 3 interferon, IFNX, IP10, TNF, IL-6, CXCL9, CCL4, CXCL11, CCL5, CCL3, or CCL8.

Cancers that can be treated or prevented by the methods of the disclosure include solid tumors and lymphomas, including but not limited to adrenal cancer, bladder cancer, bone cancer, brain cancer, breast cancer, colon cancer, colorectal cancer, eye cancer, head-and-neck cancer, kidney cancer such as renal cell carcinoma, liver cancer, lung cancer such as non-small cell lung cancer, ovarian cancer, pancreatic cancer, prostate cancer, skin cancer such as squamous cell carcinoma and melanoma, thyroid cancer, uterine cancer, vaginal cancer, and myeloma such as multiple myeloma. The cancer can be naive, or relapsed and/or refractory.

In some embodiments, the cancer is Burkitt's lymphoma, Hodgkin's lymphoma, non-Hodgkin's lymphoma (NHL), indolent non-Hodgkin's lymphoma (iNHL), refractory iNHL, multiple myeloma (MM), chronic myeloid leukemia (CML), acute lymphocytic leukemia (ALL), B-cell ALL, acute myeloid leukemia (AML), chronic lymphocytic leukemia (CLL), small lymphocytic lymphoma (SLL), myelodysplastic syndrome (MDS), myeloproliferative disease (MPD), mantle cell lymphoma (MCL), follicular lymphoma (FL), Waldestrom's macroglobulinemia (WM), T-cell lymphoma, B-cell lymphoma, diffuse large B-cell lymphoma (DLBCL), or marginal zone lymphoma (MZL). In one embodiment, the cancer is minimal residual disease (MRD). In some embodiments, the cancer is selected from Hodgkin's lymphoma, non-Hodgkin's lymphoma (NHL), indolent non-Hodgkin's lymphoma (iNHL), and refractory iNHL. In some embodiments, the cancer is indolent non-Hodgkin's lymphoma (iNHL). In some embodiments, the cancer is refractory iNHL. In some embodiments, the cancer is chronic lymphocytic leukemia (CLL). In some embodiments, the cancer is diffuse large B-cell lymphoma (DLBCL).

In some embodiments, the cancer is a solid tumor selected from the group consisting of pancreatic cancer; bladder cancer; colorectal cancer; breast cancer, including metastatic breast cancer; prostate cancer, including androgen-dependent and androgen-independent prostate cancer; kidney or renal cancer, including, *e.g.*, metastatic renal cell carcinoma; hepatocellular cancer; lung cancer, including, *e.g*., non-small cell lung cancer (NSCLC), bronchioloalveolar carcinoma (BAC), and adenocarcinoma of the lung; ovarian cancer, including, *e.g.*, progressive epithelial or primary peritoneal cancer; cervical cancer; gastric cancer; esophageal cancer; head and neck cancer, including, *e.g*., squamous cell carcinoma of the head and neck; melanoma; neuroendocrine cancer, including metastatic neuroendocrine tumors; brain tumors, including, *e.g*., glioma, anaplastic oligodendroglioma, adult glioblastoma multiforme, and adult anaplastic astrocytoma; bone cancer; and soft tissue sarcoma, hepatic carcinoma, rectal cancer, penile carcinoma, vulval cancer, thyroid cancer, salivary gland carcinoma, endometrial or uterine carcinoma, hepatoma, hepatocellular cancer, liver cancer, gastric or stomach cancer including gastrointestinal cancer, cancer of the peritoneum, squamous carcinoma of the lung, gastroesophagal cancer, biliary tract cancer, gall bladder cancer, colorectal/appendiceal cancer, squamous cell cancer (*e.g*., epithelial squamous cell cancer).

In a sixth aspect, the invention relates to pharmaceutical composition comprising a compound of formula (I), (II), (IV), (V) and/or (VI), or a pharmaceutically acceptable salt, solvate or formulation thereof, and at least one pharmaceutically acceptable excipient.

Pharmaceutical compositions comprising the compounds disclosed herein, or pharmaceutically acceptable salts, solvates or formulations thereof, may be prepared with one or more pharmaceutically acceptable excipients which may be selected in accordance with ordinary practice. Tablets may contain excipients including glidants, fillers, binders and the like. Aqueous compositions may be prepared in sterile form, and when intended for delivery by other than oral administration generally may be isotonic. All compositions may optionally contain excipients such as those set forth in the Rowe et al, Handbook of Pharmaceutical Excipients, 6th edition, American Pharmacists Association, 2009. Excipients can include ascorbic acid and other antioxidants, chelating agents such as EDTA, carbohydrates such as dextrin, hydroxyalkylcellulose, hydroxyalkylmethylcellulose, stearic acid and the like. In certain embodiments, the composition is provided as a solid dosage form, including a solid oral dosage form. The compositions include those suitable for various administration routes, including oral administration. The compositions may be presented in unit dosage form and may be prepared by any of the methods well known in the art of pharmacy. Such methods include the step of bringing into association the active ingredient (*e.g*., a compound of the present disclosure or a pharmaceutical salt thereof) with one or more pharmaceutically acceptable excipients. The compositions may be prepared by uniformly and intimately bringing into association the active ingredient with liquid excipients or finely divided solid excipients or both, and then, if necessary, shaping the product. Techniques and formulations generally are found in Remington: The Science and Practice of Pharmacy, 21st Edition, Lippincott Wiliams and Wilkins, Philadelphia, Pa., 2006.

In some embodiments, the compound of formula (II) and/or immunoconjugates of formula (IV), (V) and/or (VI) are administered to the subject parenterally, preferably intravenously, intratumorally, intramuscularly or subcutaneously.

The present application also discloses use of, in particular, compound of formula (II) and/or an immunconjugate of formula (IV), (V) and/or (VI), a pharmaceutical composition thereof, in combination with one or more additional therapeutic agents for the treatment of cancer in a subject in need thereof.

The compound of formula (II) and/or immunconjugates of formula (IV), (V) and/or (VI) can be used in combination with one or more additional therapeutic agents. In a particular embodiment, the compound of formula (II) and/or immunconjugates of formula (IV), (V) and/or (VI) can be used in combination with an immune checkpoint inhibitor. For instance, the compound of formula (II) and/or immunconjugate of formula (IV), (V) and/or (VI) can be administered with an inhibitor of PD-1, PD-L1, or CTLA-4, or a combination thereof.

### Brief Description of Drawings

Figure 1. In vivo testing of anti-HER2 mAb1 conjugated with Compounds 3.4 or 3.11 in Her2 expressing HCC1954 breast tumor xenograft model.
Figure 2. Effect of intratumoral injection of Compound 2.2 on tumor growth, induction of tumor specific CD8 T cells and immunological memory in CT26 mouse tumor model.
Figure 3. Release of the payload CDN 2.2 from anti Her2 mAb2 CDN conjugates in mouse plasma in vitro.
Figure 4. Release of the payload CDN 2.2 from anti TROP2 mAb5 CDN conjugates in mouse plasma in vitro.
Figure 5. Release of the payload CDN 2.2 from anti-HER2 mAb2 or anti-TROP2 mAb5 conjugated with Compounds 3.3, 3.4, or 3.13 in vivo and in vivo pharmacokinetic of Compound 2.2.
Figure 6. Comparative analysis of in vivo mouse plasma cytokine induction by anti-Her2 mAb2 conjugated with the compound 3.3 at the inter-chain Cys sites and anti-HER2 mAb3 conjugated with the compound 3.3 at engineered light chain C110 and heavy chain C375 sites.
Figure 7. Comparative analysis of in vivo mouse plasma cytokine induction by anti-TROP2 mAb4 conjugated with the compound 3.3 at the inter-chain Cys sites, anti-TROP2 mAb5 conjugated with compound 3.3 at engineered light chain C110 and heavy chain C375 sites and the warhead CDN 2.2.
Figure 8. Activity of anti-HER2 mAb3 conjugated with Compound 3.3 to light chain C110 and heavy chain C375 in Her2 expressing HCC1954 breast tumor xenograft model.
Figure 9. Activity of anti-TROP2 mAb5 conjugated with Compound 3.3 to engineered light chain C110 and heavy chain C375 in TROP2 expressing HCC1954 breast tumor xenograft model.
Figure 10. Activity of anti-TROP2 mAb5 conjugated with Compound 3.3 to engineered light chain C110 and heavy chain C375 in TROP2 expressing Bx-PC3 prostate tumor xenograft model.
Figure 11. Activity of anti-TROP2 mAb5 conjugated with Compound 3.3 to engineered light chain C110 and heavy chain C375 in TROP2 expressing Colo205 colon tumor xenograft model.
Figure 12. Intratumoral cytokines induced in TROP2 positive HCC1954 mouse model treated with anti-TROP2 mAb5 conjugated with Compound 3.3 to engineered light chain C110 and heavy Chain C375.
Figure 13. Activity of anti-HER2 mAb3 conjugated with Compound 3.13 to engineered light chain C110 and heavy chain C375 in Her2 expressing HCC1954 breast tumor xenograft model.
Figure 14. Activity of anti-TROP2 mAb5 conjugated with Compound 3.13 to engineered light chain C110 and heavy chain C375 in Her2 expressing HCC1954 breast tumor xenograft model.
Figure 15. Activity of anti-FOLR1 mAb6 conjugated with Compound 3.3 to engineered heavy chain C152 and C375 in FOLR1 expressing KB cervical tumor xenograft model.

### Examples of carrying out the Invention

### Abbreviations

- A: adenosine unit (NMR description)
- ACN: acetonitrile
- AcOH: acetic acid
- Bz: benzoyl
- BOC-Val-OH: *N*-(*tert*-butoxycarbonyl)-L-valine
- DBU: 1,8-diazabicyclo[5.4.0]undec-7-ene
- DCM: dichloromethane
- DEA: diethylamine
- DIPEA: *N*,*N*-diisopropylethylamine
- DMF: dimethylformamide
- DMOCP: 2-chloro-5,5-dimethyl-1,3,2-dioxaphosphorinane 2-oxide
- DMTr: dimethoxytrityl
- Et₃SiH: triethylsilane
- EtOH: ethanol
- FMOC-Cit-OH: *N-*alpha-(9-fluorenylmethyloxycarbonyl)-L-citrulline
- HATU: 1-[bis(dimethylamino)methylene]-1*H*-1,2,3-triazolo[4,5-*b*]pyridinium 3-oxid hexafluorophosphate
- HOAt: 1-hydroxy-7-azabenzotriazole
- Hpx: inosine unit (NMR description)
- LiAlH₄: lithium aluminium hydride
- Mal: maleimide unit (NMR description)
- mAb1-3.3: Antibody Drug Conjugate, Antibody 1 conjugated with Compound 3.3
- MeNH₂: methylamine
- MeOH: methanol
- NH₄OA_{C}: ammonium acetate
- (O₂NC₆H₄O)₂CO: bis(4-nitrophenyl)carbonate
- PEG: polyethylene glycol linker (NMR description)
- PhOCOCl: phenyl chloroformate
- +Psi reagent: (2*R*,3a*R*,6*S*,7a*R*)-3a-methyl-2-((perfluorophenyl)thio)-6-(prop-1-en-2-yl)hexahydrobenzo[*d*][1,3,2]oxathiaphosphole 2-sulfide
- PyBroP: Bromo-tris-pyrrolidino-phosphonium hexafluorophosphate
- r.t.: room temperature
- SE: self-immolative linker (NMR description)
- TEA: triethylamine
- TEAB: triethylammonium bicarbonate buffer
- TFA: trifluoroacetic acid
- THF: tetrahydrofuran
- TMS-Cl: chlorotrimethylsilane

### Analytical methods

| **LC-MS Method** | **Instrument** | **Time (min)** | **Solvents A-B** | | **Flow (mL/min)** | **Column and solvent system** |
|---|---|---|---|---|---|---|
| **A1** | Waters Acquity H class plus with SQ Detector 2 | 0.0 | 100 | 0 | 0.50 | Acquity BEH C18 (50 mm × 2.1 mm, 1.7 µm) |
| | | 4.0 | 0 | 100 | 0.50 | |
| | | | | | | A: 10 mM NH₄OAc in H₂O |
| | | | | | | B: ACN |

| **Prep-HPLC Method** | **Instrument** | **Time (min)** | **Solvents A/B** | | **Flow (mL/min)** | **Column and solvent system** |
|---|---|---|---|---|---|---|
| **P1** | Ingos LCP5080 with UV monitor 254 | 0 | 100 | 0 | 30 | LUNA C18 (10 µm, 200 × 90 mm) |
| | | 10 | 100 | 0 | 30 | A: 0.1M TEAB in H₂O |
| | | 70 | 70 | 30 | 30 | B: ACN |
| **P2** | Ingos LCP5020 with LCD5000 | 0 | 100 | 0 | 10 | LUNA C18 (5 µm, 250 × 21 mm) |
| | | 5 | 100 | 0 | 10 | A: 0.1M TEAB in H₂O |
| | | 65 | 50 | 50 | 10 | B: ACN |
| **P3** | Ingos LCP5020 with LCD5000 | 0 | 100 | 0 | 10 | Kinetex PFP (5 µm, 250 × 21 mm) |
| | | 5 | 100 | 0 | 10 | A: 0.05% TFA in H₂O |
| | | 65 | 75 | 25 | 10 | B: ACN |
| **P4** | Ingos LCP5020 with LCD5000 | 0 | 100 | 0 | 10 | Kinetex PFP (5 µm, 250 × 21 mm) |
| | | 5 | 100 | 0 | 10 | A: 0.05% TFA in H₂O |
| | | 65 | 50 | 50 | 10 | B: ACN |
| **P5** | Ingos LCP5020 with LCD5000 | 0 | 90 | 10 | 10 | Luna C18 (5 µm, 250 × 21 mm) |
| | | 50 | 50 | 50 | 10 | A: 0.1% TFA in H₂O |
| | | | | | | B: ACN |
| **P6** | Ingos LCP5020 with LCD5000 | 0 | 100 | 0 | 10 | Kinetex PFP (5 µm, 250 × 21 mm) |
| | | 5 | 100 | 0 | 10 | |
| | | 65 | 62 | 38 | 10 | A: 0.05% TFA in H₂O |
| | | | | | | B: ACN |
| **P7** | Ingos LCP5020 with LCD5000 | 0 | 100 | 0 | 10 | Luna C18 (5 µm, 250 × 21 mm) |
| | | 40 | 50 | 50 | 10 | A: H₂O |
| | | | | | | B: ACN |

Unless stated otherwise, all used solvents were anhydrous. Mass spectra were recorded on Acquity H class plus UPLC (Waters) and on LTQ Orbitrap XL (Thermo Fisher Scientific) using ESI ionization. Unless stated otherwise, the NMR spectra were measured on Bruker AVANCE III 600 instrument (¹H, ¹³C) with a cryoprobe, and Bruker AVANCE III 500 instrument with a cryoprobe (³¹P, ¹⁹F). The structural assignment of the proton and carbon signals was achieved using a combination of ID and 2D NMR techniques (H,H-COSY, H,C-HSQC, H,C-HMBC).

### Example 1 - Synthesis of linkers

### Example 1.1

### 4-((S)-2-((S)-2-(2-(2,5-dioxo-2,5-dihydro-1H-pyrrol-1-yl)acetamido)-3-methylbutanamido)-5-ureidopentanamido)benzyl (4-nitrophenyl) carbonate (1.1)

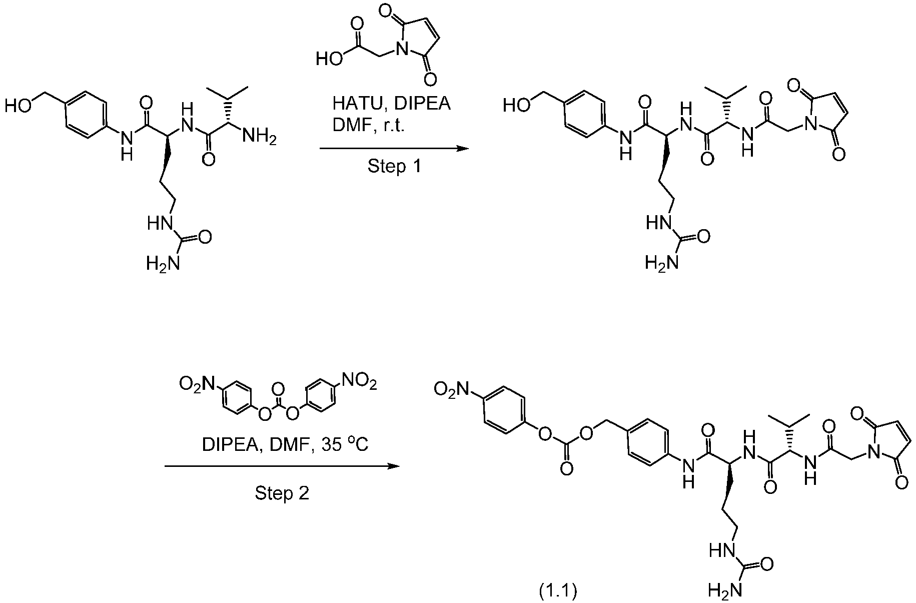

*Step 1:* HATU (228 mg; 0.6 mmol) was added to a mixture of 2-(2,5-dioxo-2,5-dihydro-1*H-*pyrrol-1-yl)acetic acid (78 mg; 0.5 mmol) and DIPEA (0.17 mL; 1.0 mmol) in DMF (4 mL) and the reaction mixture was stirred for 15 minutes at r.t. After that, ValCitPAB (0.15 g; 0.4 mmol) was added and the reaction mixture was stirred for 2 hours at r.t. The reaction mixture was evaporated. The product was purified by reverse phase chromatography (Method P5) to yield 0.15 g (73 %) of (*S*)-2-((*S*)-2-(2-(2,5-dioxo-2,5-dihydro-1*H*-pyrrol-1-yl)acetamido)-3-methylbutanamido)-*N*-(4-(hydroxymethyl)phenyl)-5-ureidopentanamide.
UPLC-MS (Method A1): Rt = 1.85 min; m/z 517.2 [M+H]⁺.

*Step 2:* Bis(4-nitrophenyl)carbonate (97 mg; 0.32 mmol) was added to a mixture of (*S*)-2-((*S*)-2-(2-(2,5-dioxo-2,5-dihydro-1*H*-pyrrol-1-yl)acetamido)-3-methylbutanamido)-*N*-(4-(hydroxymethyl)phenyl)-5-ureidopentanamide (83 mg; 0.16 mmol) and DIPEA (56 µL; 0.32 mmol) in DMF (1.6 mL) and the reaction mixture was stirred for 5 hours at 35 °C. The mixture was precipitated with diethyl ether and the precipitate was collected by centrifugation. The product was lyophilized from dioxane to yield 80 mg (74 %) of 4-((*S*)-2-((*S*)-2-(2-(2,5-dioxo-2,5-dihydro-1*H*-pyrrol-1-yl)acetamido)-3-methylbutanamido)-5-ureidopentanamido)benzyl (4-nitrophenyl) carbonate.
UPLC-MS (Method A1): Rt = 3.01 min; m/z 682.2 [M+H]⁺.
¹H NMR (401 MHz, DMSO-d6) δ 10.10 (s, 1H), 10.08 (s, 1H), 8.39 - 8.16 (m, 4H), 7.70 - 7.60 (m, 2H), 7.60 - 7.52 (m, 2H), 7.47 - 7.35 (m, 2H), 7.08 (d, J = 1.1 Hz, 2H), 5.98 (t, J = 5.9 Hz, 1H), 5.42 (s, 2H), 5.37 (s, 1H), 5.24 (s, 2H), 4.39 (p, J = 7.2 Hz, 1H), 4.26 (dd, J = 8.8, 6.5 Hz, 1H), 4.13 (s, 2H), 3.57 (s, 1H), 2.98 (dh, J = 25.9, 6.5 Hz, 2H), 1.98 (h, J = 6.7 Hz, 1H), 1.75 - 1.52 (m, 1H), 1.50 - 1.29 (m, 1H), 0.92 - 0.77 (m, 7H). ¹³C NMR (101 MHz, DMSO-d6) δ 171.30, 171.18, 166.54, 159.38, 155.76, 152.44, 145.65, 139.89, 135.32, 129.98, 129.73, 125.89, 123.10, 119.48, 70.74, 66.83, 57.94, 53.74, 31.45, 29.58, 27.33, 19.59, 18.45.

### Example 1.2

### 4-((S)-2-((S)-2-(2-(2-(2,5-dioxo-2,5-dihydro-1H-pyrrol-1-yl)acetamido)acetamido)-3-methylbutanamido)-5-ureidopentanamido)benzyl (4-nitrophenyl) carbonate (1.2)

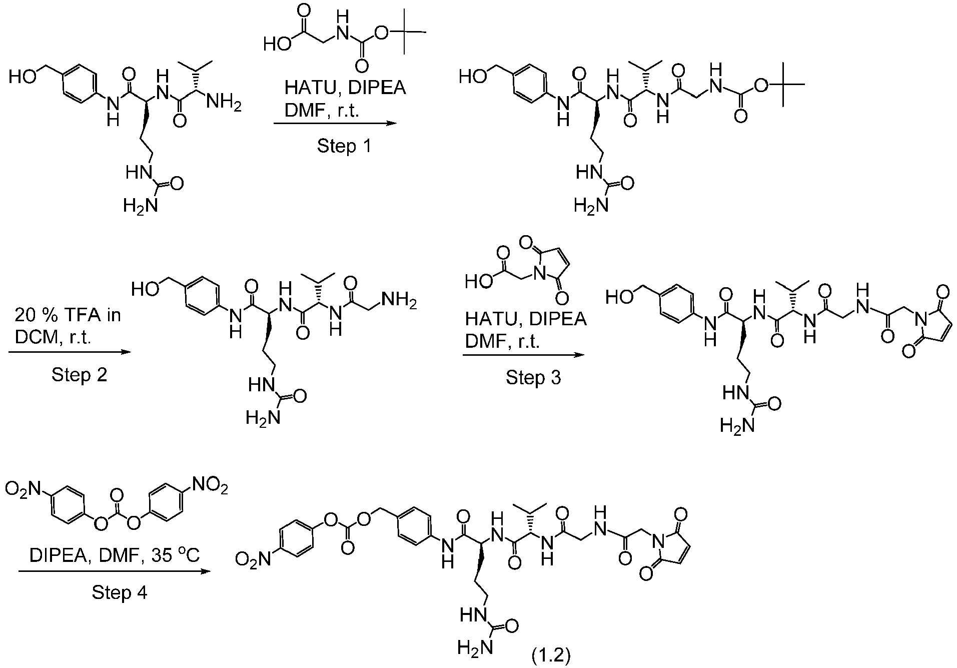

*Step 1:* HATU (228 mg; 0.6 mmol) was added to a mixture ofBOC-Gly-OH (70 mg; 0.4 mmol) and DIPEA (0.14 mL; 0.8 mmol) in DMF (2 mL) and the reaction mixture was stirred for 15 minutes at r.t. After that, ValCitPAB (0.15 g; 0.4 mmol) was added and the reaction mixture was stirred for 2 hours at r.t. The reaction mixture was evaporated. The product was purified by reverse phase chromatography (Method P7) to yield 0.2 g (92 %) of *tert*-butyl (2-(((S)-1-(((S)-1-((4-(hydroxymethyl)phenyl)amino)-1-oxo-5-ureidopentan-2-yl)amino)-3-methyl-1-oxobutan-2-yl)amino)-2-oxoethyl)carbamate.
UPLC-MS (Method A1): Rt = 2.31 min; m/z 537.5 [M+H]⁺.

*Step 2: Tert*-butyl (2-(((S)-1-(((S)-1-((4-(hydroxymethyl)phenyl)amino)-1-oxo-5-ureidopentan-2-yl)amino)-3-methyl-1-oxobutan-2-yl)amino)-2-oxoethyl)carbamate (0.2 g; 0.37 mmol) was treated with 20 % TFA in DCM (5 mL) for 3 hours at r.t. After that, the reaction mixture was evaporated. The crude was dissolved in 50 % MeOH/H₂O and treated with DOWEX OH⁻. The resin was filtered off and the solution evaporated. The product was purified by reverse phase chromatography (Method P7) to yield 0.15 g (90 %) of (S)-2-((S)-2-(2-aminoacetamido)-3-methylbutanamido)-N-(4-(hydroxymethyl)phenyl)-5-ureidopentanamide .
UPLC-MS (Method A1): Rt = 1.54 min; m/z 437.5 [M+H]⁺.

*Step 3:* Analogous to Example 1.1, Step-1 affording (S)-2-((S)-2-(2-(2-(2,5-dioxo-2,5-dihydro-1H-pyrrol-1-yl)acetamido)acetamido)-3-methylbutanamido)-N-(4-(hydroxymethyl)phenyl)-5-ureidopentanamide (20 mg, 35 %).
UPLC-MS (Method A1): Rt = 1.82 min; m/z 574.4 [M+H]⁺.

*Step 4:* Analogous to Example 1.1, Step-2 affording 4-((S)-2-((S)-2-(2-(2-(2,5-dioxo-2,5-dihydro-1H-pyrrol-1-yl)acetamido)acetamido)-3-methylbutanamido)-5-ureidopentanamido)benzyl (4-nitrophenyl) carbonate (6.4 mg, 25 %).
UPLC-MS (Method A1): Rt = 3.14 min; m/z 739.5 [M+H]⁺.

The compounds listed in the table below were prepared following an analogous procedure to the one above using commercially available or described starting materials (use of appropriate reagents and purification methods known to the skilled in the art):

| **Cmpd** | **Structure, reaction conditions and characterization** |
|---|---|
| **1.3** | 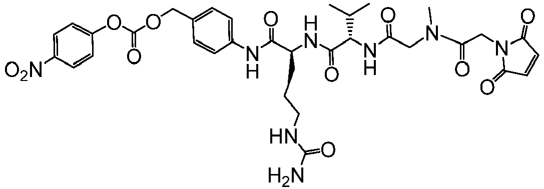 |
| | Step 1 using BOC-Sar-OH. |
| | Step 3 using PyBroP, DIPEA, DMF. |
| | UPLC-MS (Method A1): Rt = 3.03 min. |
| | m/z 753.5 [M+H]⁺ |
| **1.4** | 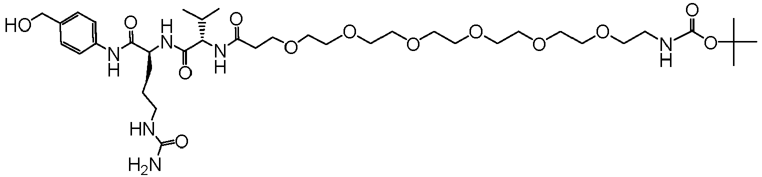 |
| | Following only Step 1 using compound CAS 882847-13-4. |
| | UPLC-MS (Method A1): Rt = 2.40 min. |
| | m/z 813.2 [M-H]⁻ |
| **1.5** | 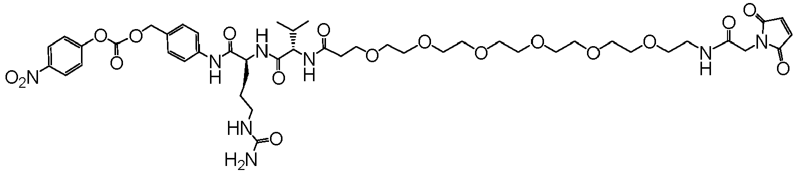 |
| | Starting from Compound 1.4 following Steps 2-4. |
| | UPLC-MS (Method A1): Rt = 2.79 min. |
| | m/z 1017.6 [M+H]⁺ |
| **1.6** | 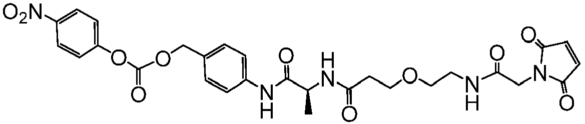 |
| | Step 1 using Ala-PAB. |
| | UPLC-MS (Method A1): Rt = 2.91 min. |
| | m/z 612.5 [M+H]⁺ |

### Example 1.7

### tert-butyl ((S)-1-(((S)-1-((3-chloro-4-(hydroxymethyl)phenyl)amino)-1-oxo-5-ureidopentan-2-yl)amino)-3-methyl-1-oxobutan-2-yl)carbamate (1.7)

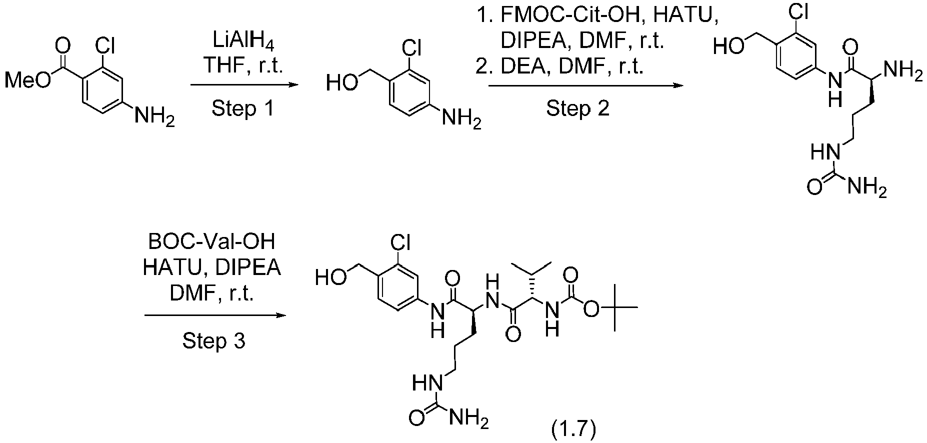

*Step 1:* Lithium aluminium hydride (2 M THF solution, 5 mL, 10 mmol) was added dropwise to a solution of methyl 4-amino-2-chlorobenzoate (0.93 g, 5 mmol) in THF (25 mL). The reaction mixture was stirred for 2 hours at r.t. After that, saturated aqueous sodium chloride was added (200 mL) and the product was extracted with chloroform (2×500 mL). The organic layer was dried over anhydrous sodium sulfate, filtered and evaporated. The product was lyofilized from dioxane to yield 0.78 g (99 %) of (4-amino-2-chlorophenyl)methanol.
UPLC-MS (Method A1): Rt = 1.63 min; m/z 158.1 [M+H]⁺.

*Step 2:* HATU (1.14 g, 3 mmol) was added to a mixture of FMOC-Cit-OH (0.79 g, 2 mmol) and DIPEA (0.52 mL, 3 mmol) in DMF (10 mL) and the reaction mixture was stirred for 15 minutes at r.t. After that, (4-amino-2-chlorophenyl)methanol (0.31 g, 2 mmol) was added and the reaction mixture was stirred for 2 hours at r.t. The reaction mixture was evaporated. The product was purified by chromatography on silica gel (gradient of methanol in chloroform). The FMOC protected derivative was then treated with DEA (1 mL) in DMF (10 mL) and the reaction mixture was stirred for 1 hour at r.t. The reaction mixture was evaporated. The crude was dissolved in water (50 mL) and washed with ethyl acetate (3x100 mL). The aqueous layer was evaporated and (*S*)-2-amino-*N*-(3-chloro-4-(hydroxymethyl)phenyl)-5-ureidopentanamide used without further purification.
UPLC-MS (Method A1): Rt = 1.41 min; m/z 315.3 [M+H]⁺.

*Step 3:* HATU (1.14 g, 3 mmol) was added to a mixture of BOC-Val-OH (0.44 g, 2 mmol) and DIPEA (0.52 mL, 3 mmol) in DMF (10 mL) and the reaction mixture was stirred for 15 minutes at r.t. After that, (*S*)-2-amino-*N*-(3-chloro-4-(hydroxymethyl)phenyl)-5-ureidopentanamide (0.57 g, 1.8 mmol) was added and the reaction mixture was stirred for 2 hours at r.t. The reaction mixture was evaporated. The product was purified by chromatography on silica gel (gradient of methanol in chloroform) to yield 0.59 g (63 %) of *tert*-butyl ((*S*)-1-(((*S*)-1-((3-chloro-4-(hydroxymethyl)phenyl)amino)-1-oxo-5-ureidopentan-2-yl)amino)-3-methyl-1-oxobutan-2-yl)carbamate.
UPLC-MS (Method A1): Rt = 2.59 min; m/z 514.4 [M+H]⁺.
¹H NMR (401 MHz, DMSO-d6) δ 10.16 (s, 1H), 8.02 (d, J = 7.4 Hz, 1H), 7.82 - 7.77 (m, 1H), 7.49 - 7.40 (m, 2H), 6.75 (d, J = 8.8 Hz, 1H), 5.96 (t, J = 5.8 Hz, 1H), 5.41 (s, 2H), 5.30 (t, J = 5.6 Hz, 1H), 4.50 (d, J = 5.4 Hz, 2H), 4.44 - 4.34 (m, 1H), 3.87 - 3.79 (m, 1H), 3.08 - 2.87 (m, 2H), 2.01 - 1.89 (m, 1H), 1.76 - 1.52 (m, 2H), 1.38 (s, 9H), 1.50 - 1.31 (m, 2H), 0.84 (dd, J = 16.5, 6.7 Hz, 6H). ¹³C NMR (101 MHz, DMSO-d6) δ 171.92, 171.30, 159.32, 155.98, 139.06, 134.63, 131.40, 128.97, 119.41, 118.06, 78.58, 60.43, 60.18, 53.52, 30.85, 29.87, 28.64, 27.18, 19.68, 18.63.

The compounds listed in the table below were prepared following an analogous procedure to the one above using commercially available or described starting materials (use of appropriate reagents and purification methods known to the skilled in the art):

| **Cmpd** | **Structure** | **Reaction conditions and characterization** |
|---|---|---|
| **1.8** | 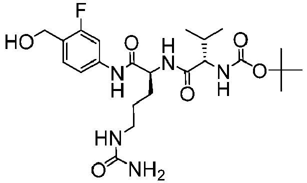 | Step 1 using methyl 4-amino-2-fluorobenzoate. |
| | | UPLC-MS (Method A1): Rt = 2.53 min. |
| | | m/z 498.4 [M+H]⁺ |
| **1.9** | 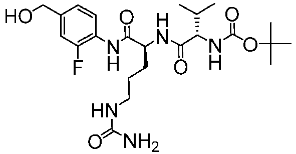 | Step 1 using methyl 4-amino-3-fluorobenzoate. |
| | | UPLC-MS (Method A1): Rt = 2.33 min. |
| | | m/z 498.4 [M+H]⁺ |

### Example 1.10

### (S)-2-(2-(2,5-dioxo-2,5-dihydro-1H-pyrrol-1-yl)acetamido)-N-((S)-1-((4-(iodomethyl)phenyl)amino)-1-oxopropan-2-yl)-3-methylbutanamide (1.10)

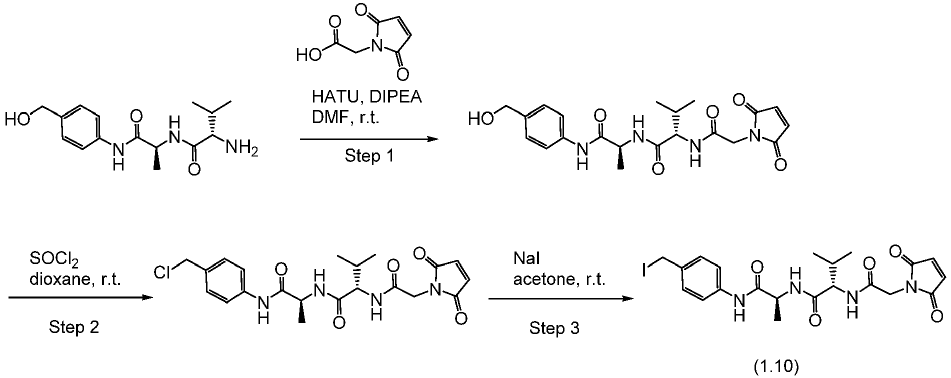

*Step 1:* Analogous to Example 1.1, Step-1 affording (*S*)-2-(2-(2,5-dioxo-2,5-dihydro-1*H*-pyrrol-1-yl)acetamido)-*N*-((*S*)-1-((4-(hydroxymethyl)phenyl)amino)-1-oxopropan-2-yl)-3-methylbutanamide (117 mg, 54 %).
UPLC-MS (Method A1): Rt = 2.00 min; m/z 431.2 [M+H]⁺.

*Step 2:* (*S*)-2-(2-(2,5-dioxo-2,5-dihydro-1*H*-pyrrol-1-yl)acetamido)-*N*-((*S*)-1-((4-(hydroxymethyl)phenyl)amino)-1-oxopropan-2-yl)-3-methylbutanamide (43 mg; 0.1 mmol) was treated with thionyl chloride (29 µL; 0.4 mmol) in dioxane (2 mL). The reaction mixture was stirred for 1 h at r.t., evaporated and coevaporated with dioxane.
UPLC-MS (Method A1): Rt = 2.79 min; m/z 449.2/451.2 [M+H]⁺.

*Step 3:* The crude (*S*)-*N*-((*S*)-1-((4-(chloromethyl)phenyl)amino)-1-oxopropan-2-yl)-2-(2-(2,5-dioxo-2,5-dihydro-1*H*-pyrrol-1-yl)acetamido)-3-methylbutanamide was treated with sodium iodide (60 mg; 0.4 mmol) in acetone (4 mL) for 16 h at r.t. The reaction mixture was filtered through syringe filter and evaporated. The product was used without further purification.
UPLC-MS (Method A1): Rt = 3.04 min; m/z 541.2 [M+H]⁺.

### Example 2 - Synthesis of CDNs

### Example 2.1

### 9-((6R,8R,9S,13aR,15R,16R,16aR,17R)-15-(6-Amino-9H-purin-9-yl)-16,17-difluoro-3,11-dimercapto-3,11-dioxidodecahydro-6,9-methanofuro[3,2-m][1,3,6,9,12]pentaoxa[2,10]diphosphacyclopentadecin-8-yl)-1,9-dihydro-6H-purin-6-one (Compounds 2.1 and 2.2)

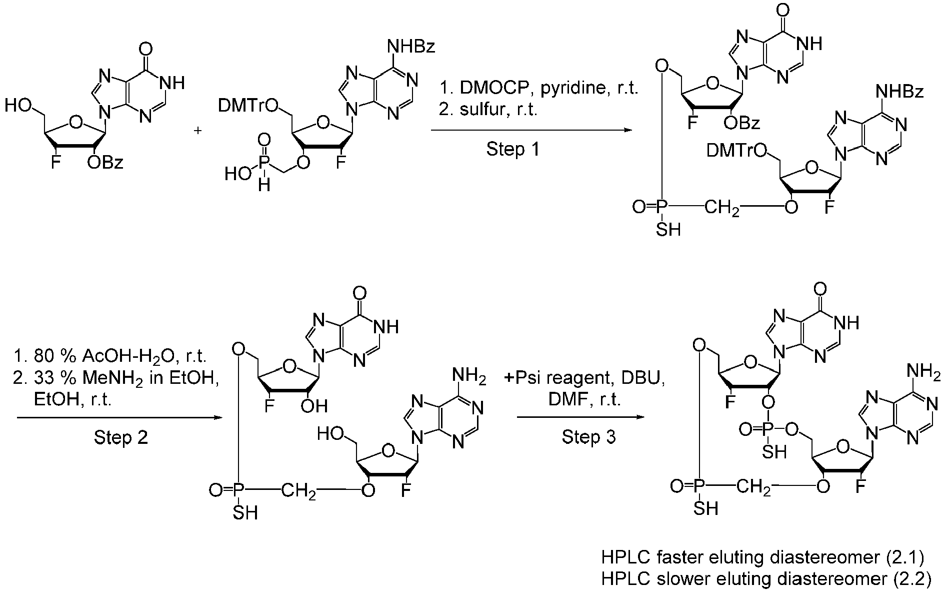

*Step 1:* DMOCP (4.3 g; 23.4 mmol) was added to a solution of (2*R*,3*S*,4*R*,5*R*)-4-fluoro-5-(hydroxymethyl)-2-(6-oxo-1,6-dihydro-9*H*-purin-9-yl)tetrahydrofuran-3-yl benzoate (2.9 g; 7.8 mmol) and ((((2*R*,3*R*,4*R*,5*R*)-5-(6-benzamido-9*H*-purin-9-yl)-2-((bis(4-methoxyphenyl)(phenyl)methoxy)methyl)-4-fluorotetrahydrofuran-3-yl)oxy)methyl)phosphinic acid TEA salt (5.6 g; 6.5 mmol) in pyridine (200 mL), and the reaction mixture was stirred for 2 hours at r.t. After that, sulfur (0.75 g; 23.4 mmol) was added and the reaction mixture was stirred for additional 30 minutes at r.t. The reaction was quenched by the addition of 1M TEAB (30 mL) and the reaction mixture was evaporated. The crude was dissolved in DCM (200 mL) and washed with 0.1 M TEAB (3x100 mL). The organic layer was dried over sodium sulfate and evaporated. (2*R*,3*S*,4*R*,5*R*)-5-(((((((2*R*,3*R*,4*R*,5*R*)-5-(6-Benzamido-9*H*-purin-9-yl)-2-((bis(4-methoxyphenyl)(phenyl)methoxy)methyl)-4-fluorotetrahydrofuran-3-yl)oxy)methyl)(mercapto)phosphoryl)oxy)methyl)-4-fluoro-2-(6-oxo-1,6-dihydro-9*H*-purin-9-yl)tetrahydrofuran-3-yl benzoate TEA salt was obtained as a mixture of phosphonothioate diastereomers and used without further purification.
UPLC-MS (Method A1): Rt = 3.52 min; m/z 1140.5 [M-H]⁻.

*Step 2:* The crude (2*R*,3*S*,4*R*,5*R*)-5-(((((((2*R*,3*R*,4*R*,5*R*)-5-(6-benzamido-9*H*-purin-9-yl)-2-((bis(4-methoxyphenyl)(phenyl)methoxy)methyl)-4-fluorotetrahydrofuran-3-yl)oxy)methyl)(mercapto)phosphoryl)oxy)methyl)-4-fluoro-2-(6-oxo-1,6-dihydro-9*H*-purin-9-yl)tetrahydrofuran-3-yl benzoate TEA salt was dissolved in 80 % AcOH in water (200 mL) and the reaction mixture was stirred for 2 hours at r.t. The reaction mixture was evaporated and co-evaporated with water. After that, the crude was treated with 33% MeNH₂ in absolute EtOH (60 mL) and EtOH (120 mL) and the reaction mixture was stirred for 30 minutes at r.t. The reaction mixture was evaporated. The crude was dissolved in 0.1M TEAB (200 mL) and washed with DCM (3x100 mL). The aqueous layer was evaporated. The product was purified by reverse phase chromatography (Method P1) to yield 3.6 g (76 %) of *O*-(((2*R*,3*S*,4*S*,5*R*)-3-fluoro-4-hydroxy-5-(6-oxo-1,6-dihydro-9*H*-purin-9-yl)tetrahydrofuran-2-yl)methyl) S-hydrogen ((((2*R*,3*R*,4*R*,5*R*)-5-(6-amino-9*H*-purin-9-yl)-4-fluoro-2-(hydroxymethyl)tetrahydrofuran-3-yl)oxy)methyl)phosphonothioate TEA salt as a 1:1 mixture of phosphonothioate diastereomers.
UPLC-MS (Method A1): Rt = 1.60 min; m/z 630.2 [M-H]⁻.
³¹P (D₂O) δ 71.59, 71.05.

*Step 3:* DBU (8.6 mL; 57.7 mmol) was added to a solution of *O*-(((2*R*,3*S*,4*S*,5*R*)-3-fluoro-4-hydroxy-5-(6-oxo-1,6-dihydro-9*H*-purin-9-yl)tetrahydrofuran-2-yl)methyl) *S*-hydrogen ((((2*R*,3*R*,4*R*,5*R*)-5-(6-amino-9*H*-purin-9-yl)-4-fluoro-2-(hydroxymethyl)tetrahydrofuran-3-yl)oxy)methyl)phosphonothioate TEA salt (3.6 g; 5.8 mmol) and (2*R*,3a*R*,6*S*,7a*R*)-3a-methyl-2-((perfluorophenyl)thio)-6-(prop-1-en-2-yl)hexahydrobenzo[*d*][1,3,2]oxathiaphosphole 2-sulfide (2.6 g; 5.8 mmol; +PSI reagent) in DMF (300 mL) and the reaction mixture was stirred for 1 hours at r.t. The reaction mixture was evaporated. The crude was treated with 0.1M TEAB (80 mL). The formed heterogeneous mixture was centrifuged. The supernatant was collected and evaporated. The diastereomers were purified by reverse phase chromatography (Method P1) and lyophilized from dioxane/water (1:1) to yield 1.35 g (30 %) of HPLC faster eluting diastereomer (Compound 2.1) and 1.45 g (32 %) of HPLC slower eluting diastereomer (Compound 2.2) as diTEA salts.

### Compound 2.1:

UPLC-MS (Method A1): Rt = 1.36 min; m/z 710.0 [M+H]⁺.

ESI-HRMS calculated for C₂₁H₂₄O₉N₉F₂P₂S₂ (M+H)⁺ 710.05762; found 710.05742.

### Compound 2.2:

UPLC-MS (Method A1): Rt = 1.60 min; m/z 710.0 [M+H]⁺.

ESI-HRMS calculated for C₂₁H₂₄O₉N₉F₂P₂S₂ (M+H)⁺ 710.05762; found 710.05750.

**Table 1. ¹H, ³¹P and ¹⁹F NMR data of Compound 2.1 and Compound 2.2 in D₂O.**

| **Cmpd** | **Unit** | **H-1'** | **H-2'** | **H-3'** | **H-4'** | **H-5'a** | **H-5'b** | **P-CH₂-O** |
|---|---|---|---|---|---|---|---|---|
| **2.1** | Hpx | 6.33 d | 5.385 dddd | 5.59 dd | 4.84 overlap | 4.255 dddd | 4.19 ddd | -- |
| | | | 2,1 = 8.2 | 3,2 = 4.0 | | 5a,4 = 2.2 | 5b,4 = 2.2 | |
| | | 1,2 = 8.2 | 2,3 = 4.0 | 3,4 = 0 | | 5a,5b = 11.5 | 5b,5a = 11.5 | |
| | | | 2,P =10.6 | 3,F = 53.0 | | 5a,P = 3.2 | 5b,P = 5.3 | |
| | | | 2,F = 24.8 | | | 5a,F = 3.0 | | |
| | A | 6.42 d | 5.70 dd | 4.69 ddd | 4.57 dq | 4.36 ddd | 4.13 ddd | 4.22 dd |
| | | | 2,1 = 0 | 3,2 = 3.6 | 4,3 = 9.0 | 5a,4 = 3.2 | 5b,4 = 2.3 | (13.4; 2.6); |
| | | 1,2 = 0 | 2,3 = 3.6 | 3,4 = 9.0 | 4,5a = 3.2 | 5a,5b = 11.9 | 5b,5a = 11.9 | |
| | | 1,F =16.9 | 2,F = 51.8 | 3,F = 24.8 | 4,5b = 2.3 | 5a,P = 4.6 | 5b,P = 4.5 | 4.02 dd |
| | | | | | 4,F =2.5 | | | (13.4; 6.9) |
| | **Nucleobase:** H-2: 8.09 s, H-8: 8.40 s (Hpx); H-2: 8.24 s, H-8: 8.17 s (A). **³¹P** 54.10, 69.00. **¹⁹F** -192.50, -199.72. | | | | | | | |
| **2.2** | Hpx | 6.325 d | 5.35 dddd | 5.495 dd | 4.81 overlap | 4.53 ddd | 4.05 dq | -- |
| | | | 2,1 = 8.2 | 3,2 = 4.2 | | 5a,4 = 2.2 | 5b,4 = 2.2 | |
| | | 1,2 = 8.2 | 2,3 = 4.2 | 3,4 = 0 | | 5a,5b = 11.8 | 5b,5a = 11.8 | |
| | | | 2,P = 10.8 | 3,F = 53.5 | | 5a,P = 8.2 | 5b,P = 2.2 | |
| | | | 2,F = 24.2 | | | | 4,F = 2.2 | |
| | A | 6.44 d | 5.69 dd | 4.70 ddd | 4.58 dq | 4.38 q | 4.12 ddd | 4.085 dd |
| | | | 2,1 = 0 | 3,2 = 3.7 | 4,3 = 9.0 | 5a,4 = 3.0 | 5b,4 = 2.3 | (12.7;1.2); |
| | | 1,2 = 0 | 2,3 = 3.7 | 3,4 = 9.0 | 4,5a = 3.0 | 5a,5b = 12.0 | 5b,5a = 12.0 | 4.08 dd |
| | | 1,F =17.3 | 2,F = 52.0 | 3,F = 25.2 | 4,5b = 2.3 | 5a,P = 3.0 | 5b,P = 4.4 | (12.7; 12.2) |
| | | | | | 4,F = 3.0 | 5a,F = 3.0 | | |
| | **Nucleobase:** H-2: 8.07 s, H-8: 8.34 s (Hpx); H-2: 8.25 s, H-8: 8.17 s (A). **³¹P** 54.18, 72.52. **¹⁹F** -192.61, -199.12. | | | | | | | |

**Table 2. ¹³C NMR data of Compound 2.1 and Compound 2.2 in D₂O.**

| **Cmpd** | **Unit** | **C-1'** | **C-2'** | **C-3'** | **C-4'** | **C-5'** | **P-CH₂-O** | **C-2** | **C-4** | **C-5** | **C-6** | **C-8** |
|---|---|---|---|---|---|---|---|---|---|---|---|---|
| **2.1** | Hpx | 88.34 (4.1) | 78.09 (15.9; 7.9) | 94.76 (184.8; 4.3) | 85.19 (23.6; 10.5) | 64.33 (11.8; 5.7) | -- | 148.59 | 152.01 | 126.72 | 161.12 | 143.09 |
| | A | 89.70 (33.6) | 93.84 (188.9) | 79.35 (16.4; 11.7) | 81.36 (11.3) | 66.21 (4.2) | 73.41 (121.5) | 155.58 | 150.65 | 121.51 | 158.30 | 141.80 |
| **2.2** | Hpx | 88.20 (3.3) | 77.77 (15.0; 8.8) | 94.52 (184.8; 5.0) | 85.44 (24.2; 10.6) | 65.16 (12.3; 5.3) | -- | 147.51 | 152.11 | 126.77 | 161.22 | 143.09 |
| | A | 89.74 (33.8) | 93.82 (188.8) | 79.56 (16.2; 13.3) | 81.26 (11.4) | 66.05 (4.4) | 72.94 (127.7) | 155.44 | 150.66 | 121.51 | 158.22 | 141.86 |

### Example 2.2 (Comparative compound)

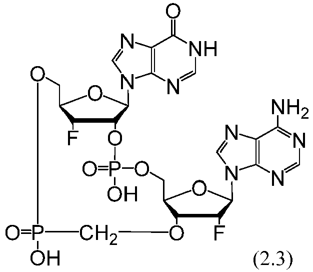

Compound 2.3 was prepared according to WO2019193542A1.

### Example 2.3 (Comparative compound)

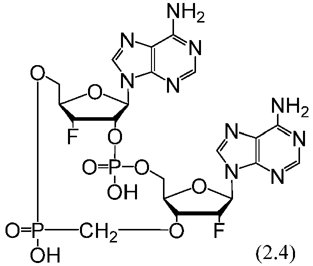

Compound 2.4 was prepared according to WO2019193542A1.

### Example 2.4 (Comparative compounds)

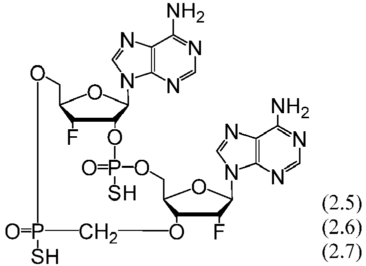

Three phosphorothioate diastereomers 2.5, 2.6 and 2.7 were prepared according to WO2020178769A1.

### Example 3 - Synthesis of CDN probes

### Example 3.1

### 2-(Methylamino)ethyl (9-((6R,8R,9S,13aR,15R,16R,16aR,17R)-16,17-difluoro-3,11-dimercapto-3,11-dioxido-8-(6-oxo-1,6-dihydro-9H-purin-9-yl)decahydro-6,9-methanofuro[3,2-m][1,3,6,9,12]pentaoxa[2,10]diphosphacyclopentadecin-15-yl)-9H-purin-6-yl)carbamate TFA salt (3.1)

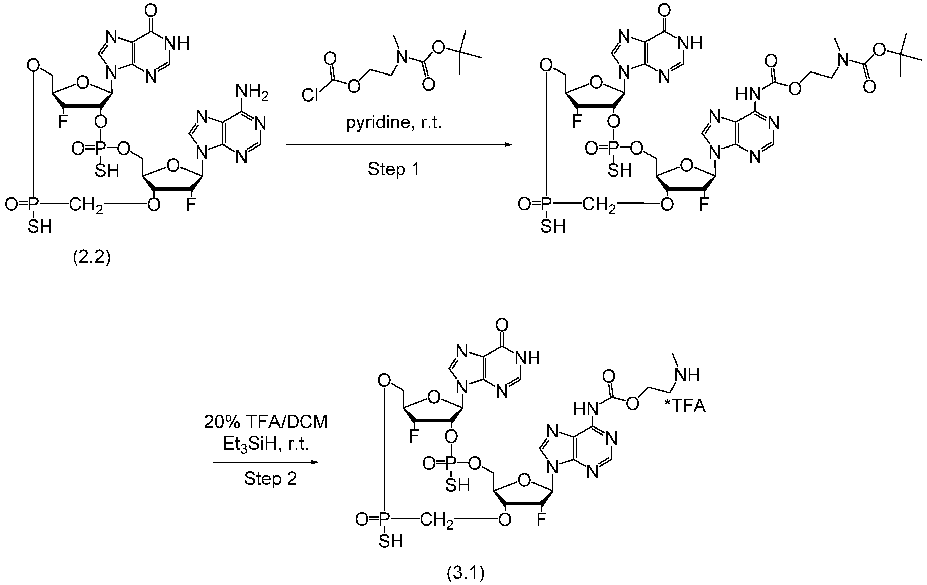

*Step 1: tert-Butyl* (2-hydroxyethyl)(methyl)carbamate (1.8 g; 10.1 mmol) and pyridine (1.9 mL; 23.1 mmol) in DCM (10 mL) were added to a solution of 20 % phosgene in toluene (11.1 mL) in DCM (50 mL) under argon at -78 °C. The reaction mixture was stirred for 10 minutes under argon at -78 °C and then left to warm up to r.t. After that, DCM was evaporated, the crude was diluted with pyridine (20 mL) and added to a stirred solution of 9-((6*R*,8*R*,9*S*,13a*R*,15*R*,16*R*,16a*R*,17*R*)-15-(6-amino-9*H*-purin-9-yl)-16,17-difluoro-3,11-dimercapto-3,11-dioxidodecahydro-6,9-methanofuro[3,2-*m*][1,3,6,9,12]pentaoxa[2,10]diphosphacyclopentadecin-8-yl)-1,9-dihydro-6*H*-purin-6-one diTEA salt (0.23 g; 0.26 mmol; Compound 2.2). The reaction mixture was stirred for 1 h at r.t. After that, the reaction mixture was cooled to 4 °C and water (2 mL) was added. The reaction mixture was left to warm up to r.t. and evaporated. The crude was dissolved in DMSO (10 mL) and directly loaded on C18 column. The products were purified by reverse phase chromatography (Method P2) as HPLC faster eluting monocarbamate and HPLC slower eluting biscarbamate. The mono and biscarbamate derivatives were evaporated. The biscarbamate derivative was treated with 60 % pyridine in water for 16 h at 55 °C affording monocarbamate derivative. Both portions of monocarbamate derivative were collected and lyophilized from dioxane/water (1: 1) to yield 0.21 g (72 %).

### biscarbamate:

UPLC-MS (Method A1): Rt = 2.73 min; m/z 1110.6 [M-H]⁻.

### monocarbamate:

UPLC-MS (Method A1): Rt = 2.11 min; m/z 909.5 [M-H]⁻.

ESI-HRMS calculated for C₃₀H₃₉O₁₃N₁₀F₂P₂S₂ (M+H)⁺ 911.15773, found 911.15756.

*Step 2:* A mixture of 20 % TFA in DCM (50 mL) and triethylsilane (0.6 mL; 3.8 mmol) was added to monocarbamate derivative (0.21 g; 0.19 mmol). The reaction mixture was stirred for 30 minutes at r.t. and evaporated. The product was purified by reverse phase chromatography (Method P3) and lyophilized from dioxane/water (1: 1) to yield 0.11 g (46 %) of Compound 3.1.

### Compound 3.1:

UPLC-MS (Method A1): Rt = 1.75 min; m/z 809.3 [M-H]⁻.

ESI-HRMS calculated for C₂₅H₃₁O₁₁N₁₀F₂P₂S₂ (M+H)⁺ 811.10530, found 811.10544.

**Table 3. ¹H, ³¹P and ¹⁹F NMR data of Compound 3.1 in DMSO-d6.**

| **Unit** | **H-1'** | **H-2'** | **H-3'** | **H-4'** | **H-5'a** | **H-5'b** | **P-CHa-O** | **P-CHb-O** | **H-2,8** |
|---|---|---|---|---|---|---|---|---|---|
| Hpx | 6.08 d (8.6) | 5.36 dddd (26.2; 8.6; 12.5; 3.9) | 5.26 dd (54.2; 3.9) | 4.60 dq (27.0; 2.2; 2.2; 2.2) | 4.12 ddd (11.6; 8.2; 2.2) | 3.87 dq (11.6; 2.2; 2.2; 2.2) | -- | -- | 8.09 d (3.7); 8.40 s |
| A | 6.47 d (16.3) | 5.92 dd (51.6; 3.2) | 5.36 ddd (27.5; 9.0; 3.2) | 4.36 dm (9.0) | 4.21 dm (11.8) | 3.71 dm (11.8) | 4.14 bd (14.6) | 3.95 bd (14.6) | 8.72 s; 8.74 s |
| **Linker: -CO-O-CH₂-CH₂-NH-CH₃:** 4.44 m and 4.39 m (O-CH₂), 3.16 m (N-CH₂), 2.57 (N-CH₃), **³¹P** 55.06, 75.14. **¹⁹F** -191.41, -200.67. | | | | | | | | | |

**Table 4. ¹³C NMR data of Compound 3.1 in DMSO-d6.**

| **Unit** | **C-1'** | **C-2'** | **C-3'** | **C-4'** | **C-5'** | **P-CH₂-O** | **C-2** | **C-4** | **C-5** | **C-6** | **C-8** |
|---|---|---|---|---|---|---|---|---|---|---|---|
| Hpx | 83.45 | 74.10 | 92.76 (185.1) | 81.72 (22.8; 9.5) | 64.52 | -- | 146.40 | 149.33 | 123.60 | 156.77 | 138.94 |
| A | 85.87 (32.6) | 91.53 (186.8) | 74.10 | 79.57 | 62.61 | 67.78 (114.6) | 152.27 | 149.48 | 122.99 | 151.63 | 141.90 |
| **Linker: -CO-O-CH₂-CH₂-NH-CH₃:** 152.02 (C=O), 60.70 (O-CH₂), 46.80 (N-CH₂), 32.07 (N-CH₃). | | | | | | | | | | | |

The Compound 3.2 in the table below was prepared following an analogous procedure to the one above using commercially available or described starting materials (use of appropriate reagents and purification methods known to the skilled in the art):

| **Structure** | **Reaction conditions and characterization** |
|---|---|
| 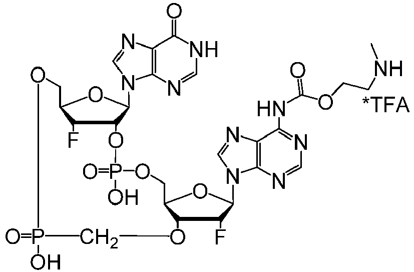 | Step 1 using Compound 2.3. |
| | Step 2 was performed in the absence of Et₃SiH. |
| | UPLC-MS (Method A1): Rt = 1.25 min. m/z 779.3 [M+H]⁺ |

### Example 3.3

### 4-((S)-2-((S)-2-(2-(2,5-dioxo-2,5-dihydro-1H-pyrrol-1-yl)acetamido)-3-methylbutanamido)-5-ureidopentanamido)benzyl (2-(((9-((6R,8R,9S,13aR,15R,16R,16aR,17R)-16,17-difluoro-3,11-dimercapto-3,11-dioxido-8-(6-oxo-1,6-dihydro-9H-purin-9-yl)decahydro-6,9-methanofuro[3,2-m][1,3,6,9,12]pentaoxa[2,10]diphosphacyclopentadecin-15-yl)-9H-purin-6-yl)carbamoyl)oxy)ethyl)(methyl)carbamate (3.3)

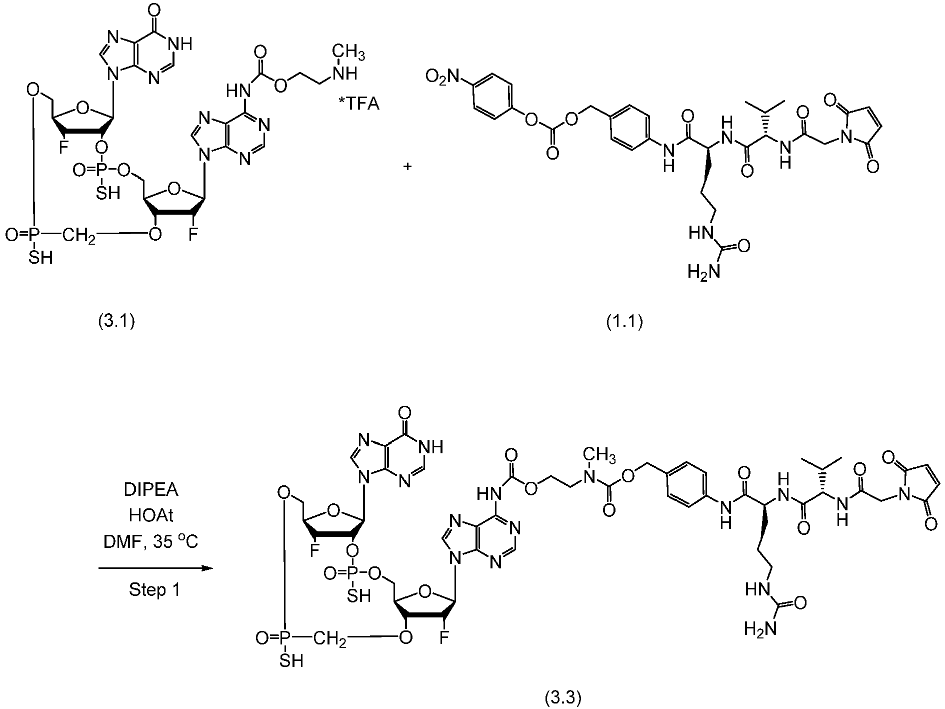

*Step 1:* DIPEA (14.6 µL; 87 µmol) and HOAt (8.2 mg; 43.5 µmol) were added to a solution of Compound 1.1 (5.9 mg; 8.7 µmol) and 2-(methylamino)ethyl (9-((6*R*,8*R*,9*S*,13a*R*,15*R*,16*R*,16a*R*,17*R*)-16,17-difluoro-3,11-dimercapto-3,11-dioxido-8-(6-oxo-1,6-dihydro-9*H*-purin-9-yl)decahydro-6,9-methanofuro[3,2-*m*][1,3,6,9,12]pentaoxa[2,10]diphosphacyclopentadecin-15-yl)-9*H*-purin-6-yl)carbamate TFA salt (8 mg; 8.7 µmol; Compound 3.1) in DMF (3 mL) and the reaction mixture was stirred for 8 h at 35 °C. The reaction mixture was evaporated. The product was purified by reverse phase chromatography (Method P4) and lyophilized from dioxane/water (1:1) to yield 8.2 mg (64 %) of Compound 3.3.

UPLC-MS (Method A1): Rt = 2.04 min; m/z 1353.8 [M+H]⁺.

ESI-HRMS calculated for C₅₀H₅₉O₁₉N₁₆F₂P₂S₂ (M-H)⁻ 1351.30326, found 1351.30228.

**Table 5. ¹H, ³¹P and ¹⁹F NMR data of Compound 3.3 in DMSO-d6.**

| **Unit** | **H-1'** | **H-2'** | **H-3'** | **H-4'** | **H-5'a** | **H-5'b** | **P-CHa-O** | **P-CHb-O** | **H-2,8** |
|---|---|---|---|---|---|---|---|---|---|
| Hpx | 6.11 d (8.6) | 5.44 dddd (24.8; 8.6; 12.6; 4.2) | 5.31 dd (54.2; 4.2) | 4.63 dq (27.8; 2.2; 2.2; 2.2) | 4.28 m | 3.92 m | -- | -- | 8.09 d (3.8); 8.35 s |
| A | 6.46 d (19.0) | 5.89 dd (52.2; 3.8) | 5.12 ddd (25.0; 8.2; 3.8) | 4.34 dm (8.2) | 4.23 m | 3.89 m | 4.17 dd (14.0; 1.8) | 4.08 dd (14.0 ; 5.0) | 8.67 s; 8.47 s |
| **Linker: Mal Gly:** 7.08 s (CH=), 4.13 s (N-CH₂); **Val:** 8.21 d (NH), 4.25 dd (N-CH), 1.98 m (CH), 0.85 d and 0.82 d (2x CH₃); **Cit:** 8.23 d (NH), 4.38 ddd (N-CH), 1.69 m and 1.59 m (CH₂), 1.44 m and 1.37 m (CH₂), 3.03 m and 2.94 m (N-CH₂); **PAB:** 10.01 bs and 9.99 bs (NH), 7.58 m, 7.54 m, 7.30 m and 7.26 m (4x ArH), 5.00 b and 4.97 b (O-CH₂); **SE:** 2.95 s and 2.92 s (N-CH₃), 3.52 m (N-CH₂), 4.25 m (O-CH₂). **³¹P** 57.70, 79.82. **¹⁹F** -191.70, -198.74. | | | | | | | | | |

**Table 6. ¹³C NMR data of Compound 3.3 in DMSO-d6.**

| **Unit** | **C-1'** | **C-2'** | **C-3'** | **C-4'** | **C-5'** | **P-CH₂-O** | **C-2** | **C-4** | **C-5** | **C-6** | **C-8** |
|---|---|---|---|---|---|---|---|---|---|---|---|
| Hpx | 83.54 | 74.12 (16.0; 8.0) | 92.13 (184.2; 2.0) | 81.59 (23.0; 10.8) | 64.47 | -- | 146.48 | 149.21 | 124.11 | 156.73 | 138.47 |
| A | 86.29 (33.7) | 91.74 (187.2) | 76.36 | 78.81 (10.8) | 63.21 | 68.44 (123.8) | 152.06 | 151.14 | 123.47 | 149.87 | 141.72 |
| **Linker: Mal-Gly:** 170.89 (C=O), 135.02 (CH=), 39.73 (N-CH₂), 166.23 (C=O); **Val:** 170.96 (C=O), 57.65 (N-CH), 31.16 (CH), 19.30 and 18.14 (2x CH₃); **Cit:** 170.71 (C=O), 53.37 (N-CH), 29.36 (CH₂), 26.94 (CH₂), 38.76 (N-CH₂), 159.15 (C=O); **PAB:** 138.80 (ArC), 119.20, 119.13, 128.63 and 128.54 (4x ArCH), 131.78 (ArC), 66.29 (O-CH₂), **SE:** 152.21 (C=O), 63.13 (O-CH₂), 47.91 and 47.37 (N-CH₂), 35.83 and 35.39 (N-CH₃), 155.75 and 155.50 (C=O) | | | | | | | | | | | |

The compounds listed in the table below were prepared following an analogous procedure to the one above using commercially available or described starting materials (use of appropriate reagents and purification methods known to the skilled in the art):

| **Cmpd** | **Structure, reaction conditions and characterization** |
|---|---|
| **3.4** | 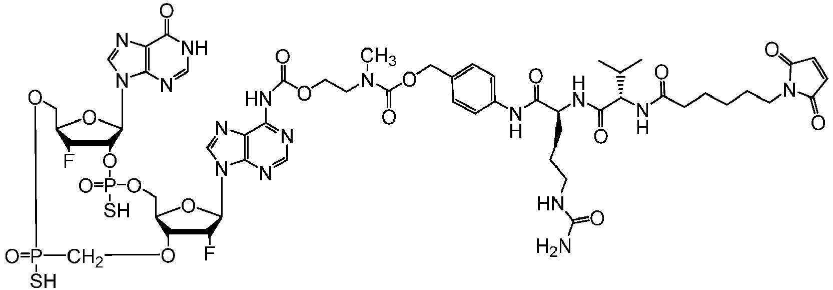 |
| | Compound 3.1 and MC-Val-Cit-PAB-PNP (CAS 159857-81-5. |
| | UPLC-MS (Method A1): Rt = 2.20 min; m/z 1409.7 [M+H]⁺. ESI-HRMS calculated for C₅₄H₆₉O₁₉N₁₆F₂P₂S₂ (M+H)⁺ 1409.38041, found 1409.38025. NMR Tables 7-8. |
| **3.5** | 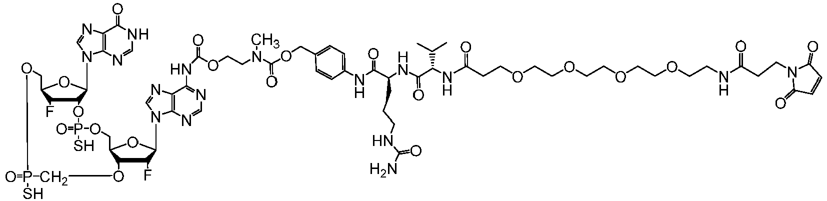 |
| | Compound 3.1 and Mal-beta-Ala-PEG(4)-Val-Cit-PAB-PNP (CAS 2003260-12-4). |
| | UPLC-MS (Method A1): Rt = 2.05 min; m/z 1614.9 [M+H]⁺. ESI-HRMS calculated for C₆₂H₈₂O₂₄N₁₇F₂P₂S₂ (M-H)⁻ 1612.46088, found 1612.45994. NMR Tables 7-8. |
| **3.6** | 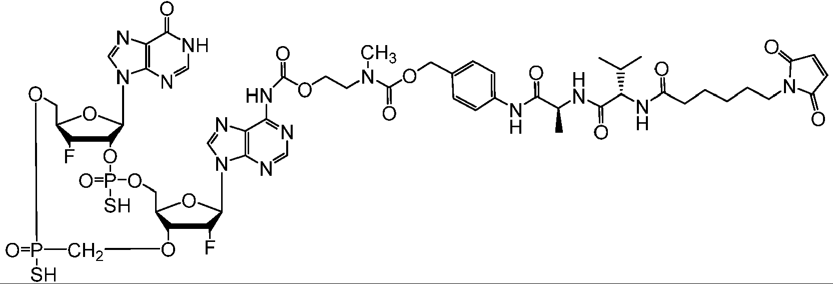 |
| | Compound 3.1 and MC-Val-Ala-PAB-PNP (CAS 1639939-40-4). |
| | UPLC-MS (Method A1): Rt = 2.28 min; m/z 1323.9 [M+H]⁺. ESI-HRMS calculated for C₅₁H₆₁O₁₈N₁₄F₂P₂S₂ (M-H)⁻ 1321.31784, found 1321.31732. NMR Tables 7-8. |
| **3.7** | 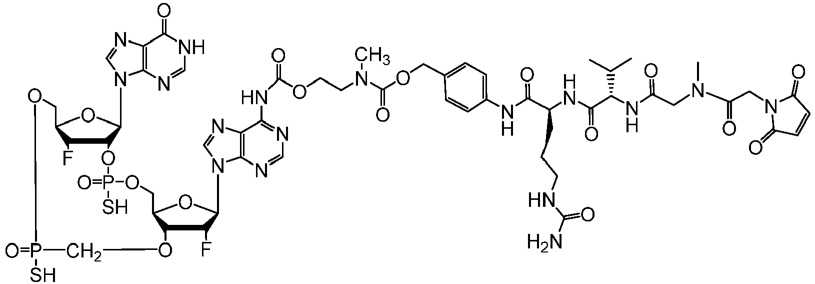 |
| | Compound 3.1 and Compound 1.3. |
| | UPLC-MS (Method A1): Rt = 2.06 min; m/z 1424.4 [M+H]⁺. ESI-HRMS calculated for C₅₃H₆₄O₂₀N₁₇F₂P₂S₂ (M-H)⁻ 1422.34037, found 1422.33973. NMR Tables 7-8. |
| **3.8** | 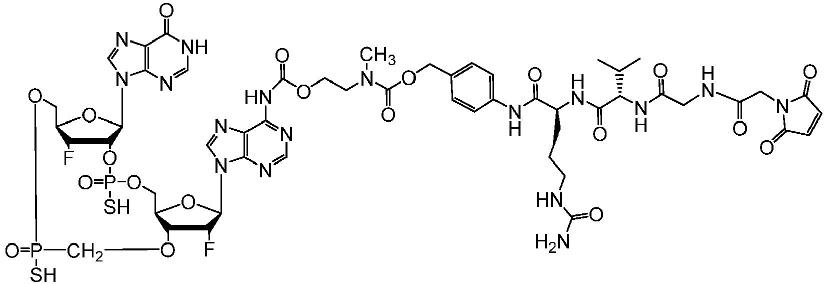 |
| | Compound 3.1 and Compound 1.2. |
| | UPLC-MS (Method A1): Rt = 1.98 min; m/z 1410.9 [M+H]⁺. ESI-HRMS calculated for C₅₂H₆₄O₂₀N₁₇F₂P₂S₂ (M+H)⁺ 1410.33927, found 1410.33956. NMR Tables 7-8. |
| **3.9** | 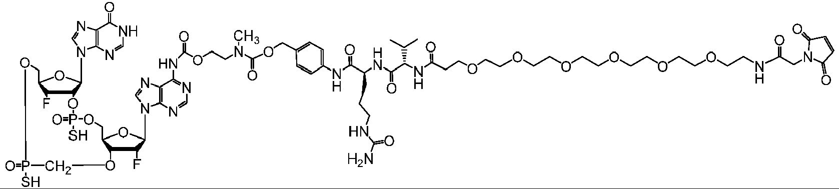 |
| | Compound 3.1 and Compound 1.5. |
| | UPLC-MS (Method A1): Rt = 2.00 min; m/z 1688.9 [M+H]⁺. ESI-HRMS calculated for C₆₅H₈₈O₂₆N₁₇F₂P₂S₂ (M-H)⁻ 1686.49766, found 1686.49665. NMR Tables 7-8. |
| **3.10** | 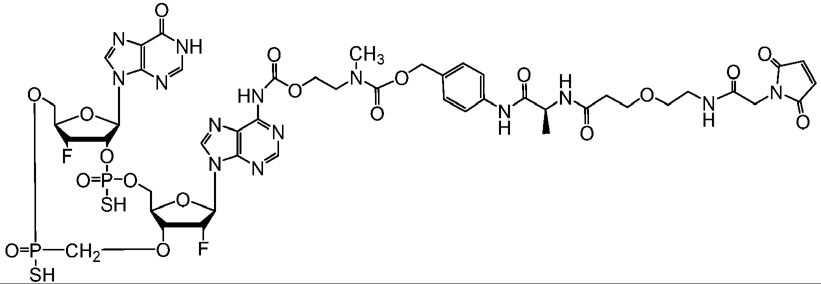 |
| | Compound 3.1 and Compound 1.6. |
| | UPLC-MS (Method A1): Rt = 1.93 min; m/z 1283.9 [M+H]⁺. ESI-HRMS calculated for C₄₇H₅₃O₁₉N₁₄F₂P₂S₂ (M-H)⁻ 1281.25016, found 1281.24931. NMR Tables 7-8. |
| **3.11** | 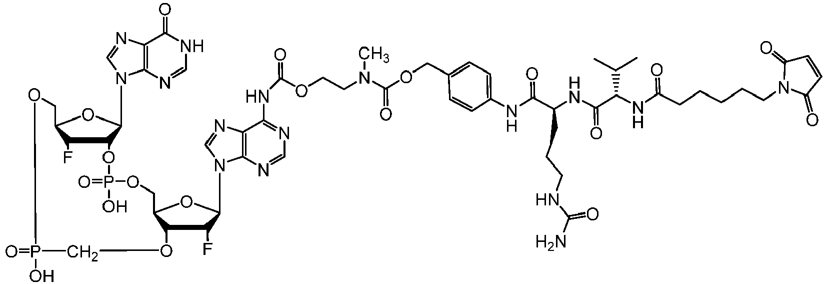 **(Comparative Compound)** |
| | Compound 3.2 and MC-Val-Cit-PAB-PNP (CAS 159857-81-5) |
| | UPLC-MS (Method A1): Rt = 2.05 min; m/z 1377.4 [M+H]⁺. ESI-HRMS calculated for C₅₄H₆₇O₂₁N₁₆F₂P₂ (M-H)⁻ 1375.41155, found 1375.41019. NMR Tables 7-8. |

**Table 7. ¹H, ³¹P and ¹⁹F NMR data of Compounds 3.4-3.11 in DMSO-d6.**

| **Cmpd** | **Unit** | **H-1'** | **H-2'** | **H-3'** | **H-4'** | **H-5'a** | **H-5'b** | **P-CHa-O** | **P-CHb-O** | **H-2,8** |
|---|---|---|---|---|---|---|---|---|---|---|
| **3.4** | Hpx | 6.11 d (8.5) | 5.46 dddd (24.9; 12.0; 8.5; 4.0) | 5.31 dd (54.0; 4.0) | 4.63 dq (27.4; 2.3; 2.3; 2.3) | 4.22 m | 3.885 dt | -- | -- | 8.09 d (3.7); 8.35 s |
| | A | 6.46 d (18.9) | 5.89 dd (52.0; 3.6) | 5.12 ddd (24.7; 8.4; 3.6) | 4.34 m | 4.29 m | 3.92 m | 4.17 dd (14.0; 1.8) | 4.08 dd (14.0; 5.4) | 8.67 s; 8.47 s |
| | **Linker: Mal:** 6.99 s (CH=); **C6:** 3.36 t (2H), 1.47 m (2H), 1.18 m (2H), 1.48 m (2H); 2.11 dt (1H), 2.18 dt (1H) (5x CH₂); **Val:** 7.79 d (NH), 4.19 dd (N-CH), 1.96 m (CH), 0.845 d and 0.81 d (2x CH₃); **Cit:** 8.065 d (NH), 4.38 m (N-CH<), 1.69 m and 1.59 m (CH₂), 1.43 m and 1.36 m, (CH₂), 3.02 m and 2.94 m (N-CH₂); **PAB:** 9.99 s and 9.97 s (NH), 7.58 m, 7.54 m, 7.30 m and 7.26 m (4x ArH), 5.00 bs and 4.98 bs (O-CH₂); **SE:** 4.25 m (O-CH₂), 3.52 m (N-CH₂), 2.95 s and 2.92 s (N-CH₃), **³¹P** 57.69, 79.88. **¹⁹F** -191.67, -198.78. | | | | | | | | | |
| **3.5** | Hpx | 6.10 d (8.6) | 5.45 dddd (25.5; 8.5; 12.0; 4.0) | 5.32 dd (53.8; 4.0) | 4.62 dq (27.4; 2.2; 2.2; 2.2) | 4.25 bd (12.0) | 3.90 bd (12.0) | -- | -- | 8.09 d (3.0); 8.36 s |
| | A | 6.45 d (19.0) | 5.88 dd (52.0; 3.8) | 5.15 ddd (24.6; 8.4; 3.8) | 4.33 m | 4.20 m | 3.88 m | 4.17 bd (14.5) | 4.05 dd (14.5; 4.5) | 8.66 s; 8.48 s |
| | **Linker: Mal-PEG:** 6.98 s (CH=), 3.58 m (N-CH₂), 2.32 dd (CH₂-CO), 3.14 q (N-CH₂), 3.35 t (O-CH₂), 3.48 m (6x O-CH₂), 3.59 m (O-CH₂), 2.47 m and 2.37 m (CH₂-CO), **Val:** 7.86 d (NH), 4.22 dd (N-CH), 1.96 m (CH), 0.85 d and 0.82 d (2x CH₃); **Cit:** 8.10 d (NH), 4.38 ddd (N-CH), 1.69 m and 1.58 m (CH₂), 1.42 m and 1.36 m (CH₂), 3.02 m and 2.93 m (N-CH₂); **PAB:** 9.98 d (NH), 7.63 m, 7.54 m, 7.30 m and 7.26 m (4x ArH), 5.00 band 4.97 b (O-CH₂); **SE:** 2.95 s and 2.92 s (N-CH₃), 3.52 m (N-CH₂), 4.25 m (O-CH₂). **³¹P** 57.50, 79.19. **¹⁹F** -191.82, -198.64. | | | | | | | | | |
| **3.6** | Hpx | 6.11 d (8.6) | 5.45 dddd (25.2; 8.6; 12.0; 4.0) | 5.31 dd (54.0; 4.0) | 4.62 dq (27.4; 2.2; 2.2; 2.2) | 4.28 m | 3.98 m | -- | -- | 8.09 d (3.6); 8.35 s |
| | A | 6.46 d (18.8) | 5.89 dd (51.8; 3.8) | 5.13 ddd (24.6; 8.4; 3.8) | 4.34 m | 4.21 m | 3.88 m | 4.17 bd (14.0) | 4.08 dd (14.0; 5.0) | 8.66 s; 8.47 s |
| | **Linker: Mal-C6:** 6.98 s (CH=), 3.36 t (N-CH₂), 1.47 m (2x CH₂), 1.18 (CH₂), 2.18 dt and 2.11 dt (CH₂-CO); **Val:** 7.80 d (NH), 4.17 dd (N-CH), 1.96 m (CH), 0.86 d and 0.82 d (2x CH₃); **Ala:** 8.14 d (NH), 4.38 p (N-CH), 1.30 d (CH₃); **PAB:** 9.93 d (NH), 7.57 m, 7.53 m, 7.30 m and 7.26 m (4x ArH), 5.00 b and 4.97 b (O-CH₂); **SE:** 2.95 s and 2.92 s (N-CH₃), 3.52 m (N-CH₂), 4.25 m (O-CH₂). **³¹P** 57.69, 79.79. **¹⁹F** -191.69, -198.81. | | | | | | | | | |
| **3.7** | Hpx | 6.09 d | 5.45 dddd | 5.32 dd | 4.61 dm | 4.24 m | 3.90 dm | -- | -- | 8.09 d (3.7) |
| | | (8.4) | (25.2; 8.4; 12.4; 4.0) | (53.8; 4.0) | (27.0) | | (11.5) | | | 8.36 s |
| | A | 6.45 d | 5.88 dd | 5.16 ddd | 4.32 dm | 4.21 m | 3.86 dm | 4.17 dd | 4.08 dd | 8.66 s |
| | | (18.6) | (52.0; 3.4) | (25.0; 8.4; 3.4) | (8.4) | | (11.6) | (14.2; 1.8) | (14.2; 5.3) | 8.48 s |
| | **Linker: Mal-GlySar**: 7.09 s (CH=), 4.16 s (N-CH₂), 2.76 s (N-CH₃), 4.30 s N-CH₂); **Val:** 7.95 d (NH), 4.23 dd (N-CH), 1.98 m (CH), 0.86 d and 0.82 d (2x CH₃); **Cit:** 8.17 d (NH), 4.38 ddd (N-CH), 1.69 m and 1.54 m (CH₂), 1.44 m and 1.37 m (CH₂), 3.02 m and 2.93 m (N-CH₂); **PAB:** 10.03 b and 9.96 b (NH), 7.57 m, 7.54 m, 7.30 m and 7.26 m (4x ArH), 5.00 bs and 4.97 bs (O-CH₂); **SE:** 2.95 s and 2.92 s (N-CH₃), 3.52 m (N-CH₂), 4.25 m (O-CH₂). **³¹P** 57.02, 78.82. **¹⁹F** -191.73, -198.89. | | | | | | | | | |
| **3.8** | Hpx | 6.11 d (8.6) | 5.46 dddd (25.4; 8.6; 12.4; 4.2) | 5.31 dd (54.2; 4.2) | 4.62 dq (27.3; 2.3; 2.3; 2.2) | 4.28 m | 3.91 dq (11.6; 2.3; 2.3; 2.3) | -- | -- | 8.09 d (3.8); 8.35 s |
| | A | 6.46 dd (19.0; 0.9) | 5.89 ddd (52.2; 3.8; 0.9) | 5.13 ddd (25.0; 8.6; 3.8) | 4.34 dm (8.6) | 4.23 bdd (11.7; 5.5) | 3.89 ddd (11.7; 4.5; 2.5) | 4.17 dd (14.2; 1.8) | 4.08 dd (14.2; 5.3) | 8.67 s; 8.47 s |
| | **Linker: Mal-GlyGly:** 7.09 s (CH=), 4.08 s (N-CH₂), 8.49 t (NH), 3.80d (N-CH₂); **Val:** 7.86 d (NH), 4.24 dd (N-CH), 1.98 m (CH), 0.86 d and 0.82 d (2x CH₃); Cit: 8.18 d (NH), 4.37 ddd (N-CH), 1.69 m and 1.57 m (CH₂), 1.44 m and 1.36 m (CH₂), 3.03 m and 2.94 m (N-CH₂); **PAB:** 9.96 bs and 9.95 bs (NH), 7.59 m, 7.55 m, 7.31 m and 7.26 m (4x ArH), 5.00 bs and 4.97 bs (O-CH₂); **SE:** 2.95 s and 2.92 s (N-CH₃), 3.52 m (N-CH₂), 4.25 m (O-CH₂). **³¹P** 57.55, 79.66. **¹⁹F** -191.69, -198.80. | | | | | | | | | |
| **3.9** | Hpx | 6.12d (8.6) | 5.46 dddd (24.9; 8.6; 12.2; 4.0) | 5.31 dd (53.9; 4.0) | 4.63 dm (27.3) | 4.30 ddd (11.6; 7.7; 1.8) | 3.92 dq (11.6; 2.4; 2.4; 2.4) | -- | -- | 8.09 d (3.7); 8.34 s |
| | A | 6.46 dd (18.7; 0.8) | 5.89 ddd (51.6; 3.8; 0.8)) | 5.12 ddd (24.8; 8.6; 3.8) | 4.34 m | 4.21 m | 3.90 m | 4.17 dd (14.2; 1.7) | 4.09 dd (14.2; 5.4) | 8.67 s; 8.46 s |
| | **Linker: Mal-Gly:** 7.08 s (CH=), 4.01 s (N-CH₂); **PEG:** 8.22 t (NH), 3.20 q (N-CH₂); 3.40 t (O-CH₂); 3.48 - 3.51 m (11x O-CH₂-), 2.47 and 2.48 (N-CH₂); **Val:** 7.86 d (NH), 4.23 dd (N-CH), 1.96 m (CH), 0.85 d and 0.82 d (2x CH₃); **Cit:** 8.10 d (NH), 4.38 ddd (N-CH), 1.69 m and 1.59 m (CH₂), 1.44 m and 1.36 m (CH₂), 3.03 m and 2.94 m (N-CH₂); **PAB:** 9.99 bs and 9.97 bs (NH), 7.56 m (2x ArH), 7.28 m (2x ArH), 5.00 bs and 4.97 bs (O-CH₂); **SE:** 2.95 s and 2.92 s (N-CH₃), 3.52 m (N-CH₂), 4.26 m (O-CH₂). **³¹P** 57.88, 80.04. **¹⁹F** -191.70, -198.69. | | | | | | | | | |
| **3.10** | Hpx | 6.12d (8.5) | 5.46 dddd (25.5; 8.5; 12.0; 4.1) | 5.31 dd (54.0; 4.1) | 4.64 dq (27.6; 2.3 2.3; 2.3) | 4.30 m | 3.92 m | -- | -- | 8.10 d (3.8); 8.34 s |
| | A | 6.46 d (19.4) | 5.89 ddd (52.1; 3.9) | 5.11 ddd (24.6; 8.6; 3.9) | 4.34 m | 4.22 m | 3.92 m | 4.18 dd (14.0; 1.7) | 4.10 dd (14.0; 5.7) | 8.66 s; 8.46 s |
| | **Linker: Mal-Gly:** 7.07 s (CH=), 4.01 s (N-CH₂); **PEG:** 8.17 t (NH), 3.19 q (N-CH₂); 3.38 t (O-CH₂), 3.60 td (O-CH₂), 2.41 td (N-CH₂); **Ala:** 8.19 d (NH), 4.42 p (N-CH), 1.28 d (CH₃); **PAB:** 10.03 bs and 10.01 bs (NH), 7.57, 7.52, 7.30 and 7.25 m (4x ArH), 5.00 bs and 4.97 bs (O-CH₂); **SE:** 2.95 s and 2.92 s (N-CH₃), 3.57 m and 3.52 m (N-CH₂), 4.26 m (O-CH₂). **³¹P** 58.14, 80.16. **¹⁹F** -191.72, -198.65. | | | | | | | | | |
| **3.11** | Hpx | 6.14 d (8.6) | 5.47 dddd (24.6; 9.4; 8.6; 3.8) | 5.39 dd (54.0; 3.8) | 4.62 dt (27.0; 3.6; 3.6) | 4.225 m | 4.075 dt | -- | -- | 8.09 d (3.6); 8.35 s |
| | A | 6.46 d (19.0) | 5.92 dd (52.0; 3.8) | 4.90 ddd (23.5; 8.0; 3.8) | 4.35 m | 4.22 m | 4.01 m | n.d. | n.d. | 8.65 s; 8.46 s |
| | **Linker: Mal:** 6.99 s (CH=); **C6:** 3.355 t (2H), 1.465 m (2H), 1.17 m (2H), 1.48 m (2H); 2.11 dt (1H), 2.17 dt (1H) (5x CH₂); **Val:** 7.81 d (NH), 4.19 dd (N-CH), 1.955 m (CH), 0.84 d and 0.81 d (2x CH₃); Cit: 8.075 d (NH), 4.37 m (N-CH), 1.68 m and 1.58 m (CH₂), 1.43 m and 1.355 m, (CH₂), 3.01 m and 2.94 m (N-CH₂); **PAB:** 9.99 s (NH), 7.57 m, 7.51 m, 7.285 m and 7.23 m (4x ArH), 4.97 b (O-CH₂); **SE:** 4.25 m (O-CH₂), 3.52 m (N-CH₂), 2.94 s and 2.91 s (N-CH₃), **³¹P** -1.91, 18.64. **¹⁹F** -192.89, -198.67. | | | | | | | | | |

**Table 8. ¹³C NMR data of Compounds 3.4-3.11 in DMSO-d6.**

| **Cmpd** | **Unit** | **C-1'** | **C-2'** | **C-3'** | **C-4'** | **C-5'** | **P-CH₂-O** | **C-2** | **C-4** | **C-5** | **C-6** | **C-8** |
|---|---|---|---|---|---|---|---|---|---|---|---|---|
| **3.4** | Hpx | 83.55 (3.2) | 74.13 | 92.13 (184.8) | 81.58 (23.0; 11.1) | 63.09 | -- | 146.40 | 149.21 | 124.11 | 156.73 | 138.47 |
| | A | 86.28 (33.7) | 91.73 (187.2) | 76.32 (16.0; 8.0) | 78.80 (10.5) | 64.45 | 68.45 (123.0) | 152.04 | 151.14 | 123.46 | 149.87 | 141.71 |
| | **Linker: Mal:** 171.24 (C=O), 134.62 (CH=); **C6:** 37.18 (N-CH₂), 27.93 (CH₂), 25.96 (CH₂), 25.09 (CH₂), 35.11 (CH₂), 172.41 (C=O); **Val:** 171.44 (C=O), 57.70 (N-CH), 30.58 (CH), 19.42 and 18.36 (2x CH₃); **Cit:** 170.73 (C=O), 53.22 (N-CH), 29.49 (CH₂), 26.91 (CH₂), 38.82 (N-CH₂), 159.11 (C=O); **PAB:** 138.77 (ArC), 119.19 (ArCH), 119.12 (ArCH), 128.60 (ArCH), 128.53 (ArCH), 131.79 (ArC), 66.26 (O-CH₂), **SE:** 152.20 (C=O),), 63.21 (O-CH₂), 47.91 and 47.36 (N-CH₂), 35.24 (N-CH₃), 155.74 and 155.49 (C=O). | | | | | | | | | | | |
| **3.5** | Hpx | 83.52 | 74.08 (15.2; 8.1) | 92.24 (185.9) | 81.64 (23.4; 10.0) | 64.54 (12.7; 5.1) | -- | 146.45 | 149.96 | 124.11 | 156.78 | 138.48 |
| | A | 86.32 (33.6) | 91.81 (187.1) | 76.39 (13.9; 8.6) | 78.92 (11.1) | 63.20 (3.8) | 68.37 (122.2) | 152.10 | 151.17 | 123.45 | 149.90 | 141.78 |
| | **Mal:** 170.37 (C=O), 134.76 (CH=), 34.28 (CH₂), 34.16 (CH₂), 169.76 (C=O), **PEG:** 38.71 (N-CH₂), 69.18, 69.98(2), 69.91(2), 69.75, 69.68, 67.13 (8x O-CH₂), 36.13 (CH₂), 170.55 (C=O); **Val:** 171.36 (C=O), 57.70 (N-CH), 30.81 (CH), 19.40 and 18.31 (2x CH₃); **Cit:** 170.77 (C=O), 53.30 (CH), 29.49 (CH₂), 26.94 (CH₂), 38.83 (N-CH₂), 159.21 (C=O); **PAB:** 138.75 (ArC), 119.26, 119.18, 128.66 and 128.97 (4x ArCH), 131.86 (ArC), 66.32 (O-CH₂), **SE:** 152.24 (C=O), 63.20 (CH₂), 47.69 (CH₂), 35.42 (CH₃), 155.56 (CO). | | | | | | | | | | | |
| **3.6** | Hpx | 83.54 | 74.10 | 92.14 (185.1) | 81.58 (22.5; 9.0) | 64.48 (12.0 ) | -- | 146.40 | 149.21 | 124.10 | 156.72 | 138.48 |
| | A | 86.26 (34.7) | 91.74 (187.1) | 76.34 | 78.83 (10.5) | 63.23 (10.6) | 68.43 (121.3) | 152.05 | 151.14 | 123.47 | 149.88 | 141.70 |
| | **Linker: Mal-C6:** 171.24 (CO), 134.61 (CH=), 37.19 (N-CH₂), 27.94, 25.96, 25.07 and 35.08 (4x - CH₂-), 172.43 (CO); ); **Val:** 171.28 (CO), 57.64 (CH), 30.58 (CH), 19.39 and 18.35 (2x CH₃); **Ala:** 171.18 (CO), 49.18 (CH), 18.14 (CH₃); **PAB:** 138.83 (ArC), 119.19, 119.11, 128.58 and 128.50 (4x ArCH), 131.76 (ArC), 66.27 (O-CH₂), **SE:** 152.21 (C=O), 63.14 (O-CH₂), 47.36 (N-CH₂), 35.14 and 35.44 (N-CH₃), 155.74 and 155.51 (C=O). | | | | | | | | | | | |
| **3.7** | Hpx | 83.50 | 73.94 | 92.28 (186.1) | 81.61 (23.0; 10.0) | 64.51 | -- | 146.38 | 149.24 | 124.06 | 156.74 | 138.50 |
| | A | 86.19 (33.9) | 91.79 (187.1) | 76.22 | 78.98 (11.0) | 63.09 | 68.33 (117.0) | 152.19 | 151.16 | 123.44 | 149.89 | 141.70 |
| | **Linker: Mal-GlySar:** 170.92 and 170.90 (C=O), 135.15 and 135.08 (CH=), 51.13 (N-CH₂), 166.77 (C=O), 34.65 (N-CH₃), 57.85 (N-CH₂), 168.02 and 168.67 (C=O); **Val:** 171.11 and 171.00 (C=O), 57.70 (N-CH), 30.86 (CH), 19.36 and 18.22 (2x CH₃); **Cit:** 170.71 (C=O), 53.27 (N-CH), 29.45 (CH₂), 26.95 (CH₂), 38.88 (N-CH₂), 159.14 (C=O); **PAB:** 138.76 (ArC), 119.18 (2), 128.62 and 128.53 (4x ArCH), 131.79 (ArC), 66.28 (O-CH₂), **SE:** 152.19 (C=O), 63.23 (O-CH₂), 47.61 (N-CH₂), 35.26 (N-CH₃), 155.75 and 155.51 (C=O). | | | | | | | | | | | |
| **3.8** | Hpx | 83.53 | 74.10 (16.0; 8.0) | 92.16 (184.7; 3.8) | 81.58 (24.1; 9.8) | 64.47 (12.2) | -- | 146.40 | 149.21 | 124.10 | 155.74 | 138.48 |
| | A | 86.26 (33.7) | 91.74 (187.7) | 76.32 | 78.84 (10.4) | 63.21 (9.2) | 68.41 (122.0) | 152.05 | 151.14 | 123.46 | 149.86 | 141.70 |
| | **Linker: Mal-GlyGly:** 170.80 (C=O), 135.14 (CH=), 39.75 (N-CH₂), 166.64 (C=O), 42.14 (N-CH₂), 168.67 (C=O); **Val:** 171.09 (C=O), 57.62 (N-CH), 30.84 (CH), 19.33 and 18.19 (2x CH₃); **Cit:** 170.71 (C=O), 53.36 (N-CH), 29.37 (CH₂), 26.97 (CH₂), 38.78 (N-CH₂), 159.12 (C=O); **PAB:** 138.72 (ArC), 119.21, 119.14, 128.61 and 128.53 (4x ArCH), 131.78 (ArC), 66.28 (O-CH₂), **SE:** 152.20 (C=O), 63.13 (O-CH₂), 47.92 and 47.37 (N-CH₂), 35.39 and 35.12 (N-CH₃), 155.74 and 155.50 (C=O). | | | | | | | | | | | |
| **3.9** | Hpx | 83.55 | 74.17 (15.2; 9.4) | 92.12 (185.6; 3.8) | 81.58 (22.8; 10.4) | 64.46 | -- | 146.42 | 149.21 | 124.12 | 156.73 | 138.46 |
| | A | 86.32 (34.6) | 91.73 (187.1) | 76.42 | 78.76 (10.3) | 63.26 | 68.46 (123.8) | 152.05 | 151.14 | 123.47 | 149.88 | 141.73 |
| | **Mal-Gly:** 170.86 (C=O), 135.04 (CH=), 39.76 (N-CH₂), 166.31 (C=O); **PEG:** 38.97 (N-CH₂), 69.07, 69.95(5), 69.88(2), 69.79, 69.64, 67.10, 66.53 (12x O-CH₂), 36.10 (-CH₂), 170.46 (C=O); **Val:** 171.31 (C=O), 57.63 (N-CH), 30.79 (CH), 19.36 and 18.28 (2x CH₃); **Cit:** 170.73 (C=O), 53.25 (N-CH), 29.46 (CH₂), 26.90 (CH₂), 38.74 (N-CH₂), 159.14 (C=O); **PAB:** 138.78 and 138.73 (ArC), 119.21, 119.13, 128.62 and 128.54 (4x ArCH), 131.81 (ArC), 66.28 (O-CH₂), **SE:** 152.22 (C=O), 63.11 (O-CH₂), 47.36 (N-CH₂), 35.33 and 35.11 (N-CH₃), 155.50 (C=O). | | | | | | | | | | | |
| **3.10** | Hpx | 83.56 | 74.23 (15.6; 8.9) | 92.08 (183.4; 3.4) | 81.58 (24.4; 10.0) | 64.44 | -- | 146.44 | 149.21 | 124.14 | 156.73 | 138.46 |
| | A | 86.36 (33.9) | 91.73 (187.3) | 76.47 (14.4; 7.7) | 78.73 (10.8) | 63.28 | 68.48 (122.8) | 152.01 | 151.14 | 123.48 | 149.84 | 141.75 |
| | **Mal-Gly:** 170.84 (C=O), 135.03 (CH=), 39.71 (N-CH₂), 166.29 (C=O); **PEG:** 38.87 (N-CH₂), 68.69 (O-CH₂), 66.67 (O-CH₂), 35.79 (-CH₂), 170.19 (C=O); **Ala:** 171.60 (C=O), 49.14 (N-CH), 18.39 (CH₃); **PAB:** 138.78 and 138.72 (ArC), 119.36, 119.26, 128.54 and 128.46 (4x ArCH), 131.81 | | | | | | | | | | | |
| | (ArC), 66.26 (O-CH₂), **SE:** 152.23 (C=O), 63.10 (O-CH₂), 47.90 and 47.36 (N-CH₂), 35.38 and 35.11 (N-CH₃), 150.75 and 155.51 (C=O). | | | | | | | | | | | |
| **3.11** | Hpx | 83.80 (6.4) | 74.14 (18.0) | 91.56 (181.0) | 81.10 (23.0; 8.5) | 62.75 | -- | 146.46 | 149.09 | 124.54 | 156.76 | 138.77 |
| | A | 86.58 (34.4) | 91.60 (187.8) | 77.27 (14.4) | 78.84 (8.5) | 63.43 | n.d. | 152.14 | 151.12 | 123.70 | 149.98 | 141.84 |
| | **Linker: Mal:** 171.26 (C=O), 134.63 (CH=); **C6:** 37.20 (N-CH₂), 27.95 (CH₂), 25.97 (CH₂), 25.10 (CH₂), 35.19 (CH₂), 172.48 (C=O); **Val:** 171.47 (C=O), 57.78 (N-CH), 30.58 (CH), 19.44 and 18.38 (2x CH₃); **Cit:** 170.74 (C=O), 53.29 (N-CH), 29.49 (CH₂), 26.87 (CH₂), 38.82 (N-CH₂), 159.17 (C=O); **PAB:** 138.77 (ArC), 119.13 (2x ArCH), 128.50 (2x ArCH), 131.72 (ArC), 66.31 (O-CH₂), **SE:** 152.21 (C=O), 63.02 (O-CH₂), 47.63 (N-CH₂), 35.13 (N-CH₃), 155.55 (C=O). | | | | | | | | | | | |

### Example 3.12

### Phenyl (9-((6R,8R,9S,13aR,15R,16R,16aR,17R)-16,17-difluoro-3,11-dimercapto-3,11-dioxido-8-(6-oxo-1,6-dihydro-9H-purin-9-yl)decahydro-6,9-methanofuro[3,2-m][1,3,6,9,12]pentaoxa[2,10]diphosphacyclopentadecin-15-yl)-9H-purin-6-yl)(phenoxycarbonyl)carbamate (3.12)

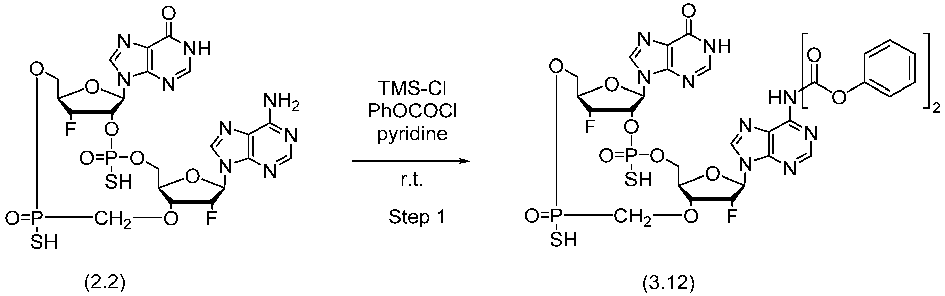

*Step 1:* Trimethylsilyl chloride (0.21 mL; 1.75 mmol) in ACN (1.5 mL) was added to a solution of 9-((6*R*,8*R*,9*S*,13a*R*,15*R*,16*R*,16a*R*,17*R*)-15-(6-amino-9*H*-purin-9-yl)-16,17-difluoro-3,11-dimercapto-3,11 -dioxidodecahydro-6,9-methanofuro [3,2-*m*][1,3,6,9,12]pentaoxa[2,10]diphosphacyclopentadecin-8-yl)-1,9-dihydro-6*H*-purin-6-one diTEA salt (0.32 g; 0.35mmol; Compound 2.2) in pyridine (18 mL) and the reaction mixture was stirred for 45 minutes at r.t. After that, phenyl chloroformate (0.21 mL; 1.75 mmol) in ACN (1.5 mL) was added and reaction mixture was stirred for additional 1.5 h at r.t. The reaction mixture was cooled to 4 °C and water (1 mL) was added. The reaction mixture was left to warm up to r.t. and carefully evaporated. The crude was dissolved in DMSO (5 mL) and directly loaded on C18 column. The product was purified by reverse phase chromatography (Method P2) and lyophilized from dioxane/water (1:1) to yield 0.26 g (64 %) of Compound 3.12 as diTEA salt.
UPLC-MS (Method A1): Rt = 2.47 min; m/z 950.2 [M+H]⁺.
ESI-HRMS calculated for C₃₅H₃₀O₁₃N₉P₂P₂S₂ (M-H)⁻ 948.08533, found 948.08422.

**Table 9. ¹H, ³¹P and ¹⁹F NMR data of Compound 3.12 in DMSO-d6.**

| **Unit** | **H-1'** | **H-2'** | **H-3'** | **H-4'** | **H-5'a** | **H-5'b** | **P-CHa-O** | **P-CHb-O** | **H-2,8** |
|---|---|---|---|---|---|---|---|---|---|
| Hpx | 6.06 d (8.5) | 5.52 dddd (26.4; 8.5; 12.8; 3.9) | 5.37 dd (54.7; 3.9) | 4.50 dq (26.8; 2.3; 2.2; 2.2) | 3.87 ddd (11.8; 8.2; 2.2) | 3.74 dq (11.8; 2.3; 2.3; 2.3) | -- | -- | 8.07 s; 8.49 s |
| A | 6.48 d (19.0) | 5.94 dd (52.0; 3.4) | 5.65 ddd (27.8; 9.4; 3.4) | 4.27 dq (9.4; 2.8; 2.0; 2.0) | 4.16 dt (12.4; 2.8; 2.8) | 3.69 ddd (12.4; 4.4; 2.0) | 4.04 dd (14.6; 4.2) | 3.64 dd (14.6; 2.1) | 9.10 s; 8.90 s |
| **Linker: 2x CO-O-C₆H₅:** 7.25 m (4x o-ArH), 7.47 m (4x m-ArH), 7.31 m (2x p-ArH). **³¹P** 54.28, 68.77. **¹⁹F** -191.99, -198.80. | | | | | | | | | |

**Table 10. ¹³C NMR data of Compound 3.12 in DMSO-d6.**

| **Unit** | **C-1'** | **C-2'** | **C-3'** | **C-4'** | **C-5'** | **P-CH₂-O** | **C-2** | **C-4** | **C-5** | **C-6** | **C-8** |
|---|---|---|---|---|---|---|---|---|---|---|---|
| Hpx | 83.63 | 73.59 (15.1; 8.3) | 93.38 (182.2) | 82.26 (22.4; 9.0) | 64.54 (12.7; 5.1) | -- | 146.08 | 149.32 | 123.78 | 156.82 | 138.96 |
| A | 85.83 (34.0) | 92.34 (186.4) | 73.37 (15.6) | 79.81 (11.6) | 62.28 (3.8) | 67.36 (103.8) | 152.55 | 153.29 | 128.35 | 147.73 | 145.39 |
| **Linker: 2x CO-O-C₆H₅:** 149.78 (C=O), 154.02 (i-ArC), 121.47 (2x o-ArC), 130.12 (2x m-ArC), 126.96 (p-ArC). | | | | | | | | | | | |

### Example 3.13

### 4-((S)-2-((S)-2-(2-(2,5-dioxo-2,5-dihydro-1H-pyrrol-1-yl)acetamido)-3-methylbutanamido)-5-ureidopentanamido)benzyl (9-((6R,8R,9S,13aR,15R,16R,16aR,17R)-16,17-difluoro-3,11-dimercapto-3,1 1-dioxido-8-(6-oxo-1,6-dihydro-9H-purin-9-yl)decahydro-6,9-methanofuro[3,2-m][1,3,6,9,12]pentaoxa[2,10]diphosphacyclopentadecin-15-yl)-9H-purin-6-yl)carbamate (3.13)

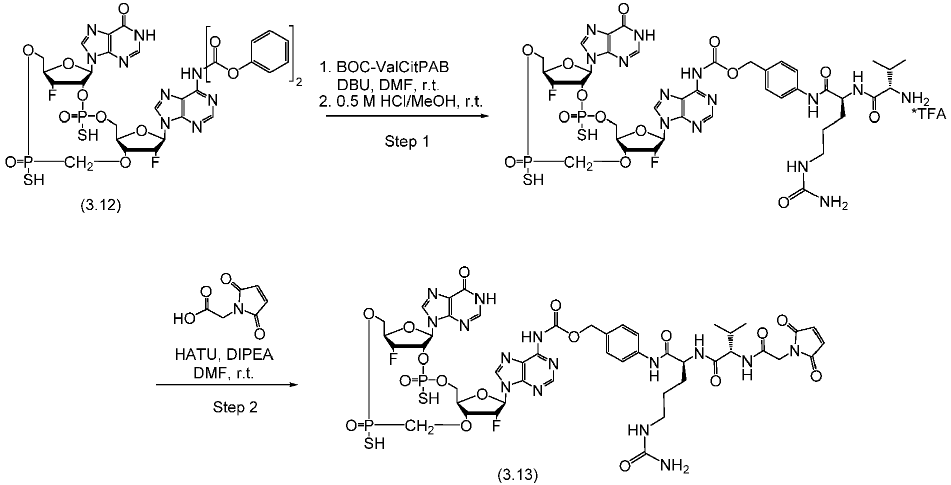

*Step 1:* DBU (61 µL; 435 µmol) was added to a solution of BOC-ValCitPAB (42 mg; 87 µmol; CAS 870487-09-5) and phenyl (9-((6*R*,8*R*,9*S*,13a*R*,15*R*,16*R*,16a*R*,17*R*)-16,17-difluoro-3,11-dimercapto-3,11-dioxido-8-(6-oxo-1,6-dihydro-9*H*-purin-9-yl)decahydro-6,9-methanofuro[3,2-*m*][1,3,6,9,12]pentaoxa[2,10]diphosphacyclopentadecin-15-yl)-9*H*-purin-6-yl)(phenoxycarbonyl)carbamate diTEA salt (100 mg; 87 µmol; Compound 3.12) in DMF (8 mL) and the reaction mixture was stirred for 6 h at r.t. The reaction mixture was evaporated. After that, the crude was treated with 0.5 M HCl in methanol (8 mL) for 16 h at r.t. and evaporated. The product was purified by reverse phase chromatography (Method P6) and lyophilized from dioxane/water (1:1) to yield 51 mg (48 %) of 4-((*S*)-2-((*S*)-2-amino-3-methylbutanamido)-5-ureidopentanamido)benzyl (9-((6*R*,8*R*,9*S*,13a*R*,15*R*,16*R*,16a*R*,17*R*)-16,17-difluoro-3,11-dimercapto-3,11-dioxido-8-(6-oxo-1,6-dihydro-9*H*-purin-9-yl)decahydro-6,9-methanofuro[3,2-*m*][1,3,6,9,12]pentaoxa[2,10]diphosphacyclopentadecin-15-yl)-9*H*-purin-6-yl)carbamate TFA salt. UPLC-MS (Method A1): Rt = 1.81 min; m/z 1113.5 [M-H]⁻.
ESI-HRMS calculated for C₄₀H₅₁O₁₄N₁₄F₂P₂S₂ (M+H)⁺ 1115.25884, found 1115.25895.

*Step 2:* 2-(2,5-dioxo-2,5-dihydro-1H-pyrrol-1-yl)acetic acid (3.6 mg; 22 µmol) and HATU (8.4 mg; 22 µmol) in DMF (1 mL) were stirred for 30 minutes at r.t. After that, 4-((*S*)-2-((*S*)-2-amino-3-methylbutanamido)-5-ureidopentanamido)benzyl (9-((6*R*,8*R*,9*S*,13a*R*,15*R*,16*R*,16a*R*,17*R*)-16,17-difluoro-3,11-dimercapto-3,11-dioxido-8-(6-oxo-1,6-dihydro-9*H*-purin-9-yl)decahydro-6,9-methanofuro[3,2-*m*][1,3,6,9,12]pentaoxa[2,10]diphosphacyclopentadecin-15-yl)-9*H*-purin-6-yl)carbamate TFA salt (27 mg; 22 µmol) and DIPEA (15.4 µL; 88 µmol) in DMF (5 mL) were added, and the reaction mixture was stirred for 1 h at r.t. The product was purified by reverse phase chromatography (Method P4) and lyophilized from dioxane/water (1:1) to yield 14 mg (47 %) of Compound 3.13.
UPLC-MS (Method A1): Rt = 1.91 min; m/z 1250.6 [M-H]⁻.
ESI-HRMS calculated for C₄₆H₅₂O₁₇N₁₅F₂P₂S₂ (M-H)⁻ 1250.25558, found 1250.25464.

**Table 11. ¹H, ³¹P and ¹⁹F NMR data of Compound 3.13 in DMSO-d6.**

| **Unit** | **H-1'** | **H-2'** | **H-3'** | **H-4'** | **H-5'a** | **H-5'b** | **P-CHa-O** | **P-CHb-O** | **H-2,8** |
|---|---|---|---|---|---|---|---|---|---|
| Hpx | 6.10 d (8.6) | 5.46 dddd (25.4; 8.6; 12.1; 3.8) | 5.31 dd (54.0; 3.8) | 4.62 dq (25.4; 2.3; 2.3; 2.3) | 4.27 m | 3.91 dq (11.6; 2.4; 2.4; 2.4) | -- | -- | 8.09 d (3.8); 8.36 s |
| A | 6.46 dd (18.6; 0.9) | 5.88 ddd (51.8; 3.8; 0.9) | 5.13 ddd (24.8; 8.5; 3.8) | 4.34 m | 4.21 ddd (11.6; 5.6; 4.0) | 3.87 ddd (11.6; 4.4; 2.3) | 4.16 dd (14.0; 2.0) | 4.07 dd (14.0; 5.0) | 8.67 s; 8.48 s |
| **Linker: Mal-Gly:** 7.08 s (CH=), 4.13 s (N-CH₂); **Val:** 8.25 d (NH), 4.25 dd (N-CH), 1.98 m (CH), 0.86 d and 0.82 d (2x CH₃); Cit: 8.25 d (NH), 4.38 ddd (N-CH), 1.70 m and 1.60 m (CH₂), 1.44 m and 1.37 m (CH₂), 3.03 dt and 2.94 dt (N-CH₂); **PAB:** 10.03 bs (NH), 7.61 m (2x ArH), 7.40 m (2x ArH), 5.17 s (O-CH₂). **³¹P** 57.36, 79.57. **¹⁹F** -191.69, -198.91. | | | | | | | | | |

**Table 12. ¹³C NMR data of Compound 3.13 in DMSO-d6.**

| **Unit** | **C-1'** | **C-2'** | **C-3'** | **C-4'** | **C-5'** | **P-CH₂**-**O** | **C-2** | **C-4** | **C-5** | **C-6** | **C-8** |
|---|---|---|---|---|---|---|---|---|---|---|---|
| Hpx | 83.54 (3.2) | 74.00 | 92.18 (185.3; 4.5) | 81.56 (23.2; 11.0) | 64.48 | -- | 146.39 | 149.22 | 124.09 | 156.74 | 138.55 |
| A | 86.20 (33.7) | 91.77 (187.1) | 76.15 | 78.87 (11.0) | 63.13 | 68.37 (123.0) | 152.11 | 151.18 | 123.52 | 149.95 | 141.66 |
| **Linker: Mal-Gly:** 170.88 (C=O), 135.03 (CH=), 39.70 (N-CH₂), 166.24 (C=O); **Val:** 170.97 (C=O), 57.66 (N-CH), 31.15 (CH), 19.31 and 18.14 (2x CH₃); **Cit:** 170.76 (C=O), 53.38 (N-CH), 29.36 (CH₂), 26.99 (CH₂), 38.80 (N-CH₂), 159.10 (C=O); **PAB:** 139.00 (ArC), 119.12 (2x ArCH), 129.02 (2x ArCH), 131.11 (ArC), 66.40 (O-CH₂), **SE:** 152.27 (C=O). | | | | | | | | | | | |

The compounds listed in the table below were prepared following an analogous procedure to the one above using commercially available or described starting materials (use of appropriate reagents and purification methods known to the skilled in the art):

| **Cmpd** | **Structure, reaction conditions and characterization** |
|---|---|
| **3.14** | 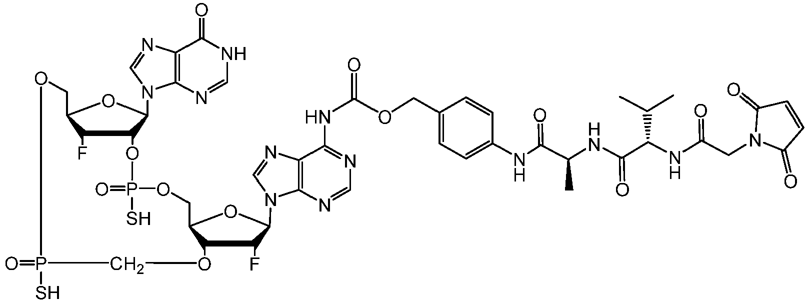 |
| | Starting materials: Compound 3.12 and BOC-ValAlaPAB (CAS 1884577-99-4). |
| | UPLC-MS (Method A1): Rt = 2.05 min; m/z 1164.5 [M-H]⁻. ESI-HRMS calculated for C₄₃H₄₅O₁₆N₁₃F₂P₂S₂ (M-2H)²⁻z=2 581.60014, found 581.60024. NMR Tables 13-14. |
| **3.15** | 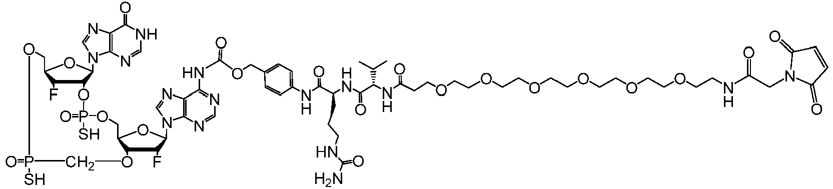 |
| | Starting materials: Compound 3.12 and Compound 1.4. |
| | UPLC-MS (Method A1): Rt = 1.94 min; m/z 1587.7 [M+H]⁺. ESI-HRMS calculated for C₆₁H₈₁O₂₄N₁₆F₂P₂S₂ (M-H)⁻ 1585.44998, found 1585.44922. NMR Tables 13-14. |
| **3.16** | 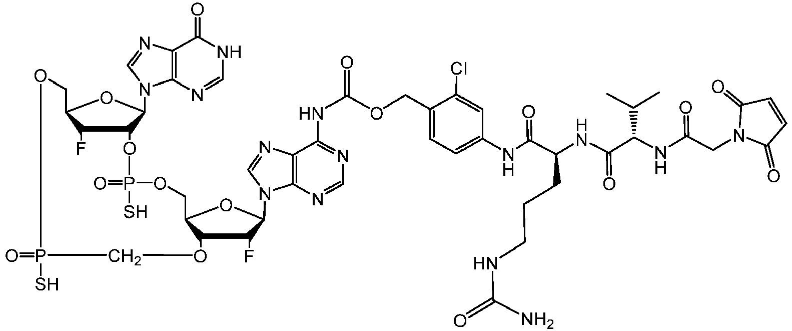 |
| | Starting materials: Compound 3.12 and Compound 1.7. |
| | UPLC-MS (Method A1): Rt = 2.09 min; m/z 1285.5 [M-H]⁻. ESI-HRMS calculated for C₄₆H₅₁O₁₇N₁₅ClF₂P₂S₂ (M-H)⁻ 1284.21661, found 1284.21534. NMR Tables 13-14. |
| **3.17** | 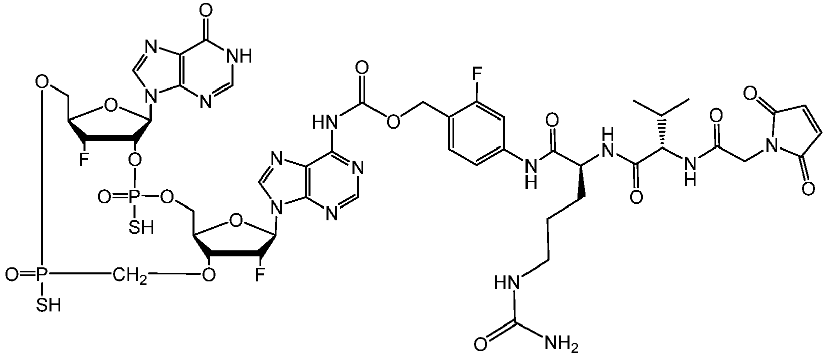 |
| | Starting materials: Compound 3.12 and Compound 1.8. |
| | UPLC-MS (Method A1): Rt = 1.93 min; m/z 1269.2 [M-H]⁻. ESI-HRMS calculated for C₄₆H₅₁O₁₇N₁₅F₃P₂S₂ (M-H)⁻ 1268.24616, found 1268.24521. NMR Tables 13-14. |
| **3.18** | 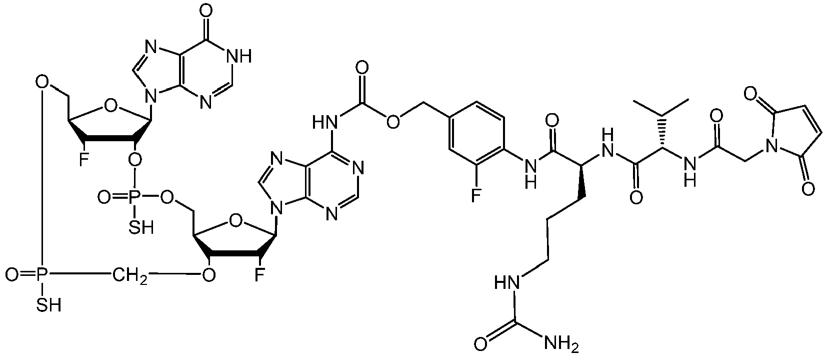 |
| | Starting materials: Compound 3.12 and Compound 1.9. |
| | UPLC-MS (Method A1): Rt = 1.89 min; m/z 1269.8 [M-H]⁻. ESI-HRMS calculated for C₄₆H₅₁O₁₇N₁₅F₃P₂S₂ (M-H)⁻ 1268.24616, found 1268.24533. NMR Tables 13-14. |

**Table 13. ¹H, ³¹P and ¹⁹F NMR data of Compounds 3.14-3.18 in DMSO-d6.**

| **Cmpd** | **Unit** | **H-1'** | **H-2'** | **H-3'** | **H-4'** | **H-5'a** | **H-5'b** | **P-CHa-O** | **P-CHb-O** | **H-2,8** |
|---|---|---|---|---|---|---|---|---|---|---|
| **3.14** | Hpx | 6.08 d (8.6) | 5.45 dddd (25.0; 8.6; 12.0; 3.8) | 5.35 dd (54.0; 3.8) | 4.59 dq (27.2; 2.3; 2.3; 2.3) | 4.17 m | 3.85 m | -- | -- | 8.09 d (3.8); 8.36 s |
| | A | 6.44 d (19.3) | 5.87 dd (52.0; 3.8) | 5.22 ddd (25.0; 8.5; 3.8) | 4.29 m | 4.16 m | 3.85 m | 4.15 m | 3.96 dd (14.0; 3.0) | 8.66 s; 8.47 s |
| | **Linker: Mal Gly:** 7.04 s (CH=), 4.12 s (N-CH₂); **Val:** 8.26 d (NH), 4.17 dd (N-CH), 1.96 m (CH), 0.86 d and 0.82 d (2x CH₃); **Ala:** 8.29 d (NH), 4.36 p (N-CH), 1.30 d (CH₃); **PAB:** 9.97 bs (NH), 7.57 m (2x ArH), 7.38 m (2x ArH), 5.15 s (O-CH₂). **³¹P** 57.03, 77.15. **¹⁹F** -192.24, -198.18. | | | | | | | | | |
| **3.15** | Hpx | 6.11d (8.4) | 5.46 dddd (25.3; 8.4; 12.0; 3.9) | 5.31 dd (51.4; 3.9) | 4.62 dq (27.5; 2.3; 2.3; 2.3) | 4.29 m | 3.92 dq (11.6; 2.3; 2.3; 2.3) | -- | -- | 8.09 d (3.8); 8.35 s |
| | A | 6.46 d (18.7) | 5.89 dd (52.0; 3.8) | 5.12 ddd (24.8; 8.5; 3.8) | 4.34 m | 4.21 m | 3.88 m | 4.17 dd (14.0; 1.7) | 4.08 dd (14.0; 5.2) | 8.67 s; 8.47 s |
| | **Linker: Mal-Gly:** 7.08 s (CH=), 4.01 s (N-CH₂); **PEG:** 8.22 t (NH), 3.20 q (N-CH₂); 3.40 t (O-CH₂); 3.48 - 3.51 m (10x O-CH₂), 3.60 m (O-CH₂), 2.48 and 2.39 (N-CH₂); **Val:** 7.86 d (NH), 4.23 dd (N-CH), 1.97 m (CH), 0.86 d and 0.83 d (2x CH₃); **Cit:** 8.11 d (NH), 4.39 ddd (N-CH), 1.70 m and 1.59 m (CH₂), 1.44 m and 1.36 m (CH₂), 3.03 m and 2.94 m (N-CH₂); **PAB:** 10.01 bs (NH), 7.62 m (2x ArH), 7.40 m (2x ArH), 5.16 s (O-CH₂). **³¹P** 57.67, 79.88. **¹⁹F** -191.69, -198.83. | | | | | | | | | |
| **3.16** | Hpx | 6.11 d (8.6) | 5.46 dddd (25.3; 8.6; 12.2; 4.0) | 5.31 bdd (53.0; 4.0) | 4.63 dq (27.4; 2.3; 2.3; 2.3) | 4.28 m | 3.91 m | -- | -- | 8.095 d (3.8); 8.35 s |
| | A | 6.46 dd (18.8; 0.8) | 5.89 ddd (51.9; 3.9; 0.8) | 5.13 ddd (24.8; 8.6; 3.9) | 4.35 m | 4.21 m | 3.885 m | 4.17 dd (14.0; 2.0) | 4.08 dd (14.0; 5.4) | 8.19 s; 8.26 s |
| | **Linker: Mal-Gly:** 7.08 s (CH=), 4.13 s (N-CH₂-CO); **Val:** 8.26 d (NH), 4.25 dd (N-CH), 1.98 m (CH), 0.86 d and 0.825 d (2x CH₃); **Cit:** 8.30 d (NH), 4.35 m (N-CH), 1.70 m and 1.605 m (CH₂), 1.46 m and 1.365 m (CH₂), 3.025 dt and 2.95 dt (N-CH₂); **PAB-2-CI:** 10.23 s (NH), 7.905 d (ArH), 7.60 d (ArH), 7.48 dd (ArH), 5.24 s (O-CH₂). **Hpx N1-H** 12.43 bd (3.8). **³¹P** 57.75, 79.88. **¹⁹F** -191.71, -198.76. | | | | | | | | | |
| **3.17** | Hpx | 6.12 d (8.6) | 5.465 dddd (25.1; 8.6; 12.1; 4.2) | 5.31 dd (53.9; 4.2) | 4.63 dq (27.5; 2.3; 2.3; 2.3) | 4.30 m | 3.92 m | -- | -- | 8.10 d (3.8); 8.35 s |
| | A | 6.56 dd (19.0; 0.7) | 5.89 ddd (51.9; 3.7; 0.7) | 5.11 ddd (24.9; 8.7; 3.7) | 4.34 dm (8.7;4.8; 4.6) | 4.215 dd (11.5; 4.8) | 3.895 ddd (11.5; 4.6; 2.3) | 4.17 dd (13.8; 2.0) | 4.09 dd (13.8; 5.7) | 8.67 s; 8.46 s |
| | **Linker: Mal-Gly:** 7.08 s (CH=), 4.13 s (N-CH₂); **Val:** 8.25 d (NH), 4.25 dd (N-CH), 1.98 m (CH), 0.86 d and 0.82 d (2x CH₃); **Cit:** 8.29 d (NH), 4.36 m (N-CH), 1.70 m and 1.60 m (CH₂), 1.44 m and 1.37 m (CH₂), 3.03 m and 2.945 m (N-CH₂); **PAB-2-F:** 10.26 s (NH), 7.645 dd (ArH), 7.51 t (ArH), 7.33 dd (ArH), 5.205 s (O-CH₂). **Hpx N1-H** 12.43 d (3.8). **³¹P** 57.99, 80.22. **¹⁹F** -191.70, -198.72. | | | | | | | | | |
| **3.18** | Hpx | 6.12 d (8.5) | 5.46 dddd (25.1; 12.3; 8.5; 4.1) | 5.31 dd (53.8; 4.1) | 4.63 dt (27.5; 2.3; 2.3) | 4.295 m | 3.92 m | -- | -- | 8.095 d (3.8); 8.35 s |
| | A | 6.46 d (18.9) | 5.89 dd (51.8; 3.7) | 5.12 ddd (24.9; 8.6; 3.7) | 4.34 um | 4.22 m | 3.89 m | 4.17 dd (14.0; 1.8) | 4.09 dd (14.0; 5.4) | 8.68 s; 8.48 s |
| | **Linker: Mal-Gly:** 7.08 s (CH=), 4.14 d and 4.105 d (N-CH₂); **Val:** 8.255 d (NH), 4.26 dd (N-CH), 1.97 m (CH), 0.84 d and 0.81 d (2x CH₃); Cit: 8.30 d (NH), 4.515 m (N-CH), 1.72 m and 1.60 m (CH₂), 1.45 m and 1.40 m (CH₂), 2.985 m (N-CH₂); **PAB-3-F:** 9.79 bs (NH), 7.86 t (ArH), 7.405 dd (ArH), 7.255 dd (ArH), 5.195 s (O-CH₂). **Hpx N1-H** 12.43 d (3.8). **³¹P** 57.97, 80.13. **¹⁹F** -191.68, -198.75. | | | | | | | | | |

**Table 14. ¹³C NMR data of Compounds 3.14-3.18 in DMSO-d6.**

| **Cmpd** | **Unit** | **C-1'** | **C-2'** | **C-3'** | **C-4'** | **C-5'** | **P-CH₂-O** | **C-2** | **C-4** | **C-5** | **C-6** | **C-8** |
|---|---|---|---|---|---|---|---|---|---|---|---|---|
| **3.14** | Hpx | 83.54 (3.2) | 74.06 (14.8; 8.2) | 92.55 (183.2) | 81.88 (23.2; 9.5) | 64.78 | -- | 146.66 | 149.47 | 124.16 | 156.98 | 138.65 |
| | A | 86.46 (34.2) | 92.04 (186.7) | 76.52 (16.3; 6.2) | 79.24 (11.4) | 63.28 | 68.22 (119.3) | 152.34 | 150.08 | 123.44 | 151.34 | 142.02 |
| | **Linker: Mal-Gly:** 171.12 (C=O), 135.22 (CH=), 39.90 (N-CH₂), 166.64 (C=O); **Val:** 171.02 (C=O), 58.07 (N-CH), 31.19 (CH), 19.47 and 18.40 (2x CH₃); Ala: 171.59 (C=O), 49.57 (N-CH), 18.12 (CH₃); **PAB:** 139.18 (ArC), 119.41 (2x ArCH), 129.22 (2x ArCH), 131.36 (ArC), 66.63 (O-CH₂), **SE:** 152.45 (C=O). | | | | | | | | | | | |
| **3.15** | Hpx | 83.55 | 74.05 (14.4; 8.8) | 92.11 (183.9; 3.8) | 81.57 (24.1; 10.4) | 64.45 | -- | 146.40 | 149.21 | 124.11 | 156.73 | 138.52 |
| | A | 86.25 (34.4) | 91.72 (187.1) | 76.29 (13.2; 8.8) | 78.80 (10.5) | 63.18 | 68.42 (121.9) | 152.09 | 151.16 | 123.52 | 149.94 | 141.68 |
| | **Linker: Mal-Gly:** 170.77 (C=O), 135.03 (CH=), 39.76 (N-CH₂), 166.30 (C=O); **PEG:** 38.96 (N-CH₂), 69.06, 69.94(6), 69.87(2), 69.78, 69.64 and 67.10 (12x O-CH₂), 36.09 (CH₂), 170.45 (C=O); **Val:** 171.32 (C=O), 57.62 (N-CH), 30.78 (CH), 19.36 and 18.27 (2x CH₃); **Cit:** 170.85 (C=O), 53.26 (N-CH), 29.46 (CH₂), 26.95 (CH₂), 38.79 (N-CH₂), 159.08 (C=O); **PAB:** 138.98 (ArC), 119.12 (2x ArCH), 129.01 (2x ArCH), 131.12 (ArC), 66.39 (O-CH₂), **SE:** 152.26 (C=O). | | | | | | | | | | | |
| **3.16** | Hpx | 83.53 | 74.09 | 92.13 (184.6) | 81.58 (23.4; 10.6) | 64.43 | -- | 146.11 | 149.22 | 124.11 | 156.75 | 138.51 |
| | A | 86.27 | 91.75 (186.8) | 76.33 | 78.81 (11.3) | 63.20 | 68.42 (121.6) | 152.13 | 151.19 | 123.53 | 149.86 | 141.76 |
| | **Linker: Mal-Gly:** 170.89 (C=O), 135.03 (CH=), 39.70 (N-CH₂), 166.30 (C=O), **Val:** 171.06 (C=O); 57.66 (N-CH), 31.13 (CH), 19.30 and 18.15 (2x CH₃); **Cit:** 171.21 (C=O), 53.54 (N-CH), 29.16 (CH₂), 26.97 (CH₂), 38.77 (N-CH₂), 159.11 (C=O); **PAB-2-CI:** 140.29 (ArC), 119.30 (ArCH), 117.82 (ArCH), 131.02 (ArCH). 132.87 (ArC), 128.22 (ArC), 63.80 (O-CH₂), 152.01 (C=O). | | | | | | | | | | | |
| **3.17** | Hpx | 83.56 (3.3) | 74.12 (14.8; 8.9) | 92.06 (184.8; 4.2) | 81.56 (23.8; 10.6) | 64.42 (11.2 ) | - | 146.42 | 148.21 | 124.14 | 156.74 | 138.52 |
| | A | 86.31 (33.9) | 91.72 (186.9) | 76.36 (15.3; 8.8) | 78.73 (11.5) | 63.24 | 68.45 (122.2) | 152.09 | 151.20 | 123.61 | 149.86 | 141.73 |
| | **Linker: Mal-Gly:** 170.88 (C=O), 135.02 (CH=), 39.67 (N-CH₂), 166.28 (C=O); **Val:** 171.06 (C=O), 57.65 (N-CH), 31.13 (CH), 19.29 and 18.14 (2x CH₃); **Cit:** 171.22 (C=O), 53.49 (N-CH), 29.16 (CH₂), 26.98 (CH₂), 38.71 (N-CH₂), 159.13 (C=O); **PAB-2-F:** 141.04 (ArC), 114.86 (ArCH), 105.90 (ArCH), 131.66 (ArCH). 160.68 (ArC), 117.56 (ArC), 60.56 (O-CH₂), 152.11 (C=O). | | | | | | | | | | | |
| **3.18** | Hpx | 83.55 (3.0) | 74.17 (15.6; 9.0) | 92.09 (184.4; 3.5) | 81.57 (23.4; 9.9) | 64.45 | -- | 146.42 | 149.21 | 124.12 | 156.74 | 138.49 |
| | A | 86.30 (33.9) | 91.73 (187.4) | 76.34 (15.1; 8.5) | 78.75 (11.0) | 63.24 | 68.45 (127.6) | 152.12 | 151.15 | 123.41 | 149.87 | 141.74 |
| | **Linker: Mal-Gly:** 170.88 (C=O), 135.02 (CH=), 39.69 (N-CH₂), 166.14 (C=O); **Val:** 171.07 (C=O), 57.53 (N-CH), 31.19 (CH), 19.27 and 18.10 (2x CH₃); **Cit:** 171.15 (C=O), 53.06 (N-CH), 29.23 (CH₂), 26.84 (CH₂), 38.90 (N-CH₂), 159.01 (C=O); **PAB-3-F:** 125.68 (ArC), 153.49 (ArC), 115.07 (ArCH), 123.82 (ArCH), 123.92 (ArCH), 133.92 (ArC), 65.54 (O-CH₂), 152.04 (C=O). | | | | | | | | | | | |

### Example 3.19 (Comparative compound)

### (2S)-N-((2S)-1-((4-((((6R,8R,9S,13aR,15R,16R,16aR,17R)-15-(6-amino-9H-purin-9-yl)-16,17-difluoro-3-mercapto-3,11-dioxido-8-(6-oxo-1,6-dihydro-9H-purin-9-yl)decahydro-6,9-methanofuro[3,2-m][1,3,6,9,12]pentaoxa[2,10]diphosphacyclopentadecin-11-yl)thio)methyl)phenyl)amino)-1-oxopropan-2-yl)-2-(2-(2,5-dioxo-2,5-dihydro-1H-pyrrol-1-yl)acetamido)-3-methylbutanamide (3.19)

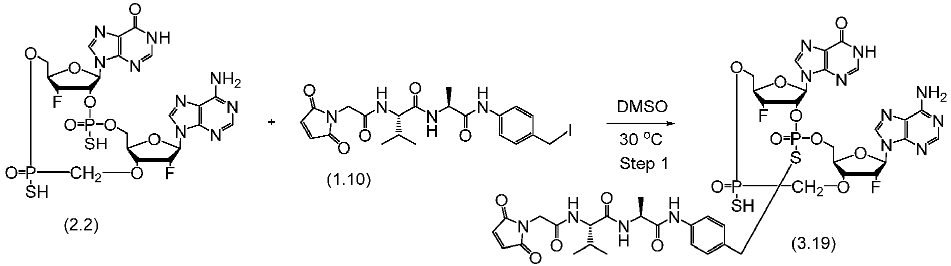

*Step 1:* Mal-Gly-Val-Ala-PAB-I (13.5 mg; 25 µmol; Compound 1.10) was added to a solution of 9-((6*R*,8*R*,9*S*,13a*R*,15*R*,16*R*,16a*R*,17*R*)-15-(6-amino-9*H*-purin-9-yl)-16,17-difluoro-3,11-dimercapto-3,11-dioxidodecahydro-6,9-methanofuro[3,2-*m*][1,3,6,9,12]pentaoxa[2,10]diphosphacyclopentadecin-8-yl)-1,9-dihydro-6*H*-purin-6-one diTEA salt (40 mg; 44 µmol; Compound 2.2) in DMSO (5 mL) and the reaction mixture was stirred for 3 h at 30 °C. The reaction mixture was directly loaded on C18 column. The product was purified by reverse phase chromatography (Method P4) as HPLC faster eluting Compound 3.19 followed by other products. The produt was lyophilized from dioxane/water (1:1) to yield 5 mg (16 %) of Compound 3.19.
UPLC-MS (Method A1): Rt = 2.30 min; m/z 1122.3 [M+H]⁺.
ESI-HRMS calculated for C₄₂H₄₆O₁₄N₁₃F₂P₂S₂ (M-H)⁻ 1120.21774, found 1120.21716.

**Table 15. ¹H, ³¹P and ¹⁹F NMR data of Compound 3.19 in DMSO-d6.**

| **Unit** | **H-1'** | **H-2'** | **H-3'** | **H-4'** | **H-5'a** | **H-5'b** | **P-CHa-O** | **P-CHb-O** | **H-2,8** |
|---|---|---|---|---|---|---|---|---|---|
| Hpx | 6.24 d (8.4) | 5.79 dddd (24.1; 10.0; 8.4; 4.0) | 5.41 dd (53.8; 4.0) | 4.72 dm (27.0) | 4.28 m | 3.945 m | -- | -- | 8.11 d (3.8); 8.50 s |
| A | 6.34 dd (19.4; 1.1) | 5.92 ddd (51.6; 4.0; 1.1) | 5.205 dm (21.8) | 4.16 m | 4.16 m | 3.96 m | 4.03 m | 4.03 m | 8.27 s; 8.33 s |
| **Linker: Mal-Gly:** 7.08 s (CH=), 4.13 s (N-CH₂); **Val**: 8.25 d (NH), 4.21 dd (N-CH), 1.955 m (CH), 0.865 d and 0.82 d (2x CH₃); **Ala:** 8.29 d (NH), 4.37 p (N-CH), 1.28 d (CH₃); **PAB:** 9.94 d (NH), 7.50 m (2x ArH), 7.23 m (2x ArH), 4.03 m (S-CH₂). **³¹P** 48.16, 28.62. **¹⁹F** -193.12, -198.27. | | | | | | | | | |

**Table 16. ¹³C NMR data of Compounds 3.19 in DMSO-d6.**

| **Unit** | **C-1'** | **C-2'** | **C-3'** | **C-4'** | **C-5'** | **P-CH₂-O** | **C-2** | **C-4** | **C-5** | **C-6** | **C-8** |
|---|---|---|---|---|---|---|---|---|---|---|---|
| Hpx | 83.31 | 75.95 (14.6; 8.0) | 91.60 (183.4) | 82.41 | 64.00 (9.4) | -- | 146.57 | 149.11 | 124.30 | 156.58 | 138.38 |
| A | 86.57 (34.6) | 92.17 (187.2) | 78.43 | 78.43 | 65.60 | 69.26 (123.1) | 151.20 | 148.63 | 119.14 | n.d. | 140.00 |
| **Linker: Mal-Gly:** 170.87 (C=O), 35.02 (CH=), 39.66 (N-CH₂), 166.72 (C=O); **Val**: 170.72 (C=O), 57.70 (N-CH), 31.08 (CH), 19.27 and 18.23 (2x CH₃); Ala: 171.24 (C=O), 49.24 (N-CH), 17.99 (CH₃); PAB: 138.74 (ArC), 119.30 (2x ArCH), 129.42 (2x ArCH), 131.51 (ArC), 34.34 (S-CH₂). | | | | | | | | | | | |

### Example 4 Activity of cyclic dinucleotides in 293T reporter assay.

### Methods

**Digitonin Assay with 293T Reporter Cells.** The generation of 293T reporter cells stably expressing different STING protein haplotypes (WT, HAQ, REF, AQ, or Q) was described previously (Novotna et al, J. Med. Chem. 2019,62, 10676). The cells were seeded at a density of 250 000 cells per cm² into 96 well white poly(D-lysine) coated plates in 100 µL of DMEM with high glucose supplemented with 10% heat inactivated FBS. Next day, the medium was removed, and compounds were added in 3-fold serial dilutions in a digitonin buffer containing 50 mM HEPES (pH 7.0), 100 mM KCl, 3 mM MgClz, 0.1 mM DTT, 85 mM sucrose, 0.2% (w/w) BSA, 1 mM ATP, 0.1 mM GTP, and 10 µg/mL digitonin A. After 30 min incubation at 37 °C with 5% CO₂, the digitonin buffer was removed, cells were washed once with 100 µL of the cultivation medium, and 100 µL of fresh medium was added to each well. The plates with cells were further incubated for 5 h at 37 °C with 5% CO₂. Thereafter, 50 µL of the medium was removed, and 30 µL of Bright-Glo Luciferase Assay System reagent was added to each well. Luminescence was measured on Spark (TECAN, Grödig, Austria), and GraphPad Prism (La Jolla, USA) was used to calculate the 50% effective concentration (EC₅₀) from an 8- point dose-response curve. Nonlinear regression curve fit with standard slope was used for EC₅₀ value calculations. The EC₅₀ value represents CDN concentration that gives half-maximal response of firefly luciferase in the 293T reporter assay.

### Results

It has been previously reported that not all cyclic dinucleotides (CDNs) exhibit equipotent activity against all STING haplotypes present in humans (Yi et al, PLoS One (2013), 8 (10), e77846CODEN). To evaluate this phenomenon, we investigated the activity of 2'3' CDNs in a 293T cell line expressing the five major STING haplotypes. In the assay we used the detergent digitonin A to permeabilize cellular membranes. This approach ensures that the uptake of negatively charged CDNs into cells is not a limiting factor in STING activation, thereby allowing us to characterize the inherent potency of cyclic dinucleotides.

2'3' cyclic dinucleotides containing both adenosine nucleobases (Compounds 2.4, 2.5, 2.6, 2.7) had potent activity against WT, HAQ and AQ STING haplotypes, but were much less active against REF haplotype. Considering the synthesis of CDN conjugates, it is beneficial to minimize the number of reactive groups on CDNs. To achieve this, we synthesized CDN 2.1, 2.2, and 2.3, utilizing hypoxanthine and adenine as nucleobases.

Notably, compounds 2.2 and 2.3 demonstrated excellent activity against all STING haplotypes, including REF haplotype which is found in 13% of humans (Yi et al; PLoS One, 2013, 8: e77846). It is advantageous for CDNs to activate all STING haplotypes because this will avoid the need for genotyping patients and allow for targeting a broader population of patients with CDN therapy. As a result of their promising performance, these compounds 2.2 and 2.3 have were chosen for further evaluation in the context of CDN conjugate synthesis (Table 17).

**Table 17.**

| | 293T STING DIGITONIN reporter assay EC₅₀ (µM) | | | | |
|---|---|---|---|---|---|
| Cmpd | WT | HAQ | REF | AQ | Q |
| 2.4 | 0.02 | 0.08 | 3 | 0.11 | 0.28 |
| 2.5 | 11.2 | >45 | >45 | 14.2 | >45 |
| 2.6 | 0.4 | 0.6 | 13.4 | 0.6 | 10.0 |
| 2.7 | 0.6 | 0.6 | 15.2 | 0.5 | 14.8 |
| 2.3 | 0.02 | 0.02 | 0.06 | 0.04 | 0.05 |
| 2.1 | 0.03 | 0.18 | 0.14 | 0.18 | 0.35 |
| 2.2 | 0.02 | 0.02 | 0.05 | 0.03 | 0.08 |

### Example 5 Cloning, expression, preparation of antibody-CDN conjugates and their characterization

### Example 5.1 Cloning of monoclonal antibodies

Sequences of Light (L) chain and Heavy (H) chains of anti-Her2 monoclonal antibody (mAb) Trastuzumab, anti-TROP2 mAb Sacituzumab, anti FOLR1 mAb Farletuzumab and isotype anti-HBsAg mAb Libivirumab were obtained from https://www.imgt.org/. Protein sequences were translated into DNA using https://www.bioinformatics.org/sms2/rev_trans.html. HindIII cloning site, Kozak's sequence, secretion signals (L chain and H chain specific) were cloned to 5' end and two stop codons (usually Ochre and Opal), XhoI cloning site at 3' end of the open reading frames for L and H chains. HindIII and XhoI restriction sites were selected for unique cloning of DNA cassettes into pTT5 vector. DNA cassette was subjected to the codon optimization for expression in HEK 293 cells except of the selected regions at 5' and 3' end (cloning sites, Kozak's sequence, stop codons). DNA sequences were synthetically prepared by ThermoFisher (Czech Republic). In some cases, cysteine (Cys) mutations were introduced engineered into heavy and/or light chains by the site directed mutagenesis.
**SEQ ID NO: 1, mAb1: Trastuzumab (anti Her2 mAb) heavy chain with engineered E152C and S375C (EU numbering)** 
GAAGTGCAGCTGGTGGAAAGCGGCGGCGGCCTGGTGCAGCCGGGCGGCAGCCTGCGCCT
GAGCTGCGCGGCGAGCGGCTTTAACATTAAAGATACCTATATTCATTGGGTGCGCCAGGC
GCCGGGCAAAGGCCTGGAATGGGTGGCGCGCATTTATCCGACCAACGGCTATACCCGCTA
 
TGCGGATAGCGTGAAAGGCCGCTTTACCATTAGCGCGGATACCAGCAAAAACACCGCGT
ATCTGCAGATGAACAGCCTGCGCGCGGAAGATACCGCGGTGTATTATTGCAGCCGCTGGG
GCGGCGATGGCTTTTATGCGATGGATTATTGGGGCCAGGGCACCCTGGTGACCGTGAGCA
GCGCGAGCACCAAAGGCCCGAGCGTGTTTCCGCTGGCGCCGAGCAGCAAAAGCACCAGC
GGCGGCACCGCGGCGCTGGGCTGCCTGGTCAAGGACTATTTTCCGTGTCCGGTGACCGTG
AGCTGGAACAGCGGCGCGCTGACCAGCGGCGTGCATACCTTTCCGGCGGTGCTGCAGAG
CAGCGGCCTGTATAGCCTGAGCAGCGTGGTGACCGTGCCGAGCAGCAGCCTGGGCACCC
AGACCTATATTTGCAACGTGAACCATAAACCGAGCAACACCAAAGTGGATAAAAAAGTG
GAACCGAAAAGCTGCGATAAAACCCATACCTGCCCGCCGTGCCCGGCGCCGGAACTGCT
GGGCGGCCCGAGCGTGTTTCTGTTTCCGCCGAAACCGAAAGATACCCTGATGATTAGCCG
CACCCCGGAAGTGACCTGCGTGGTGGTGGATGTGAGCCATGAAGATCCGGAAGTGAAAT
TTAACTGGTATGTGGATGGCGTGGAAGTGCATAACGCGAAAACCAAACCGCGCGAAGAA
CAGTATAACAGCACCTATCGCGTGGTGAGCGTGCTGACCGTGCTGCATCAGGATTGGCTG
AACGGCAAAGAATATAAATGCAAAGTGAGCAACAAAGCGCTGCCGGCGCCGATTGAAAA
AACCATTAGCAAAGCGAAAGGCCAGCCGCGCGAACCGCAGGTGTATACCCTGCCGCCGA
GCCGCGAAGAAATGACCAAAAACCAGGTGAGCCTGACCTGCCTGGTGAAAGGCTTTTAT
CCGTGCGATATTGCGGTGGAATGGGAAAGCAACGGCCAGCCGGAAAACAACTATAAAAC
CACCCCGCCGGTGCTGGATAGCGATGGCAGCTTTTTTCTGTATAGCAAACTGACCGTGGA
TAAAAGCCGCTGGCAGCAGGGCAACGTGTTTAGCTGCAGCGTGATGCATGAAGCGCTGC
ATAACCATTATACCCAGAAAAGCCTGAGCCTGAGCCCGGGCAAA
**SEQ ID NO: 2, mAb1: Trastuzumab (anti Her2 mAb) light chain** 
GCTTCCACCATGTCTGTGCCTACACAGGTGCTGGGACTGCTGCTGCTGTGGCTGACAGAT
GCCAGATGCGACATCCAGATGACACAGAGCCCTAGCAGCCTGTCTGCCAGCGTGGGAGA
CAGAGTGACCATCACCTGTAGAGCCAGCCAGGACGTGAACACAGCCGTGGCTTGGTATC
AGCAGAAGCCTGGCAAGGCCCCTAAGCTGCTGATCTACAGCGCCAGCTTTCTGTACAGCG
GCGTGCCCAGCAGATTCAGCGGCTCTAGAAGCGGCACCGACTTCACCCTGACCATAAGCA
GTCTGCAGCCCGAGGACTTCGCCACCTACTACTGTCAGCAGCACTACACCACACCTCCAA
CCTTTGGCCAGGGCACCAAGGTGGAAATCAAGCGGACAGTGGCCGCTCCTAGCGTGTTCA
TCTTTCCACCTAGCGACGAGCAGCTGAAGTCTGGCACAGCCTCTGTCGTGTGCCTGCTGA
ACAACTTCTACCCCAGAGAAGCCAAGGTGCAGTGGAAGGTGGACAACGCCCTGCAGAGC
GGCAATAGCCAAGAGAGCGTGACCGAGCAGGACAGCAAGGACTCTACCTACAGCCTGAG
CAGCACCCTGACACTGAGCAAGGCCGACTACGAGAAGCACAAAGTGTACGCCTGCGAAG
TGACCCACCAGGGCCTTTCTAGCCCTGTGACCAAGAGCTTCAACCGGGGCGAGTGCTGAT
AGCTCGAG
**SEQ ID NO: 3, mAb2: Trastuzumab (anti Her2 mAb) heavy chain** 
GAAGTGCAGCTGGTGGAAAGCGGCGGCGGCCTGGTGCAGCCGGGCGGCAGCCTGCGCCT
GAGCTGCGCGGCGAGCGGCTTTAACATTAAAGATACCTATATTCATTGGGTGCGCCAGGC
GCCGGGCAAAGGCCTGGAATGGGTGGCGCGCATTTATCCGACCAACGGCTATACCCGCTA
TGCGGATAGCGTGAAAGGCCGCTTTACCATTAGCGCGGATACCAGCAAAAACACCGCGT
ATCTGCAGATGAACAGCCTGCGCGCGGAAGATACCGCGGTGTATTATTGCAGCCGCTGGG
GCGGCGATGGCTTTTATGCGATGGATTATTGGGGCCAGGGCACCCTGGTGACCGTGAGCA
GCGCGAGCACCAAAGGCCCGAGCGTGTTTCCGCTGGCGCCGAGCAGCAAAAGCACCAGC
GGCGGCACCGCGGCGCTGGGCTGCCTGGTCAAGGACTATTTTCCGGAGCCGGTGACCGTG
AGCTGGAACAGCGGCGCGCTGACCAGCGGCGTGCATACCTTTCCGGCGGTGCTGCAGAG
CAGCGGCCTGTATAGCCTGAGCAGCGTGGTGACCGTGCCGAGCAGCAGCCTGGGCACCC
AGACCTATATTTGCAACGTGAACCATAAACCGAGCAACACCAAAGTGGATAAAAAAGTG
GAACCGAAAAGCTGCGATAAAACCCATACCTGCCCGCCGTGCCCGGCGCCGGAACTGCT
GGGCGGCCCGAGCGTGTTTCTGTTTCCGCCGAAACCGAAAGATACCCTGATGATTAGCCG
CACCCCGGAAGTGACCTGCGTGGTGGTGGATGTGAGCCATGAAGATCCGGAAGTGAAAT
TTAACTGGTATGTGGATGGCGTGGAAGTGCATAACGCGAAAACCAAACCGCGCGAAGAA
CAGTATAACAGCACCTATCGCGTGGTGAGCGTGCTGACCGTGCTGCATCAGGATTGGCTG
AACGGCAAAGAATATAAATGCAAAGTGAGCAACAAAGCGCTGCCGGCGCCGATTGAAAA
AACCATTAGCAAAGCGAAAGGCCAGCCGCGCGAACCGCAGGTGTATACCCTGCCGCCGA
GCCGCGAAGAAATGACCAAAAACCAGGTGAGCCTGACCTGCCTGGTGAAAGGCTTTTAT
CCGAGCGATATTGCGGTGGAATGGGAAAGCAACGGCCAGCCGGAAAACAACTATAAAAC
CACCCCGCCGGTGCTGGATAGCGATGGCAGCTTTTTTCTGTATAGCAAACTGACCGTGGA
TAAAAGCCGCTGGCAGCAGGGCAACGTGTTTAGCTGCAGCGTGATGCATGAAGCGCTGC
ATAACCATTATACCCAGAAAAGCCTGAGCCTGAGCCCGGGCAAA
**SEQ ID NO: 4, mAb2: Trastuzumab (anti Her2 mAb) light chain** 
GCTTCCACCATGTCTGTGCCTACACAGGTGCTGGGACTGCTGCTGCTGTGGCTGACAGAT
GCCAGATGCGACATCCAGATGACACAGAGCCCTAGCAGCCTGTCTGCCAGCGTGGGAGA
CAGAGTGACCATCACCTGTAGAGCCAGCCAGGACGTGAACACAGCCGTGGCTTGGTATC
AGCAGAAGCCTGGCAAGGCCCCTAAGCTGCTGATCTACAGCGCCAGCTTTCTGTACAGCG
GCGTGCCCAGCAGATTCAGCGGCTCTAGAAGCGGCACCGACTTCACCCTGACCATAAGCA
GTCTGCAGCCCGAGGACTTCGCCACCTACTACTGTCAGCAGCACTACACCACACCTCCAA
CCTTTGGCCAGGGCACCAAGGTGGAAATCAAGCGGACAGTGGCCGCTCCTAGCGTGTTCA
TCTTTCCACCTAGCGACGAGCAGCTGAAGTCTGGCACAGCCTCTGTCGTGTGCCTGCTGA
ACAACTTCTACCCCAGAGAAGCCAAGGTGCAGTGGAAGGTGGACAACGCCCTGCAGAGC
GGCAATAGCCAAGAGAGCGTGACCGAGCAGGACAGCAAGGACTCTACCTACAGCCTGAG
CAGCACCCTGACACTGAGCAAGGCCGACTACGAGAAGCACAAAGTGTACGCCTGCGAAG
 
TGACCCACCAGGGCCTTTCTAGCCCTGTGACCAAGAGCTTCAACCGGGGCGAGTGCTGAT
AGCTCGAG
**SEQ ID NO: 5, mAb3: Trastuzumab (anti Her2 mAb) heavy chain with engineered S375C (EU numbering)** 
GAAGTGCAGCTGGTGGAAAGCGGCGGCCTGGTGCAGCCGGGCGGCAGCCTGCGCCT
GAGCTGCGCGGCGAGCGGCTTTAACATTAAAGATACCTATATTCATTGGGTGCGCCAGGC
GCCGGGCAAAGGCCTGGAATGGGTGGCGCGCATTTATCCGACCAACGGCTATACCCGCTA
TGCGGATAGCGTGAAAGGCCGCTTTACCATTAGCGCGGATACCAGCAAAAACACCGCGT
ATCTGCAGATGAACAGCCTGCGCGCGGAAGATACCGCGGTGTATTATTGCAGCCGCTGGG
GCGGCGATGGCTTTTATGCGATGGATTATTGGGGCCAGGGCACCCTGGTGACCGTGAGCA
GCGCGAGCACCAAAGGCCCGAGCGTGTTTCCGCTGGCGCCGAGCAGCAAAAGCACCAGC
GGCGGCACCGCGGCGCTGGGCTGCCTGGTCAAGGACTATTTTCCGGAGCCGGTGACCGTG
AGCTGGAACAGCGGCGCGCTGACCAGCGGCGTGCATACCTTTCCGGCGGTGCTGCAGAG
CAGCGGCCTGTATAGCCTGAGCAGCGTGGTGACCGTGCCGAGCAGCAGCCTGGGCACCC
AGACCTATATTTGCAACGTGAACCATAAACCGAGCAACACCAAAGTGGATAAAAAAGTG
GAACCGAAAAGCTGCGATAAAACCCATACCTGCCCGCCGTGCCCGGCGCCGGAACTGCT
GGGCGGCCCGAGCGTGTTTCTGTTTCCGCCGAAACCGAAAGATACCCTGATGATTAGCCG
CACCCCGGAAGTGACCTGCGTGGTGGTGGATGTGAGCCATGAAGATCCGGAAGTGAAAT
TTAACTGGTATGTGGATGGCGTGGAAGTGCATAACGCGAAAACCAAACCGCGCGAAGAA
CAGTATAACAGCACCTATCGCGTGGTGAGCGTGCTGACCGTGCTGCATCAGGATTGGCTG
AACGGCAAAGAATATAAATGCAAAGTGAGCAACAAAGCGCTGCCGGCGCCGATTGAAAA
AACCATTAGCAAAGCGAAAGGCCAGCCGCGCGAACCGCAGGTGTATACCCTGCCGCCGA
GCCGCGAAGAAATGACCAAAAACCAGGTGAGCCTGACCTGCCTGGTGAAAGGCTTTTAT
CCGTGCGATATTGCGGTGGAATGGGAAAGCAACGGCCAGCCGGAAAACAACTATAAAAC
CACCCCGCCGGTGCTGGATAGCGATGGCAGCTTTTTTCTGTATAGCAAACTGACCGTGGA
TAAAAGCCGCTGGCAGCAGGGCAACGTGTTTAGCTGCAGCGTGATGCATGAAGCGCTGC
ATAACCATTATACCCAGAAAAGCCTGAGCCTGAGCCCGGGCAAA
**SEQ ID NO: 6, mAb3: Trastuzumab (anti Her2 mAb) light chain with engineered V110C (EU numbering)** 
AAGCTTCCACCATGTCTGTGCCTACACAGGTGCTGGGACTGCTGCTGCTGTGGCTGACAG
ATGCCAGATGCGACATCCAGATGACACAGAGCCCTAGCAGCCTGTCTGCCAGCGTGGGA
GACAGAGTGACCATCACCTGTAGAGCCAGCCAGGACGTGAACACAGCCGTGGCTTGGTA
TCAGCAGAAGCCTGGCAAGGCCCCTAAGCTGCTGATCTACAGCGCCAGCTTTCTGTACAG
CGGCGTGCCCAGCAGATTCAGCGGCTCTAGAAGCGGCACCGACTTCACCCTGACCATAAG
CAGTCTGCAGCCCGAGGACTTCGCCACCTACTACTGTCAGCAGCACTACACCACACCTCC
 
AACCTTTGGCCAGGGCACCAAGGTGGAAATCAAGCGGACATGCGCCGCTCCTAGCGTGTT
CATCTTTCCACCTAGCGACGAGCAGCTGAAGTCTGGCACAGCCTCTGTCGTGTGCCTGCT
GAACAACTTCTACCCCAGAGAAGCCAAGGTGCAGTGGAAGGTGGACAACGCCCTGCAGA
GCGGCAATAGCCAAGAGAGCGTGACCGAGCAGGACAGCAAGGACTCTACCTACAGCCTG
AGCAGCACCCTGACACTGAGCAAGGCCGACTACGAGAAGCACAAAGTGTACGCCTGCGA
AGTGACCCACCAGGGCCTTTCTAGCCCTGTGACCAAGAGCTTCAACCGGGGCGAGTGCTG
ATAGCTCGAG
**SEQ ID NO: 7, mAb4: Sacituzumab (anti TROP2 mAb) heavy chain** 
CAGGTTCAGCTGCAGCAGTCTGGCAGCGAGCTGAAGAAACCTGGCGCCTCTGTGAAGGT
GTCCTGCAAGGCCAGCGGCTACACCTTTACCAACTACGGCATGAACTGGGTCAAGCAGGC
CCCTGGACAGGGCCTGAAATGGATGGGCTGGATCAACACCTACACCGGCGAGCCCACCT
ACACAGACGACTTCAAGGGCAGATTCGCCTTCAGCCTGGACACCTCTGTGTCCACAGCCT
ACCTGCAGATCAGCAGCCTGAAGGCCGATGATACCGCCGTGTACTTCTGTGCCAGAGGCG
GCTTTGGCAGCAGCTACTGGTACTTCGATGTGTGGGGCCAGGGCAGCCTGGTCACAGTTA
GCTCTGCCTCTACAAAGGGCCCCAGCGTGTTCCCTCTGGCTCCTAGCAGCAAGTCTACAA
GCGGAGGAACAGCCGCTCTGGGCTGCCTCGTGAAGGATTACTTTCCCGAGCCTGTGACCG
TGTCCTGGAATTCTGGCGCTCTGACAAGCGGCGTGCACACCTTTCCAGCTGTGCTGCAAA
GCAGCGGCCTGTACTCTCTGAGCAGCGTCGTGACAGTGCCTAGCAGCTCTCTGGGCACCC
AGACCTACATCTGCAATGTGAACCACAAGCCTAGCAACACCAAGGTGGACAAGAGAGTG
GAACCCAAGAGCTGCGACAAGACCCACACCTGTCCTCCATGTCCTGCTCCAGAACTGCTC
GGCGGACCTTCCGTGTTTCTGTTCCCTCCAAAGCCTAAGGACACCCTGATGATCAGCAGA
ACCCCTGAAGTGACCTGCGTGGTGGTGGATGTGTCCCACGAGGATCCCGAAGTGAAGTTC
AATTGGTACGTGGACGGCGTGGAAGTGCACAACGCCAAGACCAAGCCTAGAGAGGAACA
GTACAACAGCACCTACAGAGTGGTGTCCGTGCTGACCGTGCTGCACCAGGATTGGCTGAA
CGGCAAAGAGTACAAGTGCAAGGTGTCCAACAAGGCTCTGCCCGCTCCTATCGAGAAAA
CCATCAGCAAGGCCAAGGGCCAGCCTAGGGAACCCCAGGTTTACACACTGCCTCCAAGC
CGGGAAGAGATGACCAAGAACCAGGTGTCCCTGACCTGCCTGGTCAAGGGCTTCTACCCT
TCCGATATCGCCGTGGAATGGGAGAGCAATGGCCAGCCTGAGAACAACTACAAGACAAC
CCCTCCTGTGCTGGACAGCGACGGCTCATTCTTCCTGTACAGCAAGCTGACAGTGGACAA
GTCCAGATGGCAGCAGGGCAACGTGTTCTCCTGCTCCGTGATGCACGAGGCCCTGCACAA
CCACTACACCCAGAAGTCCCTGAGCCTGTCTCCTGGCAAG
**SEQ ID NO: 8, mAb4: Sacituzumab (anti TROP2 mAb) light chain** 
GACATCCAGCTGACACAGAGCCCTAGCAGCCTGTCTGCCTCTGTGGGCGACAGAGTGTCC
ATCACATGCAAGGCCAGCCAGGACGTGTCAATTGCCGTGGCCTGGTATCAGCAGAAGCCT
GGCAAAGCCCCTAAGCTGCTGATCTACAGCGCCAGCTACAGATACACCGGCGTGCCCGAT
 
AGATTTTCCGGCTCTGGCAGCGGCACCGACTTCACCCTGACCATATCTAGCCTGCAGCCT
GAGGACTTCGCCGTGTACTACTGCCAGCAGCACTACATCACCCCTCTGACCTTTGGAGCC
GGCACCAAGGTGGAAATCAAGCGGACAGTGGCCGCTCCTAGCGTGTTCATCTTTCCACCT
AGCGACGAGCAGCTGAAGTCTGGCACAGCCTCTGTCGTGTGCCTGCTGAACAACTTCTAC
CCCAGAGAAGCCAAGGTGCAGTGGAAGGTGGACAACGCCCTGCAGAGCGGCAATAGCCA
AGAGAGCGTGACCGAGCAGGACAGCAAGGACTCTACCTACTCTCTGAGCAGCACCCTGA
CACTGAGCAAGGCCGACTACGAGAAGCACAAAGTGTACGCCTGCGAAGTGACCCACCAG
GGCCTTTCTAGCCCTGTGACCAAGAGCTTCAACCGGGGCGAGTGC
**SEQ ID NO: 9, mAb5: Sacituzumab (anti TROP2 mAb) heavy chain with engineered S375C (EU numbering)** 
CAGGTTCAGCTGCAGCAGTCTGGCAGCGAGCTGAAGAAACCTGGCGCCTCTGTGAAGGT
GTCCTGCAAGGCCAGCGGCTACACCTTTACCAACTACGGCATGAACTGGGTCAAGCAGGC
CCCTGGACAGGGCCTGAAATGGATGGGCTGGATCAACACCTACACCGGCGAGCCCACCT
ACACAGACGACTTCAAGGGCAGATTCGCCTTCAGCCTGGACACCTCTGTGTCCACAGCCT
ACCTGCAGATCAGCAGCCTGAAGGCCGATGATACCGCCGTGTACTTCTGTGCCAGAGGCG
GCTTTGGCAGCAGCTACTGGTACTTCGATGTGTGGGGCCAGGGCAGCCTGGTCACAGTTA
GCTCTGCCTCTACAAAGGGCCCCAGCGTTTTCCCACTGGCTCCTAGCAGCAAGAGCACAA
GCGGAGGAACAGCTGCTCTGGGCTGCCTGGTCAAGGACTATTTCCCCGAGCCTGTGACCG
TGTCCTGGAACTCTGGCGCTCTGACTTCTGGCGTGCACACCTTTCCAGCCGTGCTGCAAAG
CAGCGGCCTGTACTCTCTGAGCAGCGTCGTGACAGTGCCAAGCTCTAGCCTGGGCACCCA
GACCTACATCTGCAATGTGAACCACAAGCCTAGCAACACCAAGGTGGACAAGAAGGTGG
AACCCAAGAGCTGCGACAAGACCCACACCTGTCCTCCATGTCCTGCTCCAGAACTGCTCG
GCGGACCTTCCGTGTTTCTGTTCCCTCCAAAGCCTAAGGACACCCTGATGATCAGCAGAA
CCCCTGAAGTGACCTGCGTGGTGGTGGATGTGTCCCACGAGGATCCCGAAGTGAAGTTCA
ATTGGTACGTGGACGGCGTCGAGGTGCACAACGCCAAGACAAAGCCCAGAGAGGAACAG
TACAACAGCACCTACAGAGTGGTGTCCGTGCTGACAGTGCTGCACCAGGATTGGCTGAAC
GGCAAAGAGTACAAGTGCAAGGTGTCCAACAAGGCCCTGCCTGCTCCTATCGAGAAAAC
CATCAGCAAGGCCAAGGGCCAGCCTAGAGAACCCCAGGTGTACACACTGCCTCCAAGCC
GGGAAGAGATGACCAAGAACCAGGTGTCCCTGACCTGTCTCGTGAAGGGCTTCTACCCCT
GCGACATTGCCGTGGAATGGGAGAGCAATGGACAGCCCGAGAACAACTACAAGACAACC
CCTCCTGTGCTGGACAGCGACGGCTCATTCTTCCTGTACAGCAAGCTGACCGTGGACAAG
TCCAGATGGCAGCAGGGCAACGTGTTCAGCTGCAGCGTGATGCACGAGGCCCTGCACAA
CCACTACACCCAGAAGTCCCTGTCTCTGAGCCCCGGCAAG
**SEQ ID NO: 10, mAb5: Sacituzumab (anti TROP2 mAb) light chain with engineered V110C (EU numbering)** 
GACATCCAGCTGACACAGAGCCCTAGCAGCCTGTCTGCCTCTGTGGGCGACAGAGTGTCC
ATCACATGCAAGGCCAGCCAGGACGTGTCAATTGCCGTGGCCTGGTATCAGCAGAAGCCT
GGCAAAGCCCCTAAGCTGCTGATCTACAGCGCCAGCTACAGATACACCGGCGTGCCCGAT
AGATTTTCCGGCTCTGGCAGCGGCACCGACTTCACCCTGACCATATCTAGCCTGCAGCCT
GAGGACTTCGCCGTGTACTACTGCCAGCAGCACTACATCACCCCTCTGACCTTTGGAGCC
GGCACCAAGGTGGAAATCAAGCGGACATGTGCTGCCCCTAGCGTGTTCATCTTCCCACCT
TCCGACGAGCAGCTGAAGTCTGGCACAGCCTCTGTCGTGTGCCTGCTGAACAACTTCTAC
CCCAGAGAAGCCAAGGTGCAGTGGAAGGTGGACAACGCCCTGCAGAGCGGCAATAGCCA
AGAGAGCGTGACCGAGCAGGACAGCAAGGACTCTACCTACTCTCTGAGCAGCACCCTGA
CACTGAGCAAGGCCGACTACGAGAAGCACAAAGTGTACGCCTGCGAAGTGACCCACCAG
GGCCTTTCTAGCCCTGTGACCAAGAGCTTCAACCGGGGCGAGTGC
**SEQ ID NO: 11, mAb6: Farletuzumab (anti FOLR1 mAb) heavy chain with engineered E152C and S375C (EU numbering)** 
GAAGTGCAGCTGGTTGAATCTGGTGGCGGAGTGGTGCAGCCTGGCAGATCTCTGAGACTG
AGCTGTAGCGCCAGCGGCTTCACCTTTTCTGGCTACGGCCTGAGCTGGGTCCGACAGGCT
CCTGGAAAAGGCCTGGAATGGGTCGCCATGATCAGCAGCGGCGGCAGCTACACCTACTA
CGCCGATTCTGTGAAGGGCAGATTCGCCATCAGCCGGGACAACGCCAAGAATACCCTGTT
CCTGCAGATGGACAGCCTGCGGCCTGAAGATACCGGCGTGTACTTCTGTGCCAGACACGG
CGACGACCCTGCTTGGTTTGCTTATTGGGGCCAGGGCACCCCTGTGACAGTGTCTAGCGC
CTCTACAAAGGGCCCCAGCGTTTTCCCACTGGCTCCTAGCAGCAAGAGCACCTCTGGTGG
AACAGCCGCTCTGGGCTGTCTGGTCAAGGACTACTTCCCCTGTCCAGTGACCGTGTCCTG
GAATTCTGGCGCCCTGACATCTGGCGTGCACACCTTTCCAGCTGTGCTGCAGTCTAGCGG
CCTGTACTCTCTGAGCAGCGTGGTCACAGTGCCAAGCTCTAGCCTGGGCACCCAGACCTA
CATCTGCAATGTGAACCACAAGCCTAGCAACACCAAGGTGGACAAGAAGGTGGAACCCA
AGAGCTGCGACAAGACCCACACCTGTCCTCCATGTCCTGCTCCAGAACTGCTCGGCGGAC
CTTCCGTGTTTCTGTTCCCTCCAAAGCCTAAGGACACCCTGATGATCTCCAGAACACCCGA
AGTGACCTGCGTGGTGGTGGATGTGTCTCACGAGGACCCTGAAGTGAAGTTCAATTGGTA
CGTGGACGGCGTCGAGGTGCACAATGCCAAGACCAAGCCTAGAGAGGAACAGTACAACA
GCACCTACAGAGTGGTGTCCGTGCTGACCGTGCTGCACCAGGATTGGCTGAACGGCAAAG
AGTACAAGTGCAAGGTGTCCAACAAGGCCCTGCCTGCTCCTATCGAGAAAACCATCAGCA
AGGCCAAGGGCCAGCCTAGGGAACCCCAGGTTTACACACTGCCTCCAAGCCGGGAAGAG
ATGACCAAGAACCAGGTGTCCCTGACCTGCCTCGTGAAGGGCTTCTACCCCTGCGACATT
GCCGTGGAATGGGAGAGCAATGGCCAGCCTGAGAACAACTACAAGACAACCCCTCCTGT
GCTGGACTCCGACGGCTCATTCTTCCTGTACAGCAAGCTGACAGTGGACAAGTCCAGATG
GCAGCAGGGCAACGTGTTCAGCTGCAGCGTGATGCACGAGGCCCTGCACAACCACTACA
CCCAGAAGTCCCTGAGCCTGTCTCCTGGCAAG
**SEQ ID NO: 12, mAb6: Farletuzumab (anti FOLR1 mAb) light chain**

GACATCCAGCTGACACAGAGCCCTAGCAGCCTGTCTGCCTCTGTGGGCGACAGAGTGACC
ATCACCTGTAGCGTGTCCAGCAGCATCAGCAGCAACAACCTGCACTGGTATCAGCAGAAG
CCCGGCAAGGCCCCTAAGCCTTGGATCTACGGCACAAGCAATCTGGCCAGCGGCGTGCCA
AGCAGATTTTCTGGCTCTGGCAGCGGCACCGACTACACCTTCACCATATCTAGCCTGCAG
CCTGAGGATATCGCCACCTACTACTGCCAGCAGTGGTCTAGCTACCCCTACATGTACACC
TTCGGCCAGGGCACCAAGGTGGAAATCAAGAGAACAGTGGCCGCTCCTAGCGTGTTCATC
TTCCCACCTTCCGACGAGCAGCTGAAGTCTGGCACAGCCTCTGTCGTGTGCCTGCTGAAC
AACTTCTACCCCAGAGAAGCCAAGGTGCAGTGGAAGGTGGACAACGCCCTGCAGAGCGG
CAATAGCCAAGAGAGCGTGACCGAGCAGGACAGCAAGGACTCTACCTACTCTCTGAGCA
GCACCCTGACACTGAGCAAGGCCGACTACGAGAAGCACAAAGTGTACGCCTGCGAAGTG
ACCCACCAGGGCCTTTCTAGCCCTGTGACCAAGAGCTTCAACCGGGGCGAGTGC

### Example 5.2 Expression and purification of monoclonal antibodies

HEK293-E WCB cells (National Research Council Canada) were cultured to a density of 1.5×10⁶ cells/ml in 140 ml of CD293 medium supplemented with 4 mM L-Glutamine and 25 µg/ml G418. A day prior to transfection, the cell culture was centrifuged and washed with Freestyle 293 medium. The cells were resuspended in 200 ml of fresh Freestyle 293 medium at a concentration of 1×10⁶ cells/ml and transferred into a 1L spinner flask. On the day of transfection, 400 µl of Lipofectamine 2000 in 10 ml of OptiMEM medium was prepared and allowed to incubate at room temperature for 2-5 minutes. In parallel, 140 µg of the expression pTT5 plasmid encoding the light chain and 60 µg of the expression pTT5 plasmid encoding the heavy chain were diluted in 10 ml of OptiMEM medium. This mixture was combined with the Lipofectamine 2000 solution in OptiMEM medium. The resulting mixture was incubated for 5-10 minutes at room temperature and then added to the cell culture. After four hours, 200 ml of Freestyle 293 medium was added to the cells. The cells were subsequently cultured for 10-14 days while monitoring the cell number and viability. To maintain the culture, fresh Freestyle 293 medium was added multiple times. The medium volume on the day of harvest ranged from 800-1000 ml. Cells were separated by centrifugation, and EDTA-free protease inhibitors (Roche) were added to the medium. This medium was then dialyzed against PBS buffer at pH 7.4. The medium containing the monoclonal antibody (mAb) was loaded onto a HiPrep rProtein A column (5 ml, GE Healthcare) pre-equilibrated with PBS at pH 7.4. The column was washed with 10 column volumes (CV) of PBS buffer at pH 7.4, and the mAb was eluted using 0.1 M citrate buffer at pH 3.0. Following pH adjustment to a range of 7-8 using 1 M Tris at pH 8.0, the mAbs were dialyzed against PBS buffer at pH 7.4, sterile-filtered, and stored at -80°C
**SEQ ID NO: 13, mAb1: Trastuzumab (anti Her2 mAb) heavy chain with engineered E152C and S375C (EU numbering)** 
EVQLVESGGGLVQPGGSLRLSCAASGFNIKDTYIHWVRQAPGKGLEWVARIYPTNGYTRYAD
SVKGRFTISADTSKNTAYLQMNSLRAEDTAVYYCSRWGGDGFYAMDYWGQGTLVTVSSAS
TKGPSVFPLAPSSKSTSGGTAALGCLVKDYFPCPVTVSWNSGALTSGVHTFPAVLQSSGLYSL
SSVVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSREEMTKNQVSLTCLV
KGFYPCDIAVEWESNGQPENNYKTTPPVLDSDGSFFLYSKLTVDKSRWQQGNVFSCSVMHEA
LHNHYTQKSLSLSPGK
**SEQ ID NO: 14, mAb1: Trastuzumab (anti Her2 mAb) light chain** 
DIQMTQSPSSLSASVGDRVTITCRASQDVNTAVAWYQQKPGKAPKLLIYSASFLYSGVPSRFS
GSRSGTDFTLTISSLQPEDFATYYCQQHYTTPPTFGQGTKVEIKRTVAAPSVFIFPPSDEQLKSG
TASVVCLLNNFYPREAKVQWKVDNALQSGNSQESVTEQDSKDSTYSLSSTLTLSKADYEKHK
VYACEVTHQGLSSPVTKSFNRGEC
**SEQ ID NO: 15, mAb2: Trastuzumab (anti Her2 mAb) heavy chain** 
EVQLVESGGGLVQPGGSLRLSCAASGFNIKDTYIHWVRQAPGKGLEWVARIYPTNGYTRYAD
SVKGRFTISADTSKNTAYLQMNSLRAEDTAVYYCSRWGGDGFYAMDYWGQGTLVTVSSAS
TKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSREEMTKNQVSLTCLV
KGFYPSDIAVEWESNGQPENNYKTTPPVLDSDGSFFLYSKLTVDKSRWQQGNVFSCSVMHEA
LHNHYTQKSLSLSPGK
**SEQ ID NO: 16, mAb2: Trastuzumab (anti Her2 mAb) light chain** 
DIQMTQSPSSLSASVGDRVTITCRASQDVNTAVAWYQQKPGKAPKLLIYSASFLYSGVPSRFS
GSRSGTDFTLTISSLQPEDFATYYCQQHYTTPPTFGQGTKVEIKRTVAAPSVFIFPPSDEQLKSG
TASVVCLLNNFYPREAKVQWKVDNALQSGNSQESVTEQDSKDSTYSLSSTLTLSKADYEKHK
VYACEVTHQGLSSPVTKSFNRGEC
**SEQ ID NO: 17, mAb3: Trastuzumab (anti Her2 mAb) heavy chain with engineered S375C (EU numbering)** 
EVQLVESGGGLVQPGGSLRLSCAASGFNIKDTYIHWVRQAPGKGLEWVARIYPTNGYTRYAD
SVKGRFTISADTSKNTAYLQMNSLRAEDTAVYYCSRWGGDGFYAMDYWGQGTLVTVSSAS
 
TKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSREEMTKNQVSLTCLV
KGFYPCDIAVEWESNGQPENNYKTTPPVLDSDGSFFLYSKLTVDKSRWQQGNVFSCSVMHE
ALHNHYTQKSLSLSPGK
**SEQ ID NO: 18, mAb3: Trastuzumab (anti Her2 mAb) light chain with engineered V110C (EU numbering)** 
DIQMTQSPSSLSASVGDRVTITCRASQDVNTAVAWYQQKPGKAPKLLIYSASFLYSGVPSRFS
GSRSGTDFTLTISSLQPEDFATYYCQQHYTTPPTFGQGTKVEIKRTCAAPSVFIFPPSDEQLKSG
TASVVCLLNNFYPREAKVQWKVDNALQSGNSQESVTEQDSKDSTYSLSSTLTLSKADYEKHK
VYACEVTHQGLSXPVTKSFNRGEC
**SEQ ID NO: 19, mAb4: Sacituzumab (anti TROP2 mAb) heavy chain** 
QVQLQQSGSELKKPGASVKVSCKASGYTFTNYGMNWVKQAPGQGLKWMGWINTYTGEPT
YTDDFKGRFAFSLDTSVSTAYLQISSLKADDTAVYFCARGGFGSSYWYFDVWGQGSLVTVSS
ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVFLFP
PKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVMH
EALHNHYTQKSLSLSPGK
**SEQ ID NO: 20, mAb4: Sacituzumab (anti TROP2 mAb) light chain** 
DIQLTQSPSSLSASVGDRVSITCKASQDVSIAVAWYQQKPGKAPKLLIYSASYRYTGVPDRFS
GSGSGTDFTLTISSLQPEDFAVYYCQQHYITPLTFGAGTKVEIKRTVAAPSVFIFPPSDEQLKSG
TASVVCLLNNFYPREAKVQWKVDNALQSGNSQESVTEQDSKDSTYSLSSTLTLSKADYEKHK
VYACEVTHQGLSSPVTKSFNRGEC
**SEQ ID NO: 21, mAb5: Sacituzumab (anti TROP2 mAb) heavy chain with engineered S375C (EU numbering)** 
QVQLQQSGSELKKPGASVKVSCKASGYTFTNYGMNWVKQAPGQGLKWMGWINTYTGEPT
YTDDFKGRFAFSLDTSVSTAYLQISSLKADDTAVYFCARGGFGSSYWYFDVWGQGSLVTVSS
ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSV
 
LTVLHQDWLNGKEYKCKVSNKALPAPIEKTISKAKGQPREPQVYTLPPSREEMTKNQVSLTC
LVKGFYPCDIAVEWESNGQPENNYKTTPPVLDSDGSFFLYSKLTVDKSRWQQGNVFSCSVMH
EALHNHYTQKSLSLSPGK
**SEQ ID NO: 22, mAb5: Sacituzumab (anti TROP2 mAb) light chain with engineered V110C (EU numbering)** 
DIQLTQSPSSLSASVGDRVSITCKASQDVSIAVAWYQQKPGKAPKLLIYSASYRYTGVPDRFS
GSGSGTDFTLTISSLQPEDFAVYYCQQHYITPLTFGAGTKVEIKRTCAAPSVFIFPPSDEQLKSG
TASVVCLLNNFYPREAKVQWKVDNALQSGNSQESVTEQDSKDSTYSLSSTLTLSKADYEKHK
VYACEVTHQGLSSPVTKSFNRGEC
**SEQ ID NO: 23, mAb6: Farletuzumab (anti FOLR1 mAb) heavy chain with engineered E152C and S375C (EU numbering)** 
EVQLVESGGGVVQPGRSLRLSCSASGFTFSGYGLSWVRQAPGKGLEWVAMISSGGSYTYYA
DSVKGRFAISRDNAKNTLFLQMDSLRPEDTGVYFCARHGDDPAWFAYWGQGTPVTVSSAST
KGPSVFPLAPSSKSTSGGTAALGCLVKDYFPCPVTVSWNSGALTSGVHTFPAVLQSSGLYSLS
SVVTVPSSSLGTQTYICNVNHKPSNTKVDKKVEPKSCDKTHTCPPCPAPELLGGPSVFLFPPKP
KDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSREEMTKNQVSLTCLVK
GFYPCDIAVEWESNGQPENNYKTTPPVLDSDGSFFLYSKLTVDKSRWQQGNVFSCSVMHEAL
HNHYTQKSLSLSPGK
**SEQ ID NO: 24, mAb6: Farletuzumab (anti FOLR1 mAb) light chain** 
DIQLTQSPSSLSASVGDRVTITCSVSSSISSNNLHWYQQKPGKAPKPWIYGTSNLASGVPSRFS
GSGSGTDYTFTISSLQPEDIATYYCQQWSSYPYMYTFGQGTKVEIKRTVAAPSVFIFPPSDEQL
KSGTASVVCLLNNFYPREAKVQWKVDNALQSGNSQESVTEQDSKDSTYSLSSTLTLSKADYE
KHKVYACEVTHQGLSSPVTKSFNRGEC
**SEQ ID NO: 25, isotype mAb: Libivirumab (anti HBsAg mAb) - heavy chain with engineered E152C and S375C (EU numbering)** 
QVQLVESGGGVVRPGRSLRLSCAASGFAFSDYSINWVRQAPGKGLEWVAIISYDGRITYYRDS
VKGRFTISRDDSKNTLYLQMNSLRTEDTAVYYCARQYYDFWSGSSVGRNYDGMDVWGLGT
TVTVSSASTKGPSVFPLAPSSKSTSGGTAALGCLVKDYFPCPVTVSWNSGALTSGVHTFPAVL
QSSGLYSLSSVVTVPSSSLGTQTYICNVNHKPSNTKVDKKVEPKSCDKTHTCPPCPAPELLGGP
SVFLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMTKNQ
 
VSLTCLVKGFYPCDIAVEWESNGQPENNYKTTPPVLDSDGSFFLYSKLTVDKSRWQQGNVFS
CSVMHEALHNHYTQKSLSLSPG
**SEQ ID NO: 26, isotype mAb: Libivirumab (anti HBsAg mAb) - light chain**

DIVMTQSPLSLSVTPGEPASISCRSSQSLLHRSGNNYLDWYLQKPGHSPQLLIYVGSNRASGVP
DRFSGSGSGTEYTLKISRVEAEDVGVYYCMQALQTPRTFGQGTKLEIKRTVAAPSVFIFPPSDE
QLKSGTASVVCLLNNFYPREAKVQWKVDNALQSGNSQESVTEQDSKDSTYSLSSTLTLSKAD
YEKHKVYACEVTHQGLSSPVTKSFNRGEC

### Example 5.3 Preparation of antibody CDN conjugates involving anti-Her2 (mAb1, mAb3), anti-TROP2 (mAbS), anti-FOLR1 mAb (mAb6) and anti-HBsAg mAb (isotype mAb) with engineered Cysteine (Cys) mutations

Because engineered Cys residues in antibodies expressed in mammalian cells are modified by adducts (disulfides) such as glutathione (GSH) and/or cysteine during biosynthesis, the modified Cys as initially expressed are unreactive to thiol reactive reagents with maleimido group. To conjugate engineered Cys residues, glutathione or cysteine adducts need to be removed by reducing disulfides, which generally entails reducing all disulfides in the antibody. Monoclonal antibody (7-10 mg/ml) in PBS buffer containing 2mM EDTA were reduced overnight with 150-fold molar excess of DTT. Next day, the antibody was diluted with 15 volumes of 20 mM succinate buffer pH 5 and DTT was removed on cation exchanger GE HiTrap SP HP column (5mL) equilibrated in 20 mM succinate buffer pH 5. The column was washed with 10 column volumes of equilibration buffer and antibody was eluted with 50 mM Tris pH 7.5 containing 0.15 M NaCl. Antibody was re-oxidized by adding 15 equivalents of dehydroascorbic acid (DHAA) with re-oxidation being monitored by LC on Agilent, PLRP-S 1000A 8µM 150x2.1mm column. The antibody was loaded on the size exclusion GE HiLoad 26/60 Superdex 200 pg column equilibrated in PBS with 2 mM EDTA buffer to remove DHAA. Reduced-oxidized mAb were next incubated with 8 molar protein equivalents of a CDN conjugate for 3 hours at room temperature. The conjugation was quenched by adding 8 equivalents of Cys and ADC was further purified using SEC Superdex 200 Increase 10/300 GL equilibrated in PBS buffer. Antibody CDN conjugates were profiled by MS and analytical SEC as described below. Drug antibody ratio (DAR) ranged between 3.5 to 3.9.

### Example 5.4 Generation of anti-HER2 and anti-TROP2 antibody CDN conjugates through partial reduction of native disulfide bonds of non-engineered anti-HER2 (mAb2) and anti-TROP2 (mAb4) monoclonal antibodies

The compounds of the invention can also be conjugated to native cysteine residues of non-engineered antibodies using a procedure that involves partial reduction of the antibodies (Doronina, S. O. et al,, Nat. Biotechnol. 21, 778-784, 2003). Interchain disulfides bonds of anti-HER2 mAb2 and anti-TROP2 mAb4 (at a concentration of 7-10 mg/ml) were first partially reduced in PBS pH 7.4 containing 2 mM EDTA by adding 2-8 equivalents of TCEP and incubating the mixture at 37°C for 2 hours. Next, the antibody reacted for two hours at RT with 8 equivalents of a CDN-conjugate. The conjugation was quenched with 8 equivalents of Cys and ADC was purified using SEC Superdex 200 Increase 10/300 GL equilibrated in PBS buffer. Antibody CDN conjugates were profiled by MS and analytical SEC as described below. Drug antibody ratios for different ADC are shown in Tables 18 and 19.

### Example 5.5 Characterization of the anti-HER2, anti-TROP2, anti-FOLR1 and anti-HBsAg antibody CDN-conjugates

Antibody CDN-conjugates were analyzed to determine the extent of conjugation. A compound-to-antibody ratio was extrapolated from LC-MS data for reduced samples. LC-MS allows quantitation of the average number of molecules of linker-payload attached to an antibody in a conjugate sample. Mass spectral data enables identification of the component species in the mixture, e.g., LC, LC+1, LC+2, HC, HC+1, HC+2, etc. From the average loading on the LC and HC chains, the average compound to antibody ratio can be calculated for an antibody conjugate. A compound-to-antibody ratio for a given conjugate sample represents the average number of compound (linker-payload) molecules attached to a tetrameric antibody containing two light chains and two heavy chains. The samples were reduced with 40 mM TCEP or 5.5 mM DTT, injected onto a MassPREP Micro Desalting column (20 µm, 5-mm by 2.1-mm ID, Waters) equilibrated in 0.1% formic acid (FA) in H₂O and eluted by a fast gradient (4min) to 0.1% FA in acetonitrile. The separation was carried out by AQUITY UPLC I-Class system (Waters) and was on-line coupled to Mass Spectrometer Synapt G2 (Waters) to acquire mass spectra using electrospray ionization in positive mode. The TOF mass range was set from m/z 500 to 4000. The raw spectrum was subtracted and deconvoluted (MaxEnt 1, Waters) to produce the final spectrum. Conjugates were profiled using analytical size-exclusion chromatography on Superdex 200 equilibrated in PBS pH7.4; aggregation was analyzed based on analytical size exclusion chromatography.

### Example 6 In vivo testing of anti-HER2 mAb1 conjugated with compounds 3.4 or 3.11 in Her2 expressing HCC1954 breast tumor xenograft model

### Methods

Compounds 3.4 and 3.11 were attached to engineered C152 and C375 on the heavy chain of anti-Her2 mAb1. For *in vivo* testing in the Her2 expressing HCC1954 breast cancer xenograft mouse model, female SCID-beige mice at 8 to 12 weeks of age (purchased from Charles River Lab) were used for implantation. HCC1954 cells (obtained from ATCC) were cultured in RPMI medium supplemented with 10% fetal bovine serum at 37°C in a humidified incubator with 5% CO₂ On the day of implantation, 1 × 10⁷ HCC1954 cells were re-suspended in 100µL RPMI1640 serum free media containing 50% Matrigel and implanted subcutaneously in the right flank. After implant, tumors were measured by caliper and mice were weighed two times per week once tumors were palpable. Tumor volumes were calculated from caliper measurements using formula (L × W²)/2. Animals were pair matched when tumor reached volume 100 - 150 mm³ (designated as day 1). Two i.v. injections were performed on day 1 and 8 (D1 and D8) of 5mg/kg of anti-Her2 mAbl-3.4 (DAR 3.9), 5mg/kg mAbl-3.11 (DAR 3.9), 5mg/kg isotype mAb-3.4 (DAR 3.9), 5mg/kg anti-Her2 mAb1 or PBS vehicle.

### Results

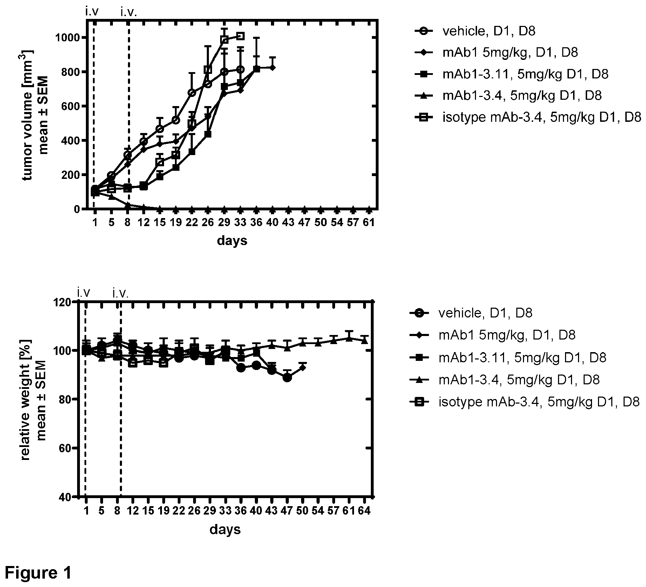

HCC1954 breast tumor xenograft mice were treated intravenously with two doses (on day one and eight) of anti-HER2 mAbl-3.4 and mAb1-3.11 conjugate, unconjugated mAb1, and isotype mAb-3.4 (6 mice per group). Treatment with anti-HER2 mAbl-3.4 led to complete regression of human HCC1954 xenograft tumors (Fig. 1). Tumor regression was not observed in mice treated with isotype control mAb-3.4 or un-conjugated mAb1 (Fig. 1). Surprisingly, anti-HER2 mAb1-3.11 exhibited only a partial tumor growth inhibition even though the payload CDN 2.3 present in compound 3.11 had similar potency in STING 293 digitonin reporter assay as payload CDN 22 present in compound 3.4 (Table 17). The treatments were well tolerated by all animals.

These data demonstrate unexpected advantage of CDN payload 2.2 over 2.3 and its ability to induce tumor regression in HER2-expressing HCC 1954 breast tumor xenografts when conjugated to anti-HER2 mAb1 using compound 3.4. Notably, the isotype control mAb-3.4 showed no activity, underscoring the specificity of the treatment.

### Example 7 Effect of intratumoral injection of the compound 2.2 on tumor growth, induction of tumor specific CD8 T cells and immunological memory

The CT26 mouse colon carcinoma cell line was obtained from American Type Culture Collection (ATCC^{®}, CRL-2638). The cells were cultured in RPMI-1640 medium containing 10% fetal bovine serum, 2 mM glutamine, 100 units/mL penicillin G sodium, and 100 µg/mL streptomycin sulfate at 37 °C in a humidified incubator with 5% CO₂. On the day of tumor implantation in ten-week-old female BALB/c mice (Charles River Laboratories), CT26 cells harvested during the exponential growth phase were resuspended in phosphate-buffered saline (PBS) at a concentration of 1 × 10⁷ cells/mL. Tumors were initiated by subcutaneous implantation of 1 × 10⁶ CT26 cells (in a 0.1 mL suspension) into the right flank of each test animal. Tumor dimensions were measured in two directions, and tumor volumes were calculated using the formula (L × W²)/2. Animals were pair matched when tumor reached volume 100 - 150 mm³ (designated as day 1) and injected intratumorally (i.t.) on day 1, 4 and 8 with 5mg/kg or 1.5mg/kg Compound 2.2 or PBS in total volume 20 µL.

The induction of tumor specific AH1+ CD8 T cells was monitored in response to Compound 2.2 treatment. Blood immune cells were collected on day 14 and red blood cells were lysed using red blood cell lysis buffer. Immune cells were immunophenotyped using viability dye (Zombie NIR Fixable Viability Kit, BioLegend) and monoclonal antibodies: BUV395 rat anti-mouse CD19, BUV395 hamster anti-mouse CD49b, APC hamster anti-mouse CD3, BV605 rat anti-mouse CD4, BV510 rat anti-mouse CD8, (all BD Biosciences). Specific staining using MHC-I dextramer: PE labelled AH1 peptide (SPSYVYHQF, Immudex) was performed according to the manufacturer's recommendation. Specific and control samples of unstained cells, positive control for dead cells (cells in PBS incubated for 10 min at 65 °C and further stained with a live/dead marker), fluorescence minus one (FMO) and isotype controls (ISO) were prepared. Samples were measured immediately without a fixation step in a single tube format using BD LSRFortessa flow cytometer (BD Biosciences) in 250 µl of FC buffer (PBS containing 5% FBS). Data was acquired using FACS Diva software (version 8.0.1, BD Biosciences). Debris was excluded by forward and side scatter gating followed by doublet and dead cell exclusion. The population of interest was gated as AH1+ CD8+ T cells, specifically CD19- CD49b- CD3+ CD4-CD8+ AH1+ cells. The data were analyzed using FlowJo software (version 10, BD Biosciences) and expressed as a frequency (%) of parent population, i.e. CD3+ T cells.

Induction of antitumoral immunologic memory was monitored using ten animals from above mentioned 1.5mg/kg CT26 efficacy study which were tumor free on day 65. The animals were split equally into two groups (five animals per group) and treated intraperitoneally with 0.4 mL (250 µg/mL in PBS) of isotype mAb (BXC-BP0090, BioXCell) or anti CD8 T cell mAb (BXC-BP0061, BioXCell) four and three days before and three days after 1 × 10⁶ CT26 cells (in a 0.1 mL suspension) were implanted into the left flank of animals (day when cell were implanted is designated as day 1). Efficiency of CD8 T cell depletion was monitored by flow-cytometry on day 3 post first i.p. of mAbs. In parallel, naive Balb/c mice were implanted with the same number of cells into the left flank. Tumors were measured in two dimensions and tumor volumes were calculated from caliper measurements using formula (L × W₂)/2 to monitor growth.

### Results

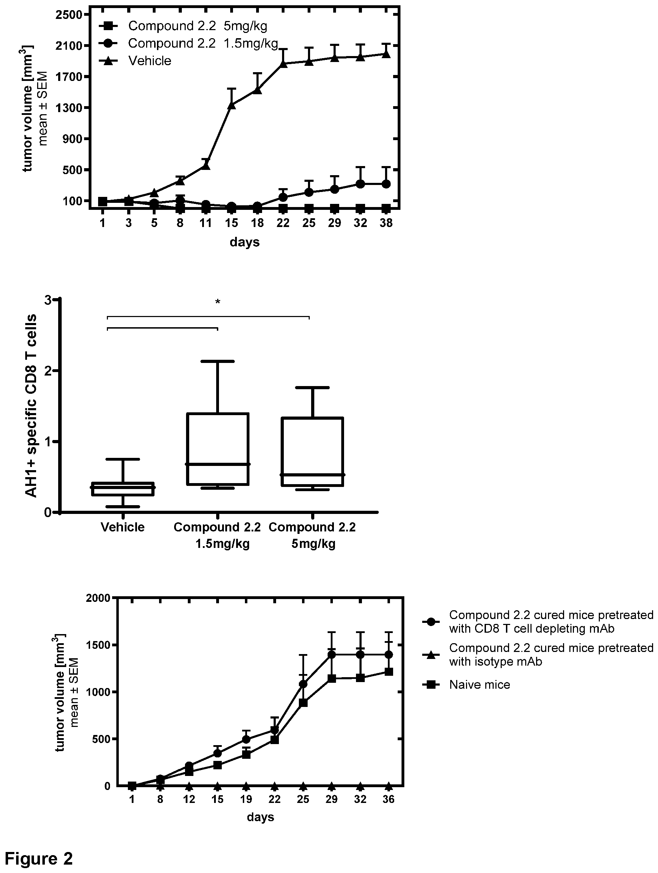

Twelve and ten out twelve tumor bearing animals treated intratumorally with 5mg/kg or 1.5mg/kg Compound 2.2 were tumor free on day 65 post first application of the compound (Fig 2). This contrasted with all animals treated with vehicle succumbing to the disease by day 40. Animals treated with Compound 2.2 also had statistically significantly increased frequency of tumor specific CD8 T cell recognizing AH1 peptide presented on MHC-1 compared to the vehicle arm (Fig 2). Finally, when the animals previously cured in the efficacy study were rechallenged with CT26 cells, they rejected the implants if they were pretreated with the isotype mAb. Conversely, the kinetics of CT26 tumor growth in the cured animals in which CD8 T-cells were depleted were comparable to that of naive animals (Fig. 2). This data indicated that animals cured with Compound 2.2 developed CD8 T cell dependent adaptive antitumoral immunity.

All together the presented data show that Compound 2.2 can induce robust antitumoral effect and antitumoral adaptive immunity in vivo when applied i.t.

### Example 8 In vitro and in vivo stability of antibody-CDN conjugates

*In vitro* plasma stability of ADC was performed by spiking mouse plasma with antibody-CDN conjugates (final concentration 100µg/mL). Samples were incubated in a 37°C incubator with 5% CO₂, aliquots were taken at various time points and stored frozen for analysis. Pharmacokinetics and pharmacodynamics of the antibody-CDN conjugates were studied following i.v. injection of 1 to 5 mg/kg ADCs into Balb/c, SCID Beige or CD1 mice. Serum samples were taken at various time points and stored frozen for analysis. Plasma levels of cytokines were determined using ProcartaPlex Immunoassays following manufacture's protocol and measured on MagPix instrument.

The total level of the human antibodies or antibody conjugates in plasma samples was measured by immunoassays. The capture was performed with F(ab')2-Goat anti-Human IgG Fc (Invitrogen cat # A24478) and detection with goat anti Human IgG F(ab) FITC (Invitrogen cat # 31628).

Payload CDN 22 retention on mAbs was studied by LC-MS. Briefly, 25µL of plasma samples were incubated with biotinylated anti-human Fcy fragment mAb (final concetration 0.18mg/mL, Jackson ImmunoResearch cat # 109-065-098) for 30 min and then mixed with 30 µL Dynabead MyOne Streptavidin T1 (Invitrogen cat # 65604D) and 70 µL PBS. After 1 hour incubation, the beads were separated on a magnet, washed with PBS containing 0.05% Tween20, PBS and water, and ADCs were eluted in the presence of 0.2% of formic acid in water. Eluate was neutralized by adding ammonium formate (400mM final concentration) and ADCs were reduced with TCEP (40mM final concentration) for 30 minutes at room temperature. Samples were then measured by LC-MS and analyzed for compound-to-antibody ratio as described above.

Pharmacokinetics of Compound 2.2 applied I.V. at dose 5mg/kg was determined in Balb/c mice. Plasma samples were precipitated with methanol (final concetration 70%) spiked with internal standard, the precipited proteins were removed by centrigugation and supernatant was evaporated. The analytes were dissolved in 100 µL of 10 mM CH₃COONH₄ by vortexing for 5 min. Compound 22 along with the corresponding internal standard were separated and detected using a liquid chromatograph Exion LC AD with tandem mass spectrometer QTrap 6500+ (both Sciex) equipped with a separation column Acquity BEH C18 1.7 µm, 50 × 2.1 mm, as well as a guard column 1.7 µm C18, 5 × 2.1 mm (both Waters). The mobile phase (A) used for separation consisted of 10 mM CH₃COONH₄ in deionized water, while mobile phase (B) was composed of 10 mM CH₃COONH₄ in methanol. The separation was carried out by using the following gradient: 0 - 2 min of 5% B, 2 - 10 min of 90% B, 10 - 15 min of 90% B, 15 - 15.1 min of 5% B, and 15.1 - 20 min of 5% B with a flow rate of 0.2 mL/min. Analytes were monitored through their specific multiple reaction monitoring (MRM) transitions by utilizing electrospray ionization (ESI) in a positive mode with the following parameters: 50 msec dwell time, entrance potential 10 V, ion source temperature 350°C, ion spray voltage 5.5 kV, curtain gas (40 psi), GS1 gas (50 psi), and GS2 gas (50 psi). Quantification of Compound 2.2 was conducted using a weighted calibration curve (1/x²) with a structurally similar internal standard.

### Results

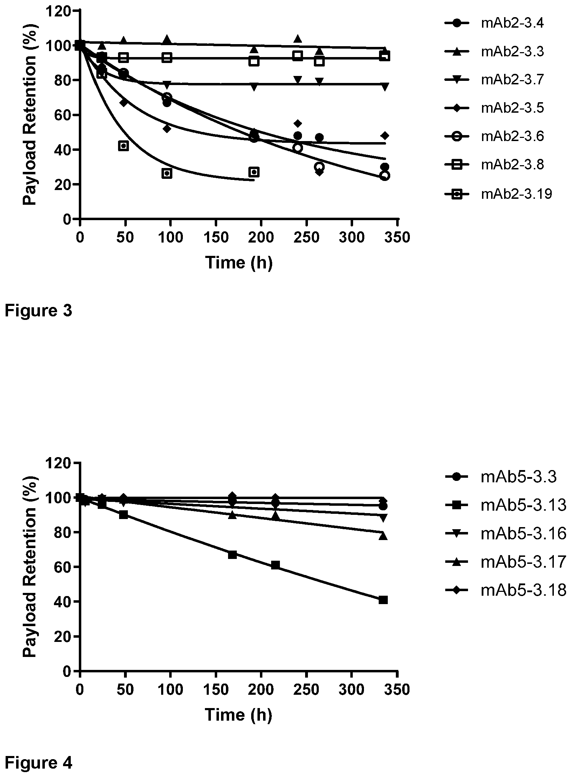

Various antibody CDN conjugates were prepared by attaching compounds to anti Her2 mAb2 via interchain cysteines as described above and their retention on mAb2 was assessed in mouse plasma *in vitro.* Anti Her2 mAb2-3.4, mAb2-3.6, mAb2-3.5 had moderate retention of the CDN warhead compound 22 (T_{1/2} 7-8 days). Importantly, compound 3.19, in which the linker is attached to the warhead CDN 2.2 via sulfur, was quite unstable (T_{1/2} 4 days) when attached to mAb2. On the contrary, attachment of compounds 3.3, 3.7, and 3.8 to anti Her2 mAb2 was stable over period up to 15 days (Fig. 3, Table 18).

In conclusion, it is disadvantegous to perfom attachment of the warhead CDN 2.2 and the linker via thiophosphate group.

**Table 18**

| Compound | Monoclonal antibody | Drug Antibody ratio (DAR) | HCC1954 IP10 EC₅₀ (µM) | Her2+ 293T EC₅₀ (µM) | Mouse plasma T _{½} (days) |
|---|---|---|---|---|---|
| 3.4 | anti Her2 mA2 | 4.5 | 0.008 | 0.019 | 7 |
| 3.5 | anti Her2 mA2 | 4.9 | 0.018 | 0.02 | 8 |
| 3.6 | anti Her2 mA2 | 5.6 | 0.05 | 0.02 | 7 |
| 3.3 | anti Her2 mA2 | 3.7 | 0.009 | 0.013 | >15 |
| 3.7 | anti Her2 mA2 | 4 | 0.007 | 0.014 | >15 |
| 3.8 | anti Her2 mA2 | 4 | 0.012 | 0.018 | >15 |
| 3.19 | anti Her2 mA2 | 2.5 | 0.002 | 0.002 | 4 |
| None | anti Her2 mA2 | - | inactive | inactive | - |
| 3.4 | isotype mAb | 4.0 | >1 | >1 | - |
| 3.3 | isotype mAb | 4.0 | >1 | >1 | - |

Compounds 3.3, 3.13, 3.16, 3.17, and 3.18 were also attached to engineered light chain V110C and heavy chain S375C cysteines of anti TROP2 targeted mAb5. As shown in Table 19 and Figure 4, with exemption of compound 3.13 all compounds had an excellent retention on anti-TROP2 mAb5. Unexpectedly, substitution of para-amino benzyl group of compound 3.13 with 2-F, 2-Cl, and 3-F groups resulted in improved plasma stability of antibody-CDN conjugates (compounds 3.16, 3.17, and 3.18).

**Table 19**

| Compound | Monoclonal antibody | Drug Antibody Ratio | HCC1954 IP10 EC₅₀ (µM) | TROP2+ 293T EC₅₀ (µM) | Mouse plasma T _{½} (days) |
|---|---|---|---|---|---|
| 3.3 | anti TROP2 mA5 | 3.7 | 0.008 | 0.008 | >15 |
| 3.13 | anti TROP2 mA5 | 3.3 | 0.002 | 0.003 | 9.6 |
| 3.16 | anti TROP2 mA5 | 3.3 | 0.005 | 0.004 | >15 |
| 3.17 | anti TROP2 mA5 | 3.6 | 0.008 | 0.003 | >15 |
| 3.18 | anti TROP2 mA5 | 3.6 | 0.006 | 0.002 | >15 |
| None | anti TROP2 mA5 | - | inactive | inactive | - |
| 3.3 | isotype mAb | 3.8 | >1 | >1 | - |
| 3.13 | isotype mAb | 3.8 | >1 | >1 | - |
| 2.2 | Warhead CDN | - | 1.7 | 0.7 | - |

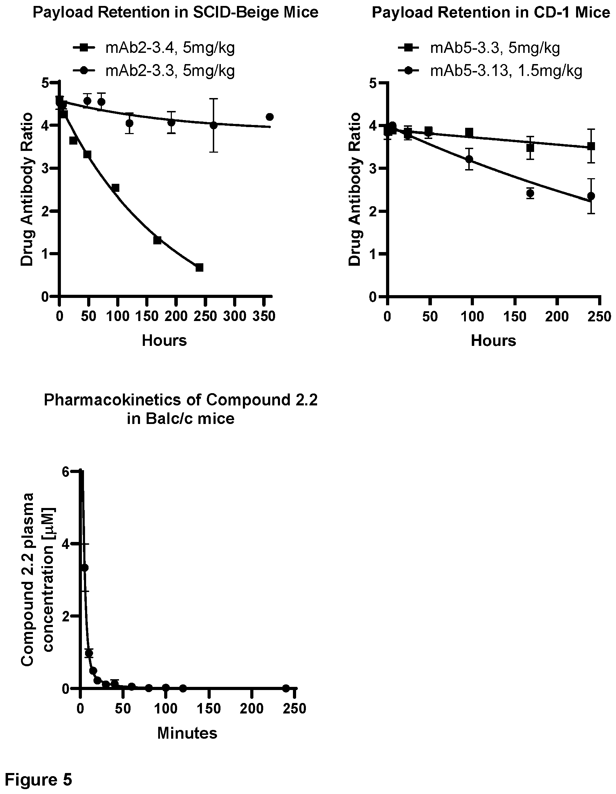

In vivo studies on payload CDN retention (Compound 2.2) were conducted in SCID Beige mice for anti-HER2 mAb2-3.4 (DAR 4.6) and anti-HER2 mAb2-3.3 (DAR 4.5) and in CD-1 nude mice for anti-TROP2 mAb5-3.3 (DAR 3.8) and anti-TROP2 mAb5-3.13 (DAR 3.8). These animals received a single intravenous bolus injection of antibody-CDN conjugates at doses of 1.5 mg/kg (mAb5-3.13) and 5 mg/kg (mAb2-3.4, mAb2-3.3, mAb5-3.3), respectively. Blood samples were collected from the treated animals at various time points, and plasma was stored frozen for subsequent analysis. Warhead CDN 2.2 retention on anti-HER2 mAb2 and anti-TROP2 mAb5 was quantified using immunoprecipitation and mass spectrometry analysis, as described above. The half-life for the warhead retention with compound 3.3 attached to both monoclonal antibodies exceeded 10 days. In line with impaired stability observed in mouse plasma in vitro (Table 18 and 19), compound 3.4 attached to anti-HER2 mAb2 had a half-life of approximately 3.7 days, while compound 3.13 conjugated to anti-TROP2 mAb5 exhibited a half-life of approximately 12 days (Fig. 5). Lastly, we evaluated the pharmacokinetics of Compound 2.2 in Balb/c mice treated with 5mg/kg i.v (Fig 5). Compound 2.2 exhibited efficient clearance with a half-life of less than 10 minutes, highlighting the advantage of conjugating Compound 2.2 to a monoclonal antibody via a linker for improved pharmacokinetics.

### Example 9 Activation of the STING pathway in HCC1954 and 293T STING reporter cells with antibody-CDN conjugates

HCC1954 and 293T reporter cells were cultured as previously described. HCC1954 cells, which naturally express both Her2 and TROP2, were seeded into collagen-coated 96-well plates at a density of 0.5×10⁶ cells/ml (100 µl/well) in RPMI medium supplemented with 10% FBS and Penicillin/Streptomycin. The following day, the medium was completely removed, and serial dilutions of mAb2-CDN and mAb5-CDN conjugates in medium were added to the wells. Levels of the STING-dependent cytokine IP10 (CXCL10) induction were determined in the medium using ProcartaPlex Immunoassays after 24 hours of incubation following the manufacturer's protocol and measured on a MagPix instrument. GraphPad Prism (La Jolla, USA) was utilized to calculate the 50% effective concentration (EC₅₀) from a 6-point dose-response curve. Nonlinear regression curve fitting was applied for EC₅₀ value calculations. The EC₅₀ value represents the concentration of the payload CDN 2.2 conjugated to anti-Her2 mAb2 or anti-TROP2 mAb5 that results in half-maximal levels of IP-10. Twenty million 293T WT STING reporter cells were seeded into two T150 flasks, each containing 30 mL of DMEM with high glucose supplemented with 10% heat-inactivated FBS. One flask was designated for transfected cells, and the other served as a non-transfected control. After 24 hours, the cell culture medium was replaced with 27 mL of fresh media. For the transfection process, a transfection mixture was prepared for each T150 flask. In one tube, 1500 µL of OptiMedium was combined with 40 µg of Her2 or TROP2 expression plasmid and 45 µL of Reagent P3000. In another tube, 1500 µL of OptiMedium was mixed with 45 µL of Lipofectamine 3000. The contents of these two tubes were then combined, and after a 10-minute incubation, the mixture was added to the cells. After 24 hours, the cell culture medium was aspirated, and the cells were washed with PBS and detached using trypsin-EDTA. Following trypsin inactivation with medium, the cells were centrifuged at 200g for 5 minutes. A total of 0.1 million cells were collected for control flow cytometry to confirm TROP2 or Her2 expression. For the antibody-CDN conjugate experiments, 25,000 cells in 50 µL were seeded into half-area 96-well plates coated with poly-lysine-D and incubated overnight. The next morning, the cell culture medium was aspirated, and the Her2 or TROP2 expressing cells were treated with 30 µL of serially diluted anti-Her2 mAb2-CDN-conjugates or anti-TROP2 mAb5-CDN conjugates, respectively. Furthermore, cells were also treated with compound 2.2, isotype mAb-CDN conjugates, or unconjugated mAb2 or mAb5. After 7-hour incubation, 20 µL of medium and 30 µL of BrightGlo per well were added, and the plates were placed on an orbital shaker (500 RPM, room temperature) for 5 minutes. A 50 µL mixture was transferred to white 96-well plates, and luminescence was measured using a TECAN reader.

EC₅₀ values were calculated from an 8-point dose-response curve using GraphPad Prism (La Jolla, USA) with nonlinear regression curve fitting. The EC₅₀ represents the concentration of the payload CDN 2.2 conjugated to anti-Her2 mAb2 or anti-TROP2 mAb5 that results in half-maximal levels of luminescence.

### Results

As evident from Table 18 and 19, the anti-HER2 and anti-TROP2 antibody-CDN conjugates effectively elicited the expression of the STING-dependent cytokine IP-10 in HCC1954 cells. Notably, the attachment of the warhead CDN compound 2.2 to both mAb2 and mAb5 resulted in a nearly three-order-of-magnitude reduction in the EC₅₀ values compared to cells treated with free compound 2.2. Similarly, in 293T STING reporter cells transiently expressing either TROP2 or HER2, mAb2 and mAb5 CDN conjugates exhibited significantly lower EC₅₀ values than the warhead CDN compound 2.2. It's important to emphasize that isotype ADCs or unconjugated mAb2 or mAb5 failed to induce IP-10 or luciferase in these assays (Table 18 and 19).

In summary, these findings strongly underscore the advantages of conjugating the disclosed CDN compound 2.2 to tumor-targeted monoclonal antibodies. Moreover, they demonstrate that compound 2.2 and its conjugates, when attached to tumor-targeted mAbs, induce a STING adaptor protein-dependent response, and stimulate the production of STING-dependent cytokine IP10.

### Example 10 Comparative analysis of in vivo plasma cytokine induction by anti-Her2 mAb2 conjugated with compound 3.3 at the inter-chain Cys sites vs. anti-HER2 mAb3 conjugated with compound 3.3 at engineered light chain C110 and heavy chain C375 sites

Compound 3.3 was conjugated to the engineered light chain C110 and heavy chain C375 of anti-Her2 mAb (mAb3) as described above (mAb3-3.3). Furthermore, the compound was also conjugated to anti-Her2 mAb (mAb2) via inter-chain Cys (mAb2-3.3). A single intravenous injection of 5 mg/kg mAb2-3.3 (DAR 3.8) and 5 mg/kg mAb3-3.3 (DAR 3.6) was administered to Balb/C mice via tail vein injection. Blood samples were collected from the injected animals at 2, 4, 8, and 20 hours post-injection, and plasma cytokines were measured using multiplex ELISA (Luminex).

### Results

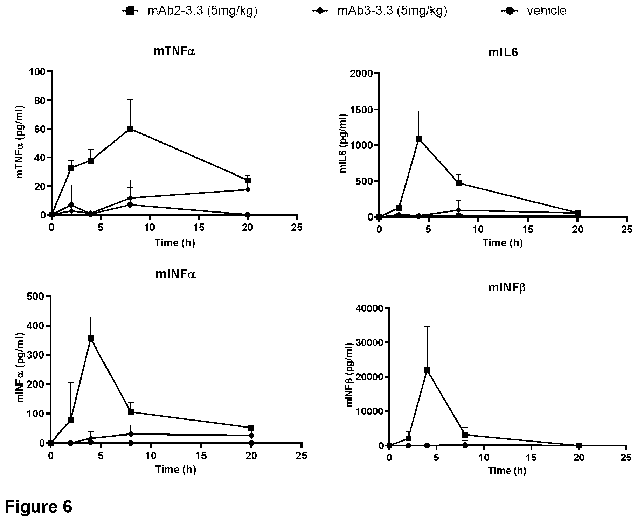

Mice injected with anti-Her2 mAb2-3.3, in which conjugation was done via inter-chain Cys, induced higher levels of plasma cytokines (Fig. 6). Surprisingly, the peak levels of cytokines induced by anti-Her2 mAb3-3.3 in which conjugation was performed to engineered Cys were significantly lower.

It can be advantageous to have an antibody-CDN conjugate that induces lower plasma levels of cytokines, as high levels of circulating cytokines can potentially lead to a severe inflammatory response, causing damage to tissues and organs, and possibly leading to multiple organ failure and death (Tisoncik et al; Microbiol. Mol. Biol. Rev. 2012, 76:16) Therefore, it is beneficial to conjugate warhead CDN compound 2.2 to the engineered light chain 110 and heavy chain 375 cysteines.

### Example 11 Comparative analysis of in vivo plasma cytokine induction by anti-TROP2 mAb4 conjugated with Compound 3.3 at the inter-chain Cys sites vs. anti-TROP2 mAb5 conjugated with Compound 3.3 at engineered light chain C110 and heavy chain C375 sites

Compound 3.3 was conjugated to anti-TROP2 mAb4 via inter-chain Cys and to the engineered light chain C110 and heavy chain C375 of anti-TROP2 mAb5, as described above. A single intravenous injection of 5mg/kg anti-TROP2 mAb4-3.3 (DAR 3.8), 5mg/kg anti-TROP2 mAb5-3.3 (DAR 3.8), 5mg/kg unconjugated anti-TROP2 mAb5, and 1.7mg/kg warhead CDN compound 2.2 were administered to Balb/C mice via tail vein injection. Blood samples were collected from the injected animals at 2, 4, 8, and 20 hours post-injection, and plasma cytokines were measured using multiplex ELISA (Luminex).

### Results

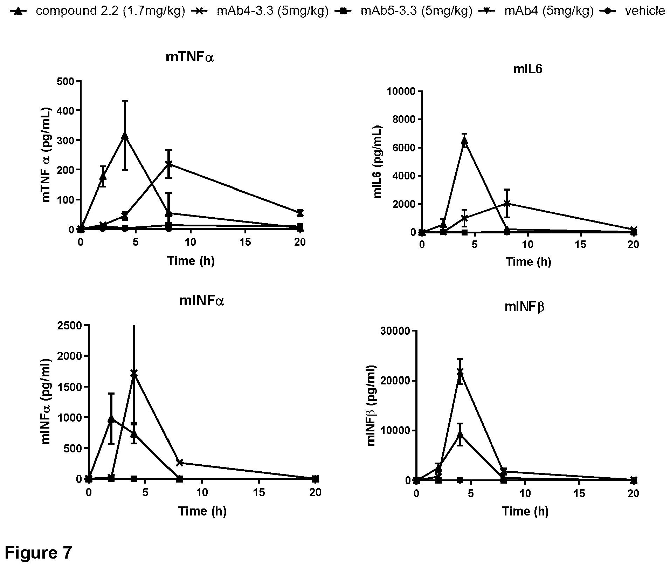

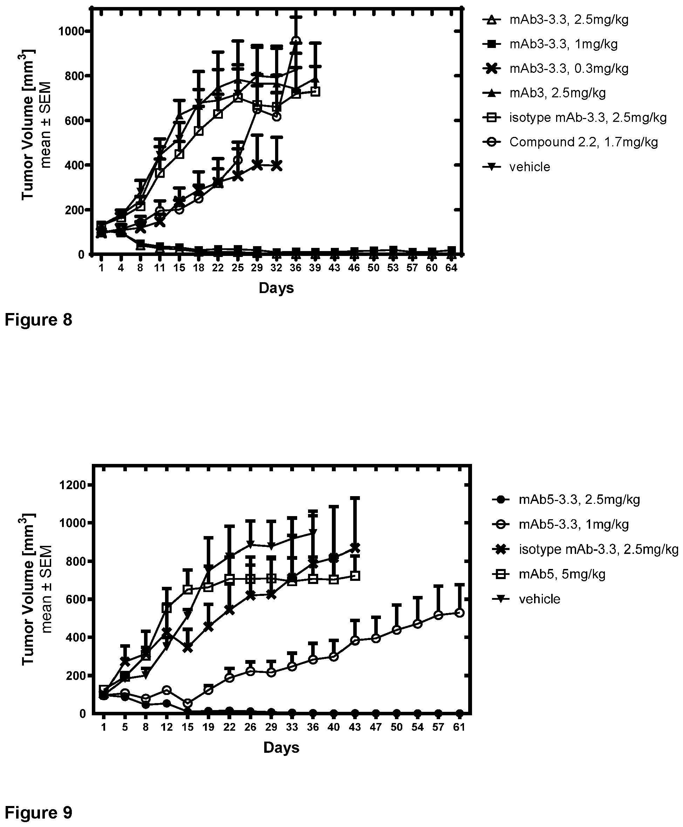

Mice injected with anti-TROP2 mAb4-3.3, where conjugation was done via inter-chain Cys, induced high levels of plasma cytokines (Fig. 7). Similarly, the payload CDN compound 2.2, injected at a dose that only weakly inhibited tumor growth (see Compound 2.2, Fig. 8), also induced high plasma levels of cytokines (Fig. 7). In contrast, the peak levels of cytokines induced by anti-TROP2 mAb5-3.3, with conjugation performed to engineered Cys, were significantly lower.

Having an antibody-CDN conjugate that induces lower plasma levels of cytokines can be advantageous, as high levels of circulating cytokines can potentially lead to a severe inflammatory response, causing damage to tissues and organs, and possibly leading to multiple organ failure and death (Tisoncik et al; Microbiol. Mol. Biol. Rev. 2012, 76:16). Therefore, it is beneficial to conjugate warhead CDN 2.2 to the engineered light chain 110 and heavy chain 375 cysteines.

### Example 12 Activity of anti-HER2 mAb3 conjugated with Compound 3.3 to light chain C110 and heavy chain C375 in Her2 expressing HCC1954 breast tumor xenograft model

Compound 3.3 was conjugated to the engineered light chain C110 and heavy chain C375 of anti-Her2 mAb (mAb3), as described earlier. An animal study was conducted using a Her2-expressing HCC1954 breast cancer xenograft mouse model as described above. Animals were pair-matched when the tumor reached a volume of 100 - 150 mm³ (designated as day 1). For in vivo testing of the anti-Her2 mAb3-3.3 (DAR 3.8), single intravenous doses of 2.5, 1, and 0.3 mg/kg were administered on day 1. Unconjugated mAb3 and isotype mAb-3.3 (DAR 3.8) were administered at a single intravenous dose of 2.5 mg/kg. The payload CDN compound 2.2 was tested using a single intravenous dose of 1.7 mg/kg.

### Results

HCC1954 breast tumor xenograft mice treated intravenously with anti-HER2 mAb3-3.3, at doses of 2.5 and 1 mg/kg, achieved complete regression of HCC1954 tumors (Fig. 8). Tumor growth inhibition was observed when a dose of 0.3 mg/kg of anti-Her2 mAb3-3.3 was used. In contrast, tumor regression was not observed in mice treated with 2.5 mg/kg of unconjugated mAb3 or isotype mAb-3.3 ADC (Fig. 8). Payload CDN compound 2.2, injected at a 40x higher dose than in the 2.5 mg/kg anti-Her2 mAb3-3.3 treatment, only triggered tumor growth inhibition.

These data demonstrate that that conjugation of the warhead CDN 2.2 to tumor targeted mAb3 is highly advantegous and that tumor regression in HER2-expressing HCC1954 breast tumor xenografts can be achieved through treatment with anti-HER2 mAb3-3.3 when conjugation is performed with the light chain C110 and heavy chain C375.

### Example 13 Activity of anti-TROP2 mAb5 conjugated with Compound 3.3 to engineered light chain C110 and heavy chain C375 in TROP2 expressing HCC1954 breast tumor xenograft model

Compound 3.3 was conjugated to the engineered light chain C110 and heavy chain C375 of anti-TROP2 mAb (mAb5), following the previously described method. An animal study was conducted using a TROP2-expressing HCC1954 breast cancer xenograft mouse model, with animals being pair-matched when the tumor reached a volume of 100 - 150 mm³ (designated as day 1). They were then injected with a single intravenous dose of 2.5 or 1 mg/kg of the anti-TROP2 mAb5-3.3 conjugate (DAR 3.6), 2.5 mg/kg of isotype mAb-3.3 conjugate (DAR 3.8), 5 mg/kg of mAb5, or PBS vehicle.

### Results

As depicted in Fig. 9, the administration of 2.5 mg/kg of anti-TROP2 mAb5-3.3 resulted in tumor regression, and treatment with 1 mg/kg of anti-TROP2 mAb5-3.3 led to the tumor growth inhibition of human breast cancer tumors expressing TROP2. In contrast, unconjugated anti-TROP2 mAb5 and isotype mAb-3.3 had no discernible effect on tumor growth.

These results demonstrate that tumor regression can be achieved in TROP2-expressing HCC1954 breast tumor xenografts through treatment with anti-TROP2 mAb5-VAP-2218 when conjugation is executed with light chain C110 and heavy chain C375.

### Example 14 Activity of anti-TROP2 mAb5 conjugated with Compound 3.3 to engineered light chain C110 and heavy chain C375 in TROP2 expressing Bx-PC3 prostate tumor xenograft model

Compound 3.3 was conjugated to the engineered light chain C110 and heavy chain C375 of anti-TROP2 mAb (mAb5), following the previously described method. The Bx-PC3 human pancreatic carcinoma cell line was obtained from American Type Culture Collection (ATCC^{®}, CRL-1687^{™}) and maintained in RPMI-1640 medium containing 10% fetal bovine serum, 2 mM glutamine, 100 units/mL penicillin G sodium, 100 µg/mL streptomycin sulfate, 25 µg/mL gentamicin, 10 mM HEPES, 0.075% sodium bicarbonate, and 1 mM sodium pyruvate. The tumor cells were grown in tissue culture flasks in a humidified incubator set at 37 °C and an atmosphere of 5% CO₂ and 95% air. On the day of tumor implantation to female CB.17 SCID mice (Charles River Laboratories), the Bx-PC3 cells were harvested during the exponential growth phase and resuspended in phosphate-buffered saline (PBS) and 50% Matrigel^{™} (BD Biosciences) mixture at 1 × 10⁸ cells/mL concentration. Tumors were initiated by subcutaneous implantation of 1 × 10⁷ Bx-PC3 cells (in a 0.1 mL suspension) into the right flank of each test animal. Tumors were measured in two dimensions and tumor volumes were calculated from caliper measurements using formula (L × W²)/2. Animals were pair matched when tumor reached volume 100 - 150 mm³ (designated as day 1) and injected with a single I.V. dose of 5mg/kg of anti-TROP2 mAb5-3.3 conjugate (DAR 3.6), 5mg/kg isotype mAb-3.3 conjugate (DAR 3.8), 5mg/kg mAb5 or PBS vehicle on day 1.

### Results

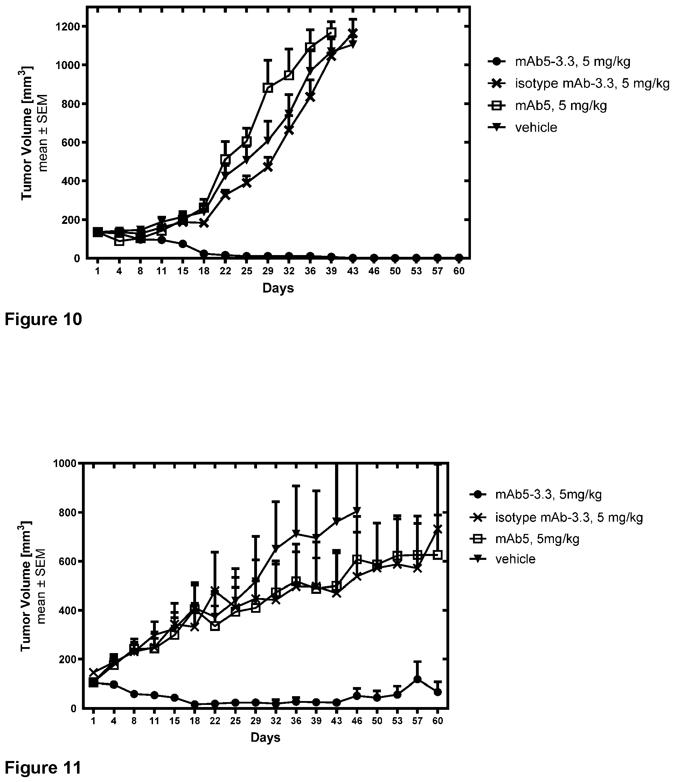

As illustrated in Fig. 10, the administration of 5 mg/kg of anti-TROP2 mAb5-3.3 resulted in tumor regressions, while unconjugated anti-TROP2 mAb5 and isotype mAb-3.3 did not exhibit any impact on tumor growth.

These findings indicate that tumor regression can be attained in TROP2-expressing Bx-PC3 pancreas tumor xenografts through treatment with anti-TROP2 mAb5-3.3 when conjugation is carried out using light chain C110 and heavy chain C375.

### Example 15 Activity of anti-TROP2 mAb5 conjugated with Compound 3.3 to engineered light chain C110 and heavy chain C375 in TROP2 expressing Colo205 colon tumor xenograft model

Compound 3.3 was conjugated to the engineered light chain C110 and heavy chain C375 of anti-TROP2 mAb (mAb5), following the previously described method. The COLO-205 human colon adenocarcinoma cell line was obtained from American Type Culture Collection (ATCC^{®}, CCL-222^{™}) and maintained in RPMI-1640 medium containing 10% fetal bovine serum, 2 mM glutamine, 10 mM HEPES, 1 mM sodium pyruvate and 4.5g/L glucose. The tumor cells were grown in tissue culture flasks in a humidified incubator set at 37°C and an atmosphere of 5% CO₂ and 95% air. On the day of tumor implantation, the COLO205 cells were harvested during the exponential growth phase and resuspended in 50% Geltrex^{™} (ThermoFisher; A14132-02) prepared in Dulbecco's Phosphate - Buffered Saline (DPBS) at 6 × 10⁷ cells/mL concentration. Tumors were initiated by subcutaneous implantation of 3 × 10⁶ COLO205 cells (in a 0.1 mL suspension) into the right flank of female CD-1 nude mice (CR - Crl:CD1-Foxn1nu). Tumors were measured in two dimensions and tumor volumes were calculated using formula (L × W²)/2. Animals were pair matched when tumor volumes reached 80-140 mm³ (designated as day 1) and injected with a single i.v. dose of 5mg/kg of anti-TROP2 mAb5-3.3 (DAR 3.6), 5mg/kg isotype mAb-VAP-3.3 (DAR 3.8), 5mg/kg mAb5 or PBS vehicle.

### Results

As depicted in Fig. 11, the administration of 5 mg/kg of anti-TROP2 mAb5-3.3 resulted in tumor regressions, while unconjugated anti-TROP2 mAb5 and isotype mAb-3.3 did not exhibit any effect on tumor growth.

These findings demonstrate that tumor regression can be achieved in TROP2-expressing Colo205 colon tumor xenografts through treatment with anti-TROP2 mAb5-3.3 when conjugation is performed using light chain C110 and heavy chain C375.

### Example 16 Intratumoral cytokines induced in TROP2 positive HCC1954 mouse model treated with anti-TROP2 mAb5 conjugated with Compound 3.3 to engineered light chain C110 and heavy chain C375

Compound 3.3 was conjugated to engineered light chain C110 and heavy chain C375 of anti-TROP2 mAb (mAb5) as described previously. For *in vivo* testing in the TROP2 expressing HCC1954 breast cancer xenograft mouse model, female CD-1 Nude (CR - Crl:CD1-Foxn1nu) immunodeficient outbred mice (Charles River Laboratories) at 9 to 10 weeks of age were used. HCC1954 cells (ATCC^{®}, CRL-2338^{™}) were cultivated in RPMI-1640 medium containing 10% fetal bovine serum, 2 mM glutamine, 10 mM HEPES, 1 mM sodium pyruvate and 4.5g/L glucose. The cells were grown in a humidified incubator set at 37°C and an atmosphere of 5% CO₂ and 95% air. On the day of tumor implantation, the HCC1954 cells were harvested during the exponential growth and resuspended in 50% Geltrex^{™} (ThermoFisher; A14132-02) prepared in Dulbecco's Phosphate - Buffered Saline (DPBS) at 1 × 10⁷ cells/mL. Tumors were initiated by subcutaneous implantation of 1 × 10⁶ HCC1954 cells (in a 0.1 mL suspension) into the right flank of each test animal. Tumors were measured in three dimensions using digital calipers to monitor growth. Animals were pair-matched when tumor volumes reached 80-140 mm³ and injected with a single i.v. dose of 5mg/kg of anti-TROP2 mAb5-3.3 (DAR 3.6), 5mg/kg isotype mAb-3.3 (DAR 3.8), 5mg/kg mAb5 or PBS vehicle.

Tumors were collected 20 h post i.v., and immediately stored in RNAprotect^{®} Tissue Reagent (Qiagen) untill further processing. Tissue was homogenized using Polytron homogenizer (Kinematica) and total RNA was isolated using RNeasy Plus Mini Kit (Qiagen). Relative expression of mouse and human cytokines was measured by using Luna Universal One-Step RT-qPCR Kit (Promega) according to the manufacturer protocol. qRT-PCR was run on Light Cycler 480 II (Roche) with annealing time 60°C for all tested primers.

| **Primer name** | **sequence** |
|---|---|
| mInfb1 F | AACTCCACCAGCAGACAGTG (SEQ ID NO: 27) |
| mInfb1 R | GGTACCTTTGCACCCTCCAG (SEQ ID NO: 28) |
| mIl6 F | GGAGGCTTAATTACACATGTTCTCTG (SEQ ID NO: 29) |
| mIl6 R | AAGTGCATCATCGTTGTTCATACA (SEQ ID NO: 30) |
| mIP10 F | GTC TGA GTG GGA CTC AAG (SEQ ID NO: 31) |
| mIP10 R | GCAGGGATGATTTCAAGC (SEQ ID NO: 32) |
| mRantes F | CCCACGTCAAGGAGTATTTC (SEQ ID NO: 33) |
| mRantes R | ACCCTCTATCCTAGCTCATC (SEQ ID NO: 34) |
| huRantes F | CTGCTGCTTTGCCTACATTG (SEQ ID NO: 35) |
| huRantes R | TCCCGAACCCATTTCTTCTC (SEQ ID NO: 36) |
| huIP10 F | GAAGGGTGAGAAGAGATGTC (SEQ ID NO: 37) |
| huIP10 R | TAGGGAAGTGATGGGAGAG (SEQ ID NO: 38) |

### Results

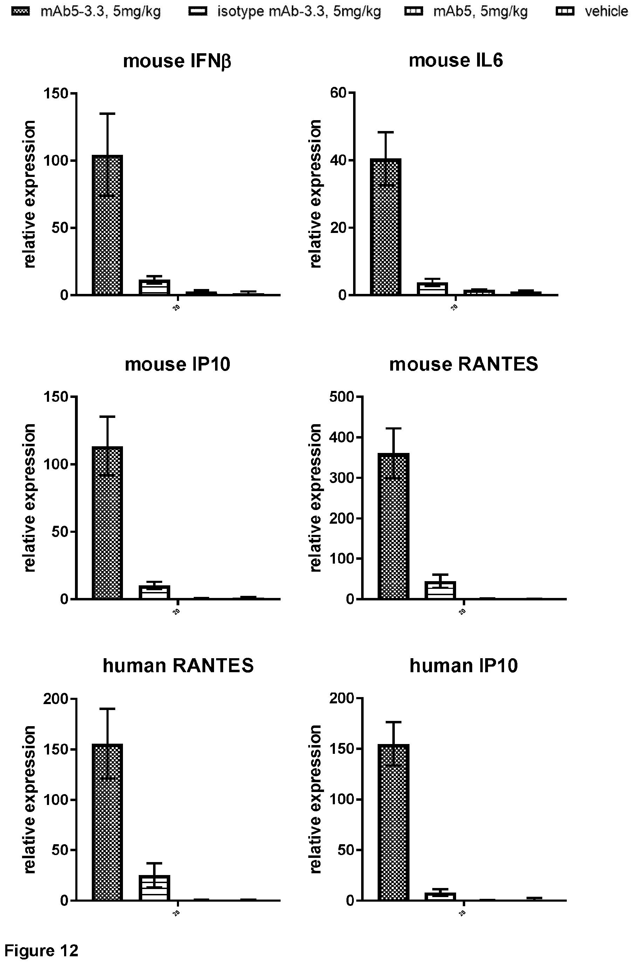

HCC1954 breast tumor xenograft mice were treated intravenously with a single I.V. dose of 5mg/kg of anti-TROP2 mAb5-3.3 (DAR 3.6), 5mg/kg isotype mAb-3.3 (DAR 3.8), 5mg/kg mAb5 or vehicle. Treatment with anti-TROP2 mAb5-3.3 (Fig. 12) resulted in statistically significant overexpression of mouse INFβ, IL6, RANTES and IP-10 and human IP10 and RANTES compared to controls.

This data show that anti-TROP2 mAb5-3.3 can selectively deliver warhead CDN compound 2.2 to the tumor tissue. Moreover, they demonstrate that anti-TROP2 mAb5-3.3 induce a STING adaptor protein-dependent response, and stimulate the production of STING-dependent cytokines in vivo.

### Example 17 Activity of anti-HER2 mAb3 conjugated with Compound 3.13 to engineered light chain C110 and heavy chain C375 in Her2 expressing HCC1954 breast tumor xenograft model

Compound 3.13 was conjugated to engineered light chain C110 and heavy chain C375 of anti-Her2 mAb (mAb3) as described above. For in vivo testing in the HCC1954 breast cancer xenograft mouse model, female SCID-beige mice at 8 to 12 weeks of age (purchased from Charles River Lab) were used for implantation. HCC1954 cells (obtained from ATCC) were cultured in RPMI medium supplemented with 10% fetal bovine serum at 37°C in a humidified incubator with 5% CO₂. On the day of implantation, 1 × 10⁷ HCC1954 cells were re-suspended in 100µL RPMI1640 serum free media containing 50% Matrigel and implanted subcutaneously in the right flank. After implant, tumors were measured by caliper and mice were weighed two times per week once tumors were palpable. Tumors were measured in two dimensions and tumor volumes were calculated from caliper measurements using formula (L × W²)/2. Animals were pair matched when tumor reached volume 100 - 150 mm³ (designated as day 1) and injected with single I.V dose 1, 0.4 and 0.2 mg/kg anti-Her2 mAb3-3.13 (DAR 3.8), 1mg/kg isotype mAb-3.13 (DAR 3.8), and PBS vehicle.

### Results

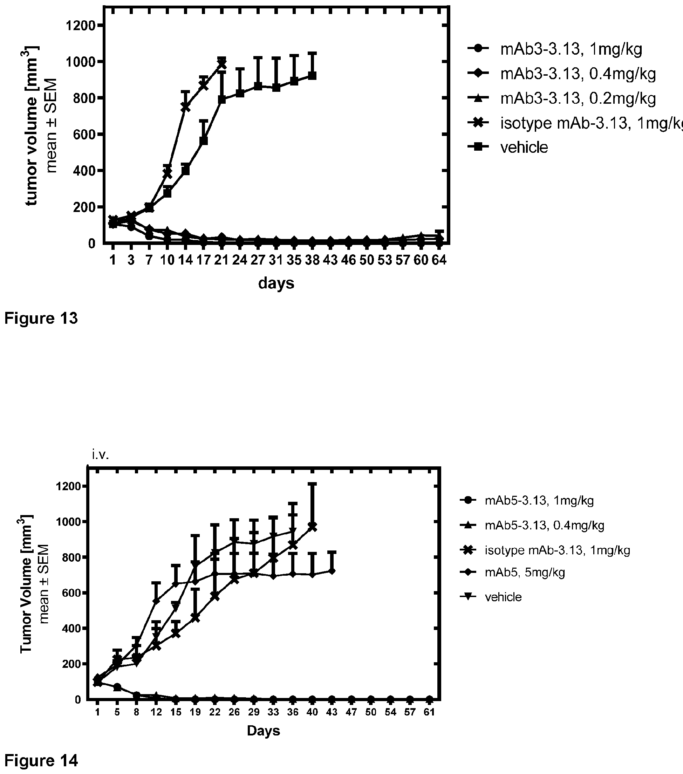

Treatment with single dose 1, 0.4 and 0.2 mg/kg anti-Her2 mAb3-3.13 led to regression of human HCC1954 xenograft tumors (Fig. 13). Tumor regression was not observed in mice treated with 1 mg/kg of isotype mAb-3.13 ADC (Fig. 13).

These data show that tumor regression can be achieved in Her2 expressing HCC1954 breast tumor xenograft by treatment with anti-Her2 mAb3-3.13. Surprisingly, mAb3-3.13 achieved tumor regression at dose 0.2mg/kg whereas mAb3-3.3 triggered only tumor growth inhibition at dose 0.3mg/kg (Fig 8) even though both conjugates contain the same warhead CDN 2.2. Therefore, it may be advantageous to exclude the self-immolative N-methylethanolamine part from the linker of the conjugate.

### Example 18 Activity of anti-TROP2 mAb5 conjugated with Compound 3.13 to engineered light chain C110 and heavy chain C375 in TROP2 expressing HCC1954 breast tumor xenograft model

Compound 3.13 was conjugated to engineered light chain C110 and heavy chain C375 of anti-TROP2 mAb5 as described above. For in vivo testing in the HCC1954 breast cancer xenograft mouse model, female SCID-beige mice at 8 to 12 weeks of age (purchased from Charles River Lab) were used for implantation. HCC1954 cells (obtained from ATCC) were cultured in RPMI medium supplemented with 10% fetal bovine serum at 37°C in a humidified incubator with 5% CO₂. On the day of implantation, 1 × 10⁷ HCC1954 cells were re-suspended in 100µL RPMI1640 serum free media containing 50% Matrigel and implanted subcutaneously in the right flank. After implant, tumors were measured by caliper and mice were weighed two times per week. Tumors were measured in two dimensions and tumor volumes were calculated from caliper measurements using formula (L × W²)/2. Animals were pair matched when tumor reached volume 100 - 150 mm³ (designated as day 1) and injected with a single i.v. dose 1 and 0.4 mg/kg anti-TROP2 mAb5-3.13 (DAR 3.6), 5mg/kg unconjugated mAb5 or 1mg/kg isotype mAb-3.13 (DAR 3.8).

### Results

As depicted in Fig.14, treatment with 1 and 0.4 mg/kg of anti-TROP2 mAb5-3.13 induced regressions of the human breast cancer tumors whereas un-conjugated anti-TROP2 mAb5 and isotype mAb-3.13 did not have any effect on tumor growth. Unexpectedly, 0.4 mg/kg of anti-TROP2 mAb5-3.13 had superior activity compared to 1 mg/kg of anti-TROP2 mAb5-3.3 in the same mouse model (Fig. 9 and 14) despite both conjugates containing the same warhead CDN compound 2.2. Therefore, it is advantageous to remove N-methylethanolamine from the linker of the conjugate.

### Example 19 Activity of anti-FOLR1 mAb6 conjugated with Compound 3.3 to engineered heavy chain C152 and C375 in FOLR1 expressing KB cervical tumor xenograft model

Compound 3.3 was conjugated to the engineered heavy chain C152 and C375 of anti-FOLR1 mAb (mAb6), following the previously described method. The KB human cervical carcinoma cell line was obtained from American Type Culture Collection (ATCC^{®}, CRL-3596^{™}) and maintained in Eagle's Minimum Essential Medium containing 10% fetal bovine serum. The tumor cells were grown in tissue culture flasks in a humidified incubator set at 37°C and an atmosphere of 5% CO₂ and 95% air. On the day of tumor implantation, the KB cells were harvested during the exponential growth phase (60-70% confluency) and resuspended in 50% Geltrex^{™} (ThermoFisher; A14132-02) prepared in Dulbecco's Phosphate - Buffered Saline (DPBS) at 3 × 10⁷ cells/mL concentration. Tumors were initiated by subcutaneous implantation of 3 × 10⁶ KB cells (in a 0.1 mL suspension) into the right flank of female CD-1 nude mice (CR - Crl:CD1-Foxn1nu). Tumors were measured in two dimensions and tumor volumes were calculated from caliper measurements using formula (L × W²)/2. Animals were pair matched when tumor volumes reached 100-140 mm³ (designated as day 1) and injected with three i.v. doses of 2.5mg/kg of anti-FOLR1 mAb6-3.3 (DAR 3.8), 2.5mg/kg isotype mAb-VAP-3.8 (DAR 3.8), 2.5mg/kg mAb6 or PBS vehicle on day 1, 8 and 15.

### Results

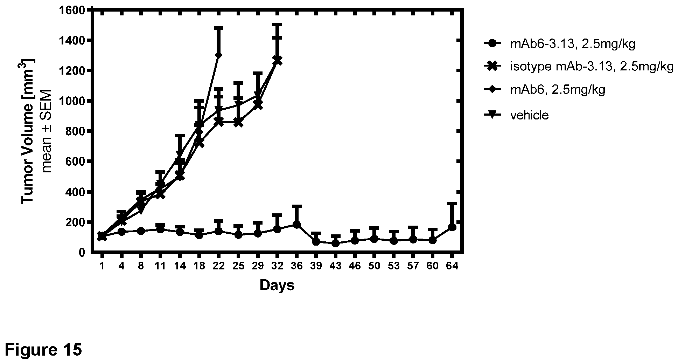

As depicted in Fig. 15, the administration of 2.5 mg/kg of anti-FOLR1 mAb6-3.3 resulted in tumor growth inhibition, while unconjugated anti-FOLR1 mAb6 and isotype mAb-3.3 did not exhibit any effect on tumor growth.

These findings demonstrate that tumor inhibition can be achieved in FOLR1-expressing KB tumor xenografts through treatment with anti-FOLR1 mAb6-3.3 when conjugation is performed using heavy chain chain C152 and C375.

## Claims

1. Compound of general formula I: 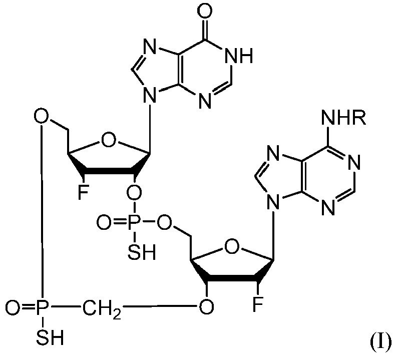 wherein
R is selected from H, 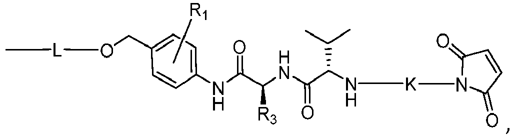 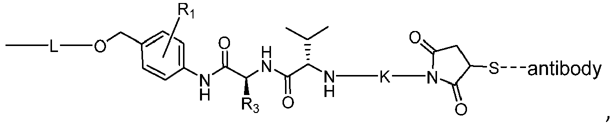 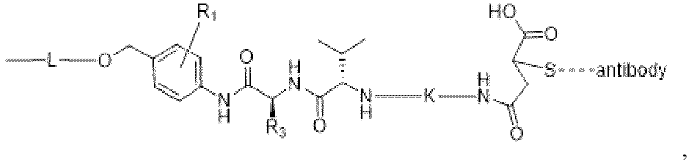 and 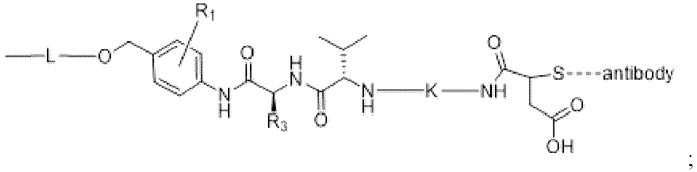 wherein
R1 is selected from H, F, and Cl;
R3 is selected from C₁-C₆ alkyl, and amino acid side chains;
-L- is selected from -C(O)- and -C(O)-O-CH₂-CH₂-N(CH₃)-C(O)-;
-K- is selected from -C(O)-(CH₂)ₘ-, -C(O)-(CH₂CH₂O)ₙ-CH₂CH₂-, -C(O)-(CH₂CH₂O)ₙ-CH₂CH₂NH-C(O)-(CH₂)ₚ-, -C(O)-(CH₂)ₘ-NH-C(O)-(CH₂)ₚ-, -C(O)-(CH₂)ₘ-N(CH₃)-C(O)-(CH₂)ₚ-;
wherein m=1-8, n=1-8, p=1-5;
or a pharmaceutically acceptable salt or solvate or formulation thereof.

2. Compound according to claim 1, wherein R3 is selected from C₁-C₆ alkyl, -(CH₂)₃-NH-C(O)-NH₂.

3. Compound according to claim 1 or 2, wherein R1 is F or Cl.

4. Compound according to claim 1, which is a compound of formula II, or its pharmaceutically acceptable salt or solvate,

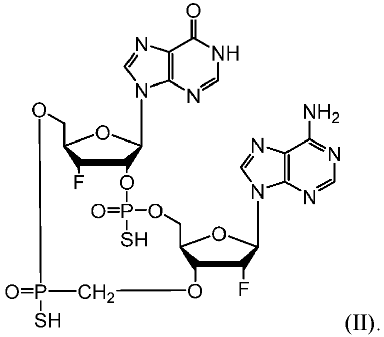

5. Compound according to claim 1 or 2 or 3, which is a compound of general formula III, or its pharmaceutically acceptable salt or solvate,

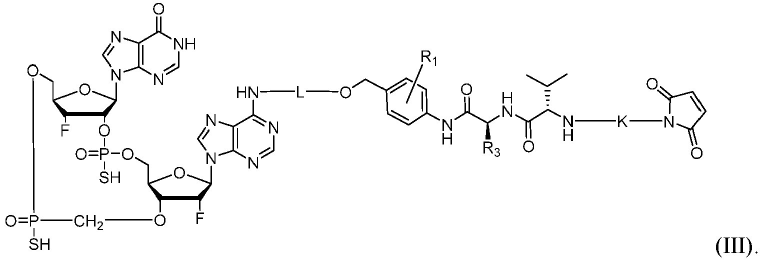

6. Compound according to claim 1 or 2 or 3, which is a compound of general formula IV, V or VI, or its pharmaceutically acceptable salt or solvate or formulation,

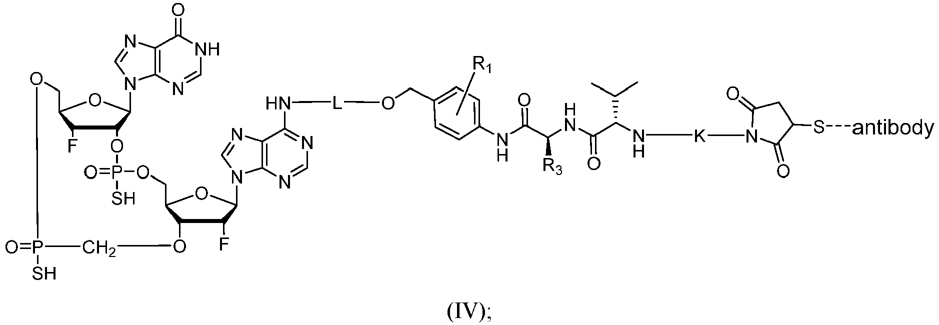

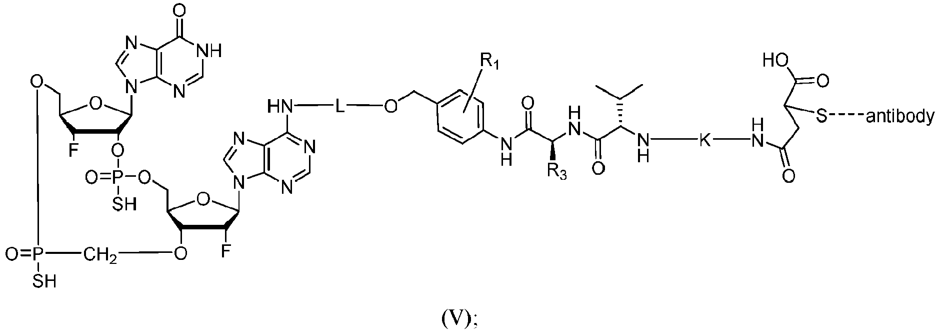

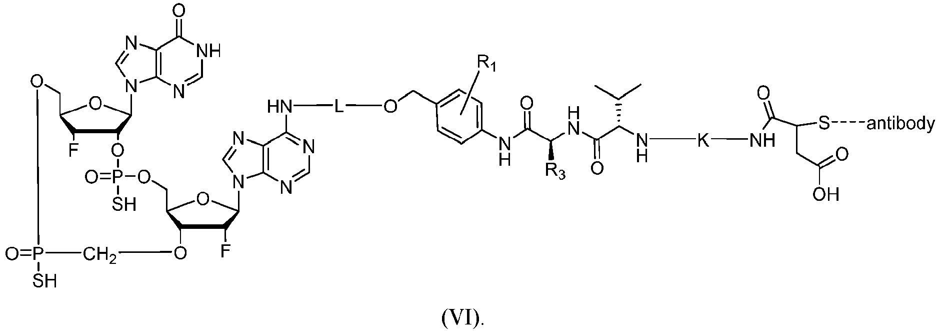

7. Compound according to claim 1 or claim 6, wherein the antibody is a monoclonal antibody, and/or wherein the antibody is human or humanized.

8. Compound according to claim 1 or 6 or 7, wherein the antibody is bound via a cysteine residue.

9. Compound according to any one of claim 1, 6, 7 or 8, wherein the antibody is engineered to contain at least one cysteine in the heavy or light chain portion, preferably at positions 110 of light chain and/or 152 and/or 375 of heavy chain accoding to EU numbering.

10. Compound of any one of claims 1-4 or 6-9 for use in the treatment or prevention of cancer.

11. Compound for use according to claim 10, wherein the cancer is selected from solid tumors and lymphomas.

12. Compound for use according to claim 10, wherein the cancer is selected from adrenal cancer, bladder cancer, bone cancer, brain cancer, breast cancer, colon cancer, colorectal cancer, eye cancer, head-and-neck cancer, kidney cancer such as renal cell carcinoma, liver cancer, lung cancer such as non-small cell lung cancer, ovarian cancer, pancreatic cancer, prostate cancer, skin cancer such as squamous cell carcinoma and melanoma, thyroid cancer, uterine cancer, vaginal cancer, and myeloma such as multiple myeloma.

13. Compound for use according to claim 10, wherein the cancer is selected from Burkitt's lymphoma, Hodgkin's lymphoma, non-Hodgkin's lymphoma (NHL), indolent non-Hodgkin's lymphoma (iNHL), refractory iNHL, multiple myeloma (MM), chronic myeloid leukemia (CML), acute lymphocytic leukemia (ALL), B-cell ALL, acute myeloid leukemia (AML), chronic lymphocytic leukemia (CLL), small lymphocytic lymphoma (SLL), myelodysplastic syndrome (MDS), myeloproliferative disease (MPD), mantle cell lymphoma (MCL), follicular lymphoma (FL), Waldestrom's macroglobulinemia (WM), T-cell lymphoma, B-cell lymphoma, diffuse large B-cell lymphoma (DLBCL), and marginal zone lymphoma (MZL).

14. Compound for use according to any one of claims 10 to 13, wherein the compound is administered parenterally.

15. A pharmaceutical composition comprising at least one compound of formula I according to claim 1 or 2 or 3, preferably at least one compound of formula II according to claim 4 and/or at least one compound of formula IV, V and/or VI according to any one of claims 6 to 9, or a pharmaceutically acceptable salt, solvate or formulation thereof, and at least one pharmaceutically acceptable excipient.
